# EUROPEAN PATENT SPECIFICATION

(11) **EP 1 367 961 B1**
(45) Date of publication and mention of the grant of the patent: **31.12.2008**
(21) Application number: 02717398.8
(22) Date of filing: 06.02.2002
(51) Int. Cl.: C12N 5/00, C12N 5/06

(54) **PEPTIDE SCAFFOLD ENCAPSULATION OF TISSUE CELLS AND USES THEREOF**
PEPTIDGERÜSTVERKAPSELUNG VON GEWEBSZELLEN UND VERWENDUNGEN DAVON
ENCAPSULATION EN ECHAFAUDAGE PEPTIDIQUE DE CELLULES TISSULAIRES, ET UTILISATIONS

(30) Priority: 06.02.2001 US 778200; 13.07.2001 US 305379 P
(43) Date of publication of application: 10.12.2003
(73) Proprietor: MASSACHUSETTS INSTITUTE OF TECHNOLOGY, Cambridge, Massachusetts 02139 (US)
(72) Inventor: KISIDAY, John, Watertown, MA 02472 (US); GRODZINSKY, Alan, J., Lexington, MA 02173 (US); ZHANG, Shuguang, Lexington, MA 02421 (US); SHERLEY, James, Boston, MA 02130 (US); SEMINO, Carlos, Cambridge, MA 02139 (US)
(74) Representative: Kirkham, Nicholas Andrew
(86) International application number: PCT/US2002/003482
(87) International publication number: WO 2002/062961

(56) References cited:
- US-A- 5 955 343
- US-A- 6 129 761
- US-A- 6 156 572
- AGGELI A ET AL: "RESPONSIVE GELS FORMED BY THE SPONTANEOUS SELF-ASSEMBLY OF PEPTIDES INTO POLYMERIC BETA-SHEET TAPES" NATURE, MACMILLAN JOURNALS LTD. LONDON, GB, vol. 386, no. 6622, 20 March 1997 (1997-03-20), pages 259-262, XP001172962 ISSN: 0028-0836
- ZHANG S ET AL: "Peptide self-assembly in functional polymer science and engineering" REACTIVE & FUNCTIONAL POLYMERS, ELSEVIER SCIENCE PUBLISHERS BV, NL, vol. 41, no. 1-3, 15 July 1999 (1999-07-15), pages 91-102, XP004172662 ISSN: 1381-5148
- KISIDAY J ET AL: "Self-assembling peptide hydrogel fosters chondrocyte extracellular matrix production and cell division: Implications for cartilage tissue repair" PROCEEDINGS OF THE NATIONAL ACADEMY OF SCIENCES OF THE UNITED STATES, vol. 99, no. 15, 23 July 2002 (2002-07-23), pages 9996-10001, XP002278385 July 23, 2002 ISSN: 0027-8424
- KISIDAY ET AL.: 'A new self-assembling peptide gel for cartilage tissue engineering: chondrocyte encapsulation and matrix production' INTERNATIONAL CARTILAGE REPAIR SOCIETY, 3RD SYMPOSIUM 27 April 2000 - 29 April 2000, (GOTHENBERG, SEDEN), page 69, XP002961633
- ZHANG ET AL.: 'Self-complementary oligopeptide matrices support mammalian cell attachment' BIOMATERIALS vol. 16, no. 18, 1995, pages 1385 - 1393, XP004032835
- HOLMES ET AL.: 'Extensive neurite outgrowth and active synapse formation on self-assembling peptide scaffolds' PROC. NATL. ACAD. SCI. USA vol. 97, no. 12, 06 June 2000, pages 6728 - 6733, XP002213924
- BUSCHMANN ET AL.: 'Mechanical compession modulates matrix biosynthesis in chondrocyte/agrose culture' JOURNAL OF CELL SCIENCE vol. 100, 1995, pages 1497 - 1508, XP002961635

## Description

### Statement as to Federally Sponsored Research

This invention was made with government support under NIH grants AR45779 and AR33236. The government therefore has certain rights in the invention.

### Background of the Invention

During the past decade there has been substantial effort expended to develop materials for the repair and replacement of various tissues, especially cartilage tissue in the knee joint. In particular, an increasing number of age-related disorders and degenerative diseases involving cartilage, heart, liver, kidney, pancreas, and the nervous system, require significant tissue repair or replacement when pharmaceuticals alone are ineffective. Although various polymeric biomaterials have been developed for tissue repair, the effectiveness of these is often compromised by immune incompatibility and improper distribution of stress. Self-assembling peptide gels for cartilage tissue engineering have been shown to support chondrocyte growth. The effect of compression schemes on such gels, however, was not known (KISIDAY ET AL., INTERNATIONAL CARTILAGE REPAIR SOCIETY, 3RD SYMPOSIUM 27 April 2000 - 29 April 2000, (GOTHENBERG, SWEDEN), page 69. Furthermore, the use of material from animals, such as cow hide or cartilage from pigs or sharks has raised concerns of possible contamination by infectious agents, such as prions. Many cell types are found within a three-dimensional environment within the body. However, the physical and chemical properties of three-dimensional environments (*e*.*g*., environments including extracellular matrix components such as collagen or fibronectin) may vary and thus may affect the ability of the environment to support the growth of different cell types.

Thus, improved materials of biological origin that have improved compatibility, present a reduced risk of contamination, and provide the proper biomechanical characteristics for tissue repair are needed. In addition, it would be desirable for such materials to promote the interaction between native tissue and implanted cells. For example, ideally implanted cell systems can integrate with surrounding tissue, enable long-term tissue formation, and restore tissue/organ function through *de novo* cellular regulation. Additionally, the implanted cells should be distributed throughout the defect and ultimately develop repair tissue that is mechanically and biologically functional.

For cartilage repair, the structural stability of the biomaterial should ideally be maintained by the biomaterial until encapsulated cells have deposited a continuous network of any needed extracelllular maxtix (ECM) throughout the implant. Replacement cartilage, for example, should integrate with surrounding normal cartilage, and the newly assembled ECM should provide tissue resilience to tissue compression that occurs during normal joint loading. The ability to control the rate of biodegradation of these materials is also desirable. In particular, the biomaterial should degrade as the ECM network matures, guiding regeneration throughout the entire biomaterial geometry.

The regenerative capacity of the body is particularly striking in the case of certain organs such as the liver, which is well-known to possess extensive capacity to regenerate after insults ranging from partial hepatectomy to toxin-induced injury. However, it has not been possible to satisfactorily maintain differentiated hepatocytes in culture. In addition, understanding of liver stem cells remains limited, and the potential of liver-derived cells has not been fully explored. Therefore, there is a need for the development of improved cell culture systems, techniques, and compositions for the propagation of liver progenitor cells and stem cells and for exploiting their differentiation and transdifferentiation potential. In addition, there is a need for the development of improved cell culture systems, techniques, and compositions for the propagation of various other progenitor or stem cells, including neural lineage cells, and for exploiting their differentiation and transdifferentiation potential.

### Summary of the Invention

The purpose of the present invention is to provide improved biomaterials and methods for tissue repair or replacement. We discovered that living cells may be encapsulated by a biodegradable peptide scaffold in a three-dimensional arrangement of predetermined geometry. The secretion of extracellular matrix components by, for example, the encapsulated cells significantly increases the stiffness of the scaffolds and thereby improves the ability of the scaffold to repair or replace endogenous cells that have been damaged, such as cartilage. Surprisingly, we have found that our scaffold systems originally developed for cartilage tissue replacement are capable of supporting a broad range of cell types, such as stem cells, neurons, and liver cells. This capacity for supporting a variety of cell types makes tissue and organ replacement possible for an extraordinary number of important therapeutic applications.

Accordingly, in one aspect, the invention features a macroscopic scaffold having amphiphilic peptides incorporating living cells. The peptides have alternating hydrophobic and hydrophilic amino acids, are complementary and structurally compatible, and self-assemble into a beta-sheet macroscopic scaffold. The macroscopic scaffold encapsulates the living cells, and the encapsulated cells are present in the macroscopic scaffold in a three-dimensional arrangement. In various embodiments, the macroscopic scaffold encapsulates cells other than chondrocytes.

In another aspect, the invention features a method of forming a macroscopic scaffold. This method involves incubating peptides and living cells in an aqueous solution having an iso-osmotic solute, desirably under conditions that do not allow the peptides to substantially self-assemble. Desirably, the solution contains less than 10, 5, 1, or 0.1 mM electrolyte or is substantially free of electrolyte. The peptides have alternating hydrophobic and hydrophilic amino acids and are complementary and structurally compatible. Sufficient electrolyte is then added to the solution to initiate self-assembly of the peptides into a beta-sheet macroscopic scaffold, whereby the cells are encapsulated by the formation of the macroscopic scaffold. The encapsulated cells are present in the macroscopic scaffold in a three-dimensional arrangement. In various embodiments, the macroscopic scaffold does not include chondrocytes. Desirably, the concentration of the added electrolyte is at least 5, 10, 20, or 50 mM. Desirable electrolytes include Li⁺, Na⁺, K⁺, and Cs⁺. In one desirable embodiment, the concentration of the iso-osmotic solute is at least 50, 150, or 300 mM. In another desirable embodiment, the concentration of the iso-osmotic solute is contained in one of the following ranges 200 to 250 mM, 250 to 270 mM, 270 to 300 mM, 300 to 400 mM, 400 to 500 mM, 500 to 600 mM, 600 to 700 mM, 700 to 800 mM, or 800 to 900 mM, inclusive. Desirable iso-osmotic solutes include carbohydrates, such as monosaccharides or disaccharides. Examples of desirable carbohydrates for use in the formation of the scaffold include sucrose, glucose, galactose, fructose, ribose, mannose, arabinose, and xylose. Still another desirable iso-osmotic solute is glycerol, such an aqueous solution of glycerol that is between 5 to 20% (v/v) glycerol.

In yet another aspect, the invention provides a method of forming a macroscopic scaffold of predetermined shape or volume. This method includes incubating peptides and living cells in an aqueous solution having an iso-osmotic solute, desirably under conditions that do not allow the peptides to substantially self-assemble. Desirably, the solution contains less than 10, 5, 1, or 0.1 mM electrolyte or is substantially free of electrolytes. The solution is contained in a pre-shaped mold dimensioned to determine the volume or shape of the macroscopic scaffold. The peptides have alternating hydrophobic and hydrophilic amino acids and are complementary and structurally compatible. Sufficient electrolyte is then added to the solution to initiate self-assembly of the peptides into a beta-sheet macroscopic scaffold, whereby the cells are encapsulated by the formation of the macroscopic scaffold in the desired comformation. The encapsulated cells are present in the macroscopic scaffold in a three-dimensional arrangement. In various embodiments, the macroscopic scaffold does not include chondrocytes. Desirably, the concentration of the added electrolyte is at least 5, 10, 20, or 50 mM. Desirable electrolytes include Li⁺, Na⁺, K⁺, and Cs⁺. In one desirable embodiment, the concentration of the iso-osmotic solute is at least 50, 150, or 300 mM. In another desirable embodiment, the concentration of the iso-osmotic solute is contained in one of the following ranges 200 to 250 mM, 250 to 270 mM, 270 to 300 mM, 300 to 400 mM, 400 to 500 mM, 500 to 600 mM, 600 to 700 mM, 700 to 800 mM, or 800 to 900 mM, inclusive. Desirable iso-osmotic solutes include carbohydrates, such as monosaccharides or disaccharides. Examples of desirable carbohydrates include sucrose, glucose, galactose, fructose, ribose, mannose, arabinose, and xylose. Still another desirable iso-osmotic solute is glycerol, such an aqueous solution of glycerol that is between 5 to 20% (v/v) glycerol.

In another aspect, the invention features a method of culturing cells. This method includes incubating peptides and living cells in an aqueous solution having an iso-osmotic solute, desirably under conditions that do not allow the peptides to substantially self-assemble. Desirably, the solution contains less than 10, 5, 1, or 0.1 mM electrolyte or is substantially free of electrolytes. The peptides have alternating hydrophobic and hydrophilic amino acids and are complementary and structurally compatible. Sufficient electrolyte is then added to the solution to initiate self-assembly of the peptides into a beta-sheet macroscopic scaffold, whereby the cells are encapsulated by the formation of the macroscopic scaffold in the desired comformation. The encapsulated cells are present in the macroscopic scaffold in a three-dimensional arrangement. The encapsulated cells are cultured in the macroscopic scaffold. In various embodiments, the macroscopic scaffold encapsulates cells other than chondrocytes. Desirably, the cells are cultured for at least 1, 2, 3, or 5 hours or 1, 2, 3, or 5 days, or 1, 2, 3, or 5 weeks. In desirable embodiments, the macroscopic scaffold is subjected to a predetermined compression scheme, as described herein. In some embodiments, the solution includes between 0.1 and 0.5%, 0.5 and 0.75%, 0.75 and 1.0, 1.0 and 1.5%, 1.5 and 2%, 2 and 3%, 3 and 5%, or 5 and 10% ITS medium, inclusive. In other embodiments, the solution includes at least 0.5, 1, 2, 3, 5, 7, or 10% ITS medium. In certain embodiments, the solution includes approximately 0.1 mg/L insulin, 0.055 mg/L transferrin, and/or 0.05 mg/L selenium. Desirably, the concentration of the added electrolyte is at least 5, 10, 20, or 50 mM. Desirable electrolytes include Li⁺, Na⁺, K⁺, and Cs⁺. In one desirable embodiment, the concentration of the iso-osmotic solute is at least 50, 150, or 300 mM. In another desirable embodiment, the concentration of the iso-osmotic solute is contained in one of the following ranges 200 to 250 mM, 250 to 270 mM, 270 to 300 mM, 300 to 400 mM, 400 to 500 mM, 500 to 600 mM, 600 to 700 mM, 700 to 800 mM, or 800 to 900 mM, inclusive. Desirable iso-osmotic solutes include carbohydrates, such as monosaccharides or disaccharides. Examples of desirable carbohydrates include sucrose, glucose, galactose, fructose, ribose, mannose, arabinose, and xylose. Still another desirable iso-osmotic solute is glycerol, such an aqueous solution of glycerol that is between 5 to 20% (v/v) glycerol.

In still another aspect, the invention features a method of regenerating a tissue within the body. This method includes administering to a mammal a macroscopic scaffold having amphiphilic peptides and encapsulated living cells. The peptides have alternating hydrophobic and hydrophilic amino acids, are complementary and structurally compatible, and self-assemble into a beta-sheet macroscopic scaffold. The encapsulated cells are present in the macroscopic scaffold in a three-dimensional arrangement. In various embodiments, the macroscopic scaffold encapsulates cells other than chondrocytes. Desirably, the method is used to treat or prevent a cartilage defect, connective tissue defect, nervous tissue defect, liver defect, epidermal lining defect, endothelial lining defect, diabetes, or arthritis. Desirable connective tissues include ligaments and tendons, and a desirable epidermal lining is skin. In some embodiments, the cells are autologogous or allogeneic. Desirable routes of administration include oral, percutaneous, intramuscular, intravenous, subcutaneous, and surgical. A desirable surgical administration is arthroscopic surgery. Desirably, the mammal is human.

In a related aspect, the invention provides another method of regenerating a tissue within the body. This method involves administering to a mammal a solution having amphiphilic peptides, living cells, and an iso-osmotic solute. The peptides have alternating hydrophobic and hydrophilic amino acids and are complementary and structurally compatible. The peptides do not substantially self-assemble prior to administration, but they self-assemble into a beta-sheet macroscopic scaffold after administration to the mammal. The formation of the macroscopic scaffold encapsulates the cells *in vivo,* and the encapsulated cells are present in the macroscopic scaffold in a three-dimensional arrangement. In various embodiments, the macroscopic scaffold encapsulates cells other than chondrocytes. Desirably, the administered solution contains less than 10, 5, 1.0, or 0.1 mM electrolyte or is substantially free of electrolyte. Desirably, the concentration of the iso-osmotic solute is at least 50, 150, or 300 mM. In another desirable embodiment, the concentration of iso-osmotic solute is contained in one of the following ranges 200 to 250 mM, 250 to 270 mM, 270 to 300 mM, 300 to 400 mM, 400 to 500 mM, 500 to 600 mM, 600 to 700 mM, 700 to 800 mM, or 800 to 900 mM, inclusive. Desirable iso-osmotic solutes include carbohydrates, such as monosaccharides or disaccharides. Examples of desirable carbohydrates include sucrose, glucose, galactose, fructose, ribose, mannose, arabinose, and xylose. Still another desirable iso-osmotic solute is glycerol, such an aqueous solution of glycerol that is between 5 to 20% (v/v) glycerol. Desirably, this method is used to treat or prevent a cartilage defect, connective tissue defect, liver defect, nervous tissue defect, epidermal lining defect, endothelial lining defect, diabetes, or arthritis. A desirable connective tissue is a ligament or tendon, and a desirable epidermal lining is skin. Desirably, the cells are autologogous or allogeneic. Desirable routes of administration include oral, percutaneous, intramuscular, intravenous, subcutaneous, and surgical. A desirable surgical administration is arthroscopic surgery. Desirably, the mammal is human.

In another aspect, the invention provides methods of treating an individual comprising identifying an individual in need of treatment and administering cells within the scaffold of the invention to the individual, wherein the cells have been induced to differentiate or transdifferentiate by culturing them encapsulated in a cell culture material comprising amphiphilic peptides. The peptides comprise substantially equal proportions of hydrophilic and hydrophobic amino acids, are complementary and structurally compatible, and are capable of self-assembling into a beta-sheet macroscopic structure, and wherein the cells are exposed to a differentiation-enhancing agent. The cells may be optionally extracted from the structure prior to administration or the cell/peptide structure may be introduced into the individual.

In another aspect, the invention provides a cell culture kit comprising (i) amphiphilic peptides, wherein the peptides comprise substantially equal proportions of hydrophilic and hydrophobic amino acids, are complementary and structurally compatible, and are capable of self-assembling into a beta-sheet macroscopic structure; and (ii) instructions for initiating self-assembly of the peptides into a macroscopic structure. The kit may further comprise at least one element selected from the group consisting of: a population of cells, cell culture medium, a predetermined amount of a growth factor, a predetermined amount of an electrolyte, instructions for encapsulating cells within a peptide hydrogel structure and for other uses of the system, instructions for inducing cells to differentiate or transdifferentiate within the scaffold, a vessel in which the encapsulation may be performed, a liquid in which the peptide can be dissolved, an electrolyte for initiating peptide self-assembly, medium for tissue culture, and one or more differentiation-enhancing agents.

In a related aspect, the invention provides a kit for treating a tissue defect in a patient. The kit includes peptides that have alternating hydrophobic and hydrophilic amino acids and are complementary and structurally compatible, an iso-osmotic solute, and sufficient electrolyte to initiate self-assembly of the peptides into a beta-sheet macroscopic scaffold, whereby cells are encapsulated by the formation of the scaffold and are present in said scaffold in a three-dimensional arrangement. The kit also desirably includes instructions for initiating self-assembly of said peptides into said scaffold. Desirably, the kit includes a means for preparing the cells for addition to a solution comprising the peptides, a means for applying pressure to the scaffold, a therapeutic agent or growth factor, and/or a mold for the formation of the scaffold in a desired geometry and/or volume.

The invention also provides three-dimensional nanoscale environment scaffolds that encapsulate cells and methods for using these scaffolds to treat an individual in need of the encapsulated cell type (*e*.*g*., for the treatment of an individual with a tissue or organ defect).

Accordingly, in one aspect, the invention provides methods of culturing cells comprising encapsulating the cells in a three-dimensional nanoscale environment scaffold. In certain embodiments, the nanoscale environment scaffold comprises a protein or peptide hydrogel. The hydrogel may be a self-assembling peptide hydrogel as described herein. In some embodiments, the peptides comprise amphiphilic peptides, wherein the peptides comprise substantially equal proportions of hydrophilic and hydrophobic amino acids, are complementary and structurally compatible, and are capable of self-assembling into a beta-sheet macroscopic structure. In some embodiments, the nanoscale environment scaffold comprises nanofibers. The nanofibers may be comprised of self-assembling peptides, *e*.*g*., any of the peptides described herein. In some embodiments, the cells comprise or consist of progenitor cells, stem cells, liver-derived cells, or cells derived from neural tissue. Progenitor cells or stem cells may be treated to induce differentiation prior to, during, or after implantation of the scaffold. For example, the cells may be instructed or induced to differentiate along a liver cell lineage pathway and/or along a neural lineage pathway. In some embodiments, cells comprise liver cells (*e*.*g*., liver progenitor cells, liver stem cells, hepatocytes, oval cells, bile duct cells) and/or neural lineage cells (*e*.*g*., neurons or cells associated with neural tissues, such as glia). While any cell type may be used in the scaffodl of the invention, in particular embodiments, the cells are chondrocytes, heart cells, bone marrow cells, peristeal cells, perichondrial cells, fibroblasts, neuronal cells, hippocampal cells, epidermal cells, endothelial cells, keratinocytes, basal cells, spinous cells, granular cells, non-human embryonic stem cells, ovarian cells, pancreatic cells, cervical cells, liver cells, and foreskin cells. In various embodiments, the nanoscale environment scaffold excludes chondrocytes prior to or at the time of implantation.

In another aspect, the invention provides a nanoscale environment scaffold encapsulating cells. The scaffolds encapsulating cells may be prepared according to the methods described herein or variations thereof, readily employed by those skilled in the art. In certain embodiments, the nanoscale environment scaffold comprises a protein or peptide hydrogel. The hydrogel may be a self-assembling peptide hydrogel, as described herein. In some embodiments, the peptides comprise amphiphilic peptides, wherein the peptides comprise substantially equal proportions of hydrophilic and hydrophobic amino acids, are complementary and structurally compatible, and are capable of self-assembling into a beta-sheet macroscopic structure. In certain embodiments, the nanoscale environment comprises or consists of an artificial material. In some embodiments, the artificial material comprises or consists of a material not naturally found in the body. Artificial material also encompasses certain materials obtained by isolating and processing substances produced by a living source. However, a material that remains substantially intact and substantially retains the structure in which it is naturally found within the body of an organism is not considered an artificial material. Any of a variety of artificial materials may be used. In some embodiments, the nanoscale environment scaffold comprises nanofibers. The nanofibers may be comprised of self-assembling peptides, *e*.*g*., any of the peptides described herein. In particular embodiments, the cells comprise isolated cells, *e*.*g*., cells that are not in their natural environment within the body of a subject. For example, the cells may comprise cells that have been removed from a subject. Such cells may have been cultured following removal prior to encapsulation. The cells may comprise a cell line. In some embodiments, the cells comprise or consist of progenitor cells. In certain embodiments, the cells comprise or consist of stem cells. In some embodiments, the cells comprise liver-derived cells or cells derived from neural tissue. In some embodiments, the cells comprise stem cells or progenitor cells that have been instructed or induced to differentiate. The cells may be instructed or induced to differentiate along a liver cell lineage pathway and/or along a neural lineage pathway. In some embodiments, these cells comprise liver cells (*e*.*g*., liver stem cells, liver progenitor cells, hepatocytes, oval cell, bile duct cells) and/or neural lineage cells (*e*.*g*., neurons or non-neuronal cells associated with neural tissues, such as glia). In some embodiments, the cells are heart cells, bone marrow cells, peristeal cells, perichondrial cells, fibroblasts, neuronal cells, hippocampal cells, epidermal cells, endothelial cells, keratinocytes, basal cells, spinous cells, granular cells, non-human embryonic stem cells, ovarian cells, pancreatic cells, cervical cells, liver cells, and foreskin cells. In various embodiments, the nanoscale environment scaffold excludes chondrocytes pior to or at the time of implantation. Of course the nanoscale environment scaffold encapsulating cells may encapsulate a combination of different cell types. When a combination of cell types is employed, chondrocytes may optionally be included.

In another aspect, the invention provides methods of treating an individual comprising identifying an individual in need of treatment and administering a nanoscale environment scaffold encapsulating cells to the individual. The nanoscale environment scaffold encapsulating cells may be any of the nanoscale environment scaffolds described herin. In particular embodiments, the nanoscale environment scaffold encapsulating cells comprise or consist of stem cells or progenitor cells. In some embodiments, the cells comprise liver cell lineage cells or neural lineage cells. In some embodiments, the cells comprise stem cells or progenitor cells that have been instructed or induced to differentiate. The cells may be instructed or induced to differentiate along a liver cell lineage pathway and/or along a neural lineage pathway. In some embodiments, the cells may comprise liver cells (*e*.*g*., liver stem cells, liver progenitor cells, hepatocytes, oval cells, bile duct cells) and/or neural lineage cells (*e*.*g*., neurons or glia). In other embodiments, the cells are chondrocytes, heart cells, bone marrow cells, peristeal cells, perichondrial cells, fibroblasts, neuronal cells, hippocampal cells, epidermal cells, endothelial cells, keratinocytes, basal cells, spinous cells, granular cells, non-human embryonic stem cells, ovarian cells, pancreatic cells, cervical cells, liver cells, and foreskin cells. In various embodiments, the nanoscale environment scaffold comprises cells other than chondrocytes.

In another aspect, the invention provides a method of testing a candidate compound that involves contacting a cell encapsulated by a macroscopic scaffold or nanoscale environment scaffold of the invention with a candidate compound and measuring the activity or expression level of a molecule expressed by the encapsulated cell (*e*.*g*., a protein, enzyme, or mRNA). If the level of activity or expression of the molecule differs in the presence of the candidate compound, then the candidate compound is determined to modulate the activity or expression of the molecule.

In desirable embodiments of various aspects of the invention, the macroscopic scaffold is enzymatically degradable. In other desirable embodiments, the macroscopic scaffold is cleaved by a metalloprotease, collagenase, or aggrecanase *in vivo* or *in vitro*. Desirably, an enzyme capable of cleaving the scaffold is produced by the encapsulating cells or nearby cells. In yet other desirable embodiments, the macroscopic scaffold further encapsulates a therapeutically active compound or chemoattractant. Examples of such therapeutically active compounds include synthetic organic molecules, naturally occurring organic molecules, nucleic acid molecules, biosynthetic proteins, or modified naturally occurring proteins. In still other desirable embodiments, the macroscopic scaffold further encapsulates a growth factor, such as a cartilage-derived growth factor, transforming growth factor-β, platelet derived growth factor, insulin-like growth factor, acidic fibroblast growth factor, basic fibroblast growth factor, epidermal growth factor, hepatocytic growth factor, keratinocyte growth factor, or bone morphogenic protein. Combinations of growth factors and/or therapeutic agents or chemoattractants may also be used. Desirable macroscopic scaffolds have peptides which include an adhesion site, growth factor binding site, growth factor, or sequence that provides targeting to a cell, tissue, organ, organ system, or site within an mammal.

Other desirable macroscopic scaffolds have a pre-determined volume or shape. In desirable embodiments, the encapsulated cells are substantially uniformly distributed. In other desirable embodiments, the encapsulated cells are neurons, and the macroscopic scaffold allows axonal outgrowth by the neurons. Desirably, the axons extend beyond the surface of the macroscopic scaffold. In yet other desirable embodiments, axons from neurons outside of the macroscopic scaffold extend into the macroscopic scaffold. In still other desirable embodiments, the cells differentiate after encapsulation by the macroscopic scaffold. Desirably, cells such as bone marrow cells, peristeal cells, perichondrial cells, or non-human embryonic stem cells differentiate into cartilage cells. In other desirable embodiments, the cells are polypotent or pluripotent. In some embodiments, the cells are progenitor or stem cells. In certain embodiments, the progenitor cells are derived from the liver, nervous tissue, cartilage, bladder, brain, , esophagus, fallopian tube, heart, intestines, gallbladder, kidney, liver, lung, ovaries, pancreas, prostate, spinal cord, spleen, stomach, testes, thymus, thyroid, trachea, ureter, urethra, or uterus. In some embodiments, the cells include progenitor cells that have been instructed or induced to differentiate. Desirably, the macroscopic structure renders at least a portion of the progenitor cells capable of differentiation in response to a differentiation-enhancing agent. The method may further include adding a differentiation-enhancing agent, such as a growth factor, added either before self-assembly or to media in which the cell/peptide structure is incubated. In some embodiments, the differentiation-enhancing agent causes a portion of the progenitor cells or their progeny to differentate or transdifferentate.

In still yet other desirable embodiments, the cells are differentiated prior to encapsulation by the macroscopic scaffold and remain differentiated after the encapsulation. In other desirable embodiments of any aspect of the invention, the encapsulated cells are cultured under conditions that inhibit or prevent de-differentiation. Desirably, at least 50, 60, 70, 80, 90, 95, or 100% of the encapsulated cells remain differentiated for at least 1, 2, 3, or 5 days or at least 1, 2, 3, or 5 weeks or longer.

Desirable cells include heart cells, bone marrow cells, peristeal cells, perichondrial cells, fibroblasts, neuronal cells, hippocampal cells, epidermal cells, endothelial cells, keratinocytes, basal cells, spinous cells, granular cells, embryonic stem cells, ovarian cells, pancreatic cells (*e*.*g*., islet cells), cervical cells, liver cells, and foreskin cells. Other examples of cells that may be encapsulated in some embodiments include chondrocytes. The cells may be from any suitable source such as a human or bovine cells. Source of the cells may also include embryos or fetal or adult mammals or established cell lines. In some embodiments, the cells comprise cell types that are normally found within a three-dimensional environment within the body. According to certain embodiments, the cell types are not naturally found in a cartilaginous environment within the body. In some embodiments, chondrocytes or chondrocyte precursor cells are excluded from the scaffold, except in combination with other cell types.

Desirably, at least 40, 50, 60, 70, 80, 90, or 95% of the encapsulated cells are viable 1, 2, 4, 6, or more weeks after formation of the macroscopic scaffold. In another desirable embodiment, at least 80 or 90% of the encapsulated cells are viable one day or one week after formation of the macroscopic scaffold. More desirably, at least 90% or 95% of the encapsulated cells are viable 6 weeks after formation of the macroscopic scaffold. Desirably, the number of living cells encapsulated by the macroscopic scaffold five days after formation of the macroscopic scaffold is at least 1, 1.5, 3, 5, or 10 million per ml of the volume of the macroscopic scaffold. In another desirable embodiment, the number of living cells encapsulated by the macroscopic scaffold 3, 5, 10, 15, or more days after scaffold formation is at least 2, 3, 5, 10, or 20-fold greater than the initial number of encapsulated cells. In yet another desirable embodiment, at least 60, 70, 80, 90, or 95% of the encapsulated cells are in cell-cell contact with another encapsulated cell or with a cell outside of the scaffold. Desirably, the encapsulated cells are maintained in culture. In some embodiments, the method also includes removing the encapsulated cells from the three-dimensional nanoscale environment scaffold. In particular embodiments, the nanoscale environment scaffold includes a protein or peptide hydrogel (*e*.*g*., a self-assembling peptide hydrogel).

In yet other desirable embodiments of any aspect of the invention, the macroscopic scaffold encapsulates a solution that includes insulin, transferrin, and/or selenium. In particular embodiments, the macroscopic scaffold encapsulates a solution having between 0.1 and 0.5%, 0.5 and 0.75%, 0.75 and 1.0, 1.0 and 1.5%, 1.5 and 2%, 2 and 3%, 3 and 5%, or 5 and 10% ITS medium, inclusive. In other embodiments, the macroscopic scaffold encapsulates a solution having at least 0.1, 0.5, 1, 2, 3, 5, 7, or 10% ITS medium. In still other embodiments, the macroscopic scaffold encapsulates a solution having between 0.01 and 10.00 mg/L insulin, 0.05 and 2 mg/L insulin, or 0.1 and 1 mg/L insulin, inclusive. In some embodiments, macroscopic scaffold encapsulates a solution having between 0.0055 and 5.5 mg/L transferrin, 0.055 and 1.0 mg/L transferrin, or 0.1 and 0.5 mg/L transferrin, inclusive. In various embodiments, macroscopic scaffold encapsulates a solution having between 0.005 and 5.0 mg/L selenium, 0.05 and 1.0 mg/L selenium, or 0.1 and 0.5 mg/L selenium, inclusive. In some embodiments, the solution includes at least 00.1 mg/L insulin, at least 0.005 mg/L transferrin, and/or at least 0.005 mg/L selenium. In certain embodiments, the solution includes approximately 0.1 mg/L insulin, 0.055 mg/L transferrin, and/or 0.05 mg/L selenium. In still other embodiments, the macroscopic scaffold encapsulates a solution that includes at least 0.1% fetal bovine serum (FBS). In yet other embodiments, the solution includes between 0.01 and 20% FBS, 0.1 and 5% FBS, 0.1 and 1% FBS, 0.1 and 0.5% FBS, or 0.2 and 1% FBS. Desirably, the solution includes approximately 1% ITS and 0.2% FBS. In other embodiments, ligament or tendon cells are encapsulated in a macroscopic scaffold and cultured in a medium without ITS, such as a medium that includes at lest 5, 10, 15, 18, or 20% FBS. A particularly useful medium to culture such cells is 18.5% FBS.

In other desirable embodiments, the encapsulated cells secrete extracellular matrix components. Desirably, the secretion of extracellular matrix components increases the equilibrium compression modulus of the macroscopic scaffold by at least 5, 10, 20, 30, 40, 50, 70, 80, 90, 100, 200, 300, 400, or 500 kPA or by at least 2, 5, 25, 50, 75, 100, 150, 200, or 250-fold. Desirably, the extracellular matrix secreted by a cell is in contact with the extracellular matrix secreted by another cell. More desirably, the extracellular matrix secreted by at least 60, 70, 80, 90, or 95% of the encapsulated cells is in contact with the extracellular matrix secreted by another encapsulated cell or secreted by a cell outside of the scaffold.

Desirable macroscopic scaffolds further include a biodegradable sealant, glue, or polymer attached to the surface of the macroscopic scaffold that increases the equilibrium compression modulus of the macroscopic scaffold. Desirably, the scaffold encapsulates cells of a desired cell type and chondrocytes, and the presence of chondrocytes in the scaffold leads to an increase in the stiffness of said scaffold.

In desirable embodiments of any of the methods of the invention, the macroscopic scaffold is subjected to a predetermined compression scheme. For example, the macroscopic scaffold may be subjected to constant or variable pressure for a predetermined amount of time, such as for as few as 1, 2, or 3 weeks to as long as 6, 8, or more weeks. A desirable compression scheme includes dynamic compression at 0.01 to 3 Hz or more desirably 0.1 to 1 Hz, superimposed on a static offset compression. Typically, the dynamic strain amplitude is between 0.01 and 10%, desirably, between 1 and 5%, and, more desirably, between 3 and 5%, and the static offset compression is between 5 and 15%.

In other embodiments, the compression scheme includes shear loading. In particular embodiments for shear loading, the macroscopic scaffold is compressed for a static offset of 0 to 45%, 0 to 40%, 0 to 30%, 0 to 20%, 0 to 10%, or 10 to 20%. In some embodiments, a shear strain of 0.01 to 30%, 0.05 to 20%, 0.1 to 10%, 1 to 10%, or 2 to 5% is applied to the macroscopic scaffold. In various embodiments, the frequency is 0.001 to 20 Hz, 0.001 to 10 Hz, 0.02 to 10 Hz, 0.1 to 10 Hz, or 1 to 5 Hz. In various embodiments, the shear loading can be continuous or variable.

A desirable compression/loading scheme includes subjecting the scaffold to pressure for one hour and incubating the scaffold in the absence of added pressure (*e*.*g*., incubating the scaffold at normal atmospheric pressure) for between one and seven hours, inclusive. In another embodiment, the compression/loading scheme includes subjecting the scaffold to pressure less than one hour (*e*.*g*., approximately 15, 30, or 45 minutes) and incubating the scaffold in the absence of added pressure for one of the following time ranges: (i) less than one hour, (ii) between one and seven hours, inclusive, or (iii) more than seven hours. In another embodiment, the compression/loading scheme includes subjecting the scaffold to pressure more than one hour (*e*.*g*., approximately 2, 3, 4, 6, 8, or more hours) and incubating the scaffold in the absence of added pressure for one of the following time ranges: (i) less than one hour, (ii) between one and seven hours, inclusive, or (iii) more than seven hours. In certain embodiments, the scaffold is incubated in the absence of added pressure for between five and six hours, inclusive, such as for five hours and 15 minutes or for five hours and 45 minutes. Desirably, the compression/loading scheme is repeated at least 1, 2, 3, 4, 5, 8, 10, or more times.

In other embodiments, the compression/loading scheme includes one or more cycles performed within 24 hours (*e*.*g*., performed on the same day) that include subjecting the scaffold to pressure and then incubating the scaffold in the absence of added pressure. These cycles are followed by 5-50 hours (*e*.*g*., 5-10, 10-20, 20-30, 30-40, or 40-50 hours) of incubating the scaffold in the absence of added pressure. According to these embodiments, the sequence involving (i) cycles of added pressure and no added pressure and (ii) a subsequent incubation without added pressure may be repeated, if desired, at least 1, 2, 3, 4, 6, 8, 10, 15, 20, or more times. Exemplary compression/loading schemes include one or more cycles of (i) subjecting the scaffold to pressure for 15, 30, 45, or 60 minutes and (ii) incubating the scaffold in the absence of added pressure (*e*.*g*., incubation for five hours and 15 minutes or five hours and 45 minutes), and, after completion of the aforementioned cycles, an incubation for approximately 15, 20, 24, or 30 hours without added pressure. In various embodiments, the compression/loading scheme is started approximately 2, 5, 10, 12, 15, 18, 21, 25, or more days after the cells are encapsulated.

Desirably, the compression scheme increases the equilibrium compression modulus of the macroscopic scaffold by at least 5, 10, 20, 30, 40, 50, 70, 80, 90, 100, 200, 300, 400, or 500 kPA or by at least 2, 5, 25, 50, 75, 100, 150, 200, or 250-fold compared to a control macroscopic scaffold not subjected to the compression scheme. In other embodiments, the dynamic stiffness of the macroscopic scaffold at 1 Hz reached is at least 0.05, 0.5, 1.0, 1.28, 1.5, 2. 5, or 3 MPa. In another desirable embodiment, the compression scheme induces the secretion of extracellular matrix components by the cells. In another embodiment, the compression scheme increases the rate of protein synthesis by the cells by at least 20, 50, 80, 100, 150, 200, or 300%.

Desirable peptides forming the macroscopic scaffold contain between 8 and 200 amino acids, 8 to 36 amino acids, or 8 to 16 amino acids, inclusive. Desirably, the concentration of the peptides is between 1 and 10 mg/ml or between 4 and 8 mg/ml, inclusive. Desirably, the macroscopic scaffold is pre-shaped to interfit a tissue defect. In one desirable embodiment, the tissue defect is in a joint, such as a knee, hip, shoulder, wrist, finger, ankle, toe, elbow, or neck. Desirably the tissue is an epithelial, connective, muscular, or nervous tissue. In one desirable embodiment, the cartilage is articular, costal, fibrous, hyaline, semilunar, thyroid, or elastic cartilage. In another desirable embodiment, the scaffold does not elicit an adverse immune or inflammatory response.

It is also contemplated that the methods of the present invention may be used to repair an injury to an organ, muscle, or other body structure or to form an organ, muscle, or other body structure. Desirable organs include the bladder, brain, nervous tissue, esophagus, fallopian tube, heart, pancreas, intestines, gallbladder, kidney, liver, lung, ovaries, prostate, spinal cord, spleen, stomach, testes, thymus, thyroid, trachea, ureter, urethra, and uterus.

In one embodiment of any of the aspects of the invention, specifically excluded are those macroscopic scaffolds encapsulating chondrocytes as the only encapsulated cell type or those macroscopic scaffolds encapsulating at least one chondrocyte in the absence of other cell types. In yet another embodiment, macroscopic scaffolds that include n-KLDLKLDLKLDL-c as their only peptide component or that include at least one n-KLDLKLDLKLDL-c peptide are specifically excluded. In still another embodiment, specifically excluded are those macroscopic scaffolds encapsulating a solution that does not include insulin, transferrin, or selenium. In yet another embodiment, macroscopic scaffolds encapsulating a solution that includes insulin, transferrin, or selenium or that includes 1% ITS medium are excluded. In another embodiment, specifically excluded are compression schemes that include subjecting the scaffold to pressure for one hour and incubating the scaffold in the absence of added pressure for between one and seven hours, inclusive. In another embodiment, specifically excluded are compression schemes that include four cycles of subjecting the scaffold to pressure for 45 minutes and incubating the scaffold in the absence of added pressure for five hours and 15 minutes, followed by 24 hours of no added pressure.

By "scaffold" is meant a three-dimensional structure capable of encapsulating cells. The beta-sheet secondary structure of the scaffold may be confirmed using standard circular dichroism to detect an absorbance minimum at approximately 218 nm and a maximum at approximately 195 nm. Desirably, the scaffold is formed from the self-assembly of peptides that include L-amino acids, D-amino acids, natural amino acids, non-natural amino acids, or a combination thereof. If L-amino acids are present in the scaffold, degradation of the scaffold produces amino acids which may be reused by the host tissue. It is also contemplated that the peptides may be covalently linked to a compound, such as a chemoattractant or a therapeutically active compound. The peptides may be chemically synthesized or purified from natural or recombinant sources, and the amino- and carboxy-termini of the peptides may be protected or not protected. The peptide scaffold may be formed from one or more distinct molecular species of peptides which are complementary and structurally compatible with each other. Peptides containing mismatched pairs, such as the repulsive pairing of two similarly charged residues from adjacent peptides, may also form scaffolds if the disruptive force is dominated by stabilizing interactions between the peptides.

Although the practice of the transdifferentiation aspects of the invention to date has involved encapsulation of cells within scaffolds, the possibility that cells may become permissive to instruction by differentiation-enhancing agents when grown on the surface of a peptide structure or membrane may be employed in combination with the three-dimensional distribution of cells within the scaffold. Thus the invention also includes growing progenitor cells and their progeny on the surface of peptide hydrogel scaffolds, wherein the peptide scaffold renders the progenitor cells and/or their progeny permissive for instruction by differentiation-enhancing agents when done in combination with cells dispersed in three-dimensions within the scaffolds.

Scaffolds are also referred to herein as peptide structures, peptide hydrogel structures, peptide gel structures, or hydrogel structures.

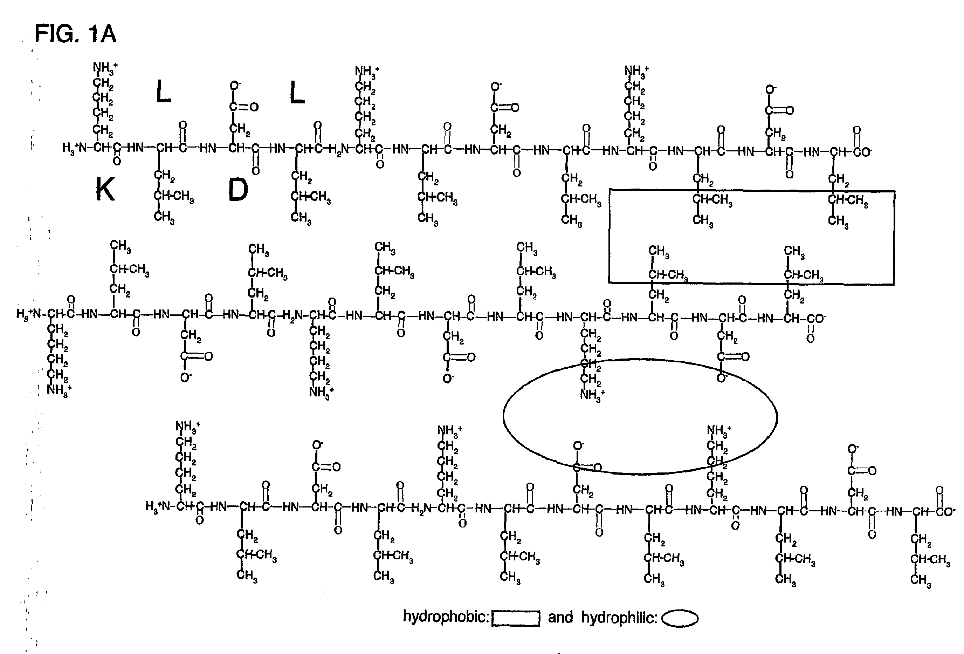

By "complementary" is meant the capable of forming ionic or hydrogen-bonding interactions between hydrophilic residues from adjacent peptides in the scaffold, as illustrated in Fig. 1A. Desirably, each hydrophilic residue in a peptide either hydrogen-bonds or ionically pairs with a hydrophilic residue on an adjacent peptide or is exposed to solvent.

By "structurally compatible" is meant capable of maintaining a sufficiently constant interpeptide distance to allow scaffold formation. Desirably the variation in the interpeptide distance is less than 4, 3, 2, or 1 Å. It is also contemplated that larger variations in the interpeptide distance may not prevent scaffold formation if sufficient stabilizing forces are present. This distance may be calculated based on molecular modeling or based on a simplified procedure that has been previously reported (U.S. Patent Number 5,670,483). In this method, the interpeptide distance is calculated by taking the sum of the number of unbranched atoms on the side-chains of each amino acid in a pair. For example, the interpeptide distance for a lysine-glutamic acid ionic pair is 5+4=9 atoms, and the distance for a glutamine-glutamine hydrogen-bonding pair is 4+4=8 atoms. Using a conversion factor of 3 Å per atom, the variation in the interpeptide distance of peptides having lysine-glutaminic acid pairs and glutamine-glutamine pairs (*i*.*e*., 9 versus 8 atoms) is 3 Å.

By "three-dimensional arrangement" is meant existing in three dimensions. Cells having a three-dimensional arrangement are not all part of the same monolayer. As used herein, a monolayer is a cross section through the peptide scaffold that has a thickness equal to the average diameter of the encapsulated cells and that includes at least one encapsulated cell. In one desirable embodiment, the encapsulated cells are neurons, and the average diameter of the neurons is determined by measuring the average diameter of the cell bodies of the neurons. An encapsulated cell is considered part of the monolayer if at least 51 % of the volume of the cell is contained in the monolayer. Desirably, immediately after scaffold formation, at least one monolayer contains less than 75, 50, 25, 20, 15, 10, 5, or 1% (in order of preference) of the encapsulated cells. More desirably, immediately after scaffold formation, less than 75, 50, 25, 20, 15, 10, 5, or 1% (in order of preference) of the encapsulated cells are part of the same monolayer.

By "substantially uniformly distributed" is meant that immediately after scaffold formation the center of mass of at least 50, 60, 70, 80, 90, or 100% of the cells encapsulated by the scaffold are separated from each other by distances that vary by less than 500, 100, 50, 20, 10, or 1 µM. Desirably, 1, 2, 3, or 4 weeks after scaffold formation, the center of mass of cell clusters or cell pairs for at least 50, 60, 70, 80, 90, or 100% of the encapsulated cells are separated from each other by distances that vary by less than 500, 100, 50, 20, or 10 µM.

By "iso-osmotic solute" is meant a non-ionizing compound dissolved in an aqueous solution.

By "solution that is substantially free of electrolytes" is meant a solution in which no electrolytes have been added or in which the concentration of electrolytes is less than 0.01 or 0.001 mM.

By "Insulin-Transferrin-Selenium medium" or "ITS medium" is meant a medium that includes insulin, transferrin, and selenium. One hundred percent ITS medium ("100% ITS") includes 10 mg/L insulin, 5.5 mg/L transferrin, and 5 mg/L selenium. One percent ITS medium (" 1 % ITS ") includes 0.1 mg/L insulin, 0.055 mg/L transferrin, and/or 0.05 mg/L selenium.

By "nanoscale" is structures having dimensions that can be expressed in terms of nanometers. For example, the term "nanoscale structure" may refer to a structure having a largest dimension of approximately 500 nm or less, approximately 100 nm or less, approximately 50 nm or less, approximately 20-50 nm, approximately 10-20 nm, approximately 5-10 nn, approximately 1-5 nm, approximately 1 nm, or between 0.1 and 1 nm. By "approximately" is meant that the measurement may deviate by 10% from the numeral given, and the ranges listed are assumed to include both endpoints. The relevant dimension may be, *e*.*g*., length, width, depth, breadth, height, radius, diameter, circumference, or an approximation of any of the foregoing in the case of structures that do not have a regular two or three-dimensional shape such as a sphere, cylinder, cube, *etc.* Any other relevant dimension may also be used to determine whether a structure is a nanoscale structure, depending on the shape of the structure.

By "nanofiber" is meant a fiber having a diameter of nanoscale dimensions. Typically a nanoscale fiber has a diameter of 500 nm or less. In some embodiments, a nanofiber has a diameter of less than 100 nm. In other embodiments, a nanofiber has a diameter of less than 50 nm. In some other embodiments, a nanofiber has a diameter of less than 20 nm. According to certain other embodiments, a nanofiber has a diameter of between 10 and 20 nm. In some other embodiments, a nanofiber has a diameter of between 5 and 10 nm. In other embodiments, a nanofiber has a diameter of less than 5 nm.

By "nanoscale environment scaffold" is meant a a scaffold comprising nanofibers. In some embodiments, at least 50% of the fibers comprising the scaffold are nanofibers. In some embodiments, at least 50% of the fibers comprising the scaffold are nanofibers. In some embodiments, at least 75% of the fibers comprising the scaffold are nanofibers. According to certain embodiments, at least 90% of the fibers comprising the scaffold are nanofibers. In some embodiments, at least 95% of the fibers comprising the scaffold are nanofibers. In some embodiments, at least 99% of the fibers comprising the scaffold are nanofibers. Of course the scaffold may also comprise non-fiber constituents, e.g., water, ions, growth and/or differentiation-inducing agents such as growth factors, therapeutic agents, or other compounds.

By "microscale" is meant structures having dimensions that may most conveniently be expressed in terms of micrometers. For example, the term "microscale structure" may refer to a structure having a largest dimension of approximately 500 µm or less, approximately 100 µm or less, approximately 50 µm or less, approximately 20-50 µm, approximately 10-20 µm, approximately 5-10 µm, approximately 1-5 µm, approximately 1 µm, or between 0.1 and 1 µm.

By "microfiber" is meant a a fiber having a diameter of microoscale dimensions. Typically a microscale fiber has a diameter of 500 µm or less, a diameter of less than 100 µm, a diameter of less than 50 µm, a diameter of less than 20 µm, a diameter of between 10 and 20 µm, or a diameter of between 5 and 10 µm.

By "therapeutically active" is meant useful for the treatment, stabilization, or prevention of a disease or condition. Exemplary therapeutically active compounds modulate cell growth, differentiation, cell death, migration, adhesion, interactions with other proteins, enzymatic activity, protein phosphorylation or dephosphorylation, transcription, and/or translation.

The present invention provides a number of advantages related to the repair or replacement of tissues. For example, these methods enable the encapsulation of living cells by a peptide scaffold in a three-dimensional arrangement and in a substantially uniform distribution, which may promote the viability and proliferation of the cells. The cells are present in an architecture that more closely approximates the natural arrangement of cells in the body than would otherwise occur in culture in a traditional plastic culture dish or other two-dimensional substrate. As demonstrated by electron microscopy, the peptide scaffolds comprise a network of nanofibers with intervening spaces rather than a solid matrix. Such a structure may allow cell penetration and cell-cell interaction in a way that more closely resembles the setting of cells within the body than allowed by other culture techniques and materials. The ability of cells to adhere to a substrate may influence cell morphology (see, *e*.*g*., Powers M. et al., Biotech. and Bioeng. 53, 415-426, 1997).

The peptide scaffolds also have the advantage of not eliciting a detectable immune or inflammatory response in mammals. Further, the peptide scaffolds exhibited no detectable swelling when the scaffold was added to a saline solution. This lack of swelling is probably due to the high water content of the scaffold (>99%). This unique property of the scaffold reduces the probability of an unregulated expansion of the scaffold that could lead to adverse physiological effects on neighboring tissues. Moreover, if desired, the *in vivo* rate of degradation of the scaffolds may be modulated by the incorporation of protease cleavage sites into the scaffold. The secretion of extracellular matrix components by the encapsulated chondrocytes increased the stiffness of the scaffold by over 50-fold, improving the ability of the scaffold to be used to replace endogenous cartilage. Furthermore, adding growth factors to the scaffold to stimulate the chondrocytes, compressing the scaffold, or increasing the initial concentrations of the cells or peptides may further increase the stiffness of the scaffold.

The peptide scaffolds described herein are significantly different from other biopolymer-based biomaterials for several reasons. Most biomaterials have fiber sizes diameters generally in the 10-20 micron range (microscale), similar in scale to the size of many types of cells. When grown in an environment comprising such microscale fibers, cells attach to the microfiber with a curvature. Cells grown in a typical biopolymer-based biomaterial are also less hydrated than when grown in the nanoscale environment created by the self assembling peptide scaffolds described herein. In certain embodiments, the self-assembling peptides described herein are approximately 5 nm in length and approximately 1 nm in diameter. Such peptides undergo self-assembly to form nanofibers (*e*.*g*., fibers having a diameter of approximately 10-20 nm). While not wishing to be bound by any theory, inventors suggest that in such an environment cells truly experience three dimensional spatial enclosures on a scale that is relevant to cellular dimensions. The peptides undergo self-assembly to form nanofibers that are highly hydrated (e.g., up to 99.5-99.9%% (1-5 mg/ml) water). Because the scaffold has such an extremely high water content, cells can freely migrate and form intercellular contacts and structures such as the spheroids described herein. Such environment also permits diffusion of small molecules including proteins and signaling molecule exchanges.

Other features and advantages of the invention will be apparent from the following detailed description and from the claims.

### Brief Description of the Drawings

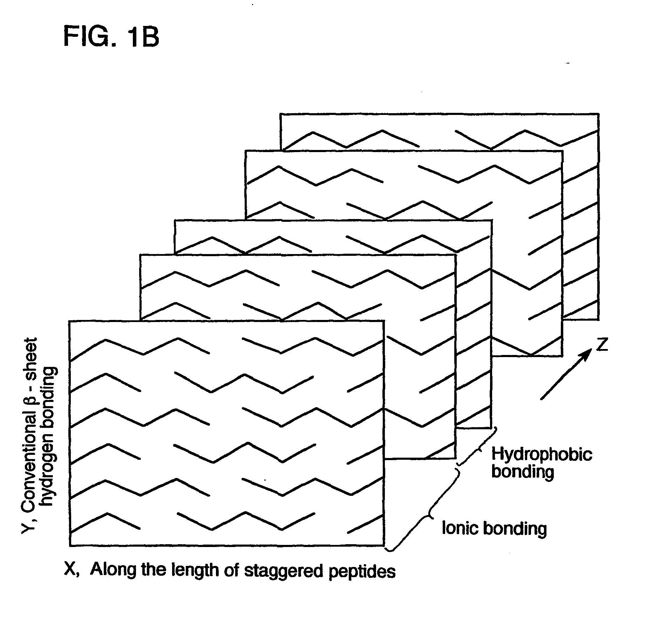

Figs. 1A and 1B are schematic illustrations of the interactions between peptides in the peptide scaffold. Various peptides with amino acid sequences of alternating hydrophobic and hydrophilic residues self-assemble to form a stable scaffold of beta-sheets when exposed to physiologically-equivalent electrolyte solutions (U.S. Patent Numbers 5,955,343 and 5,670,483). The peptide scaffolds are stabilized by numerous interactions between the peptides. For example, the positively charged and negatively charged amino acid side chains from adjacent peptides form complementary ionic pairs, and other hydrophilic residues such as asparagine and glutamine participate in hydrogen-bonding interactions. The hydrophobic groups on adjacent peptides participate in van der Waals interactions. The amino and carbonyl groups on the peptide backbone also participate in intermolecular hydrogen-bonding interactions.

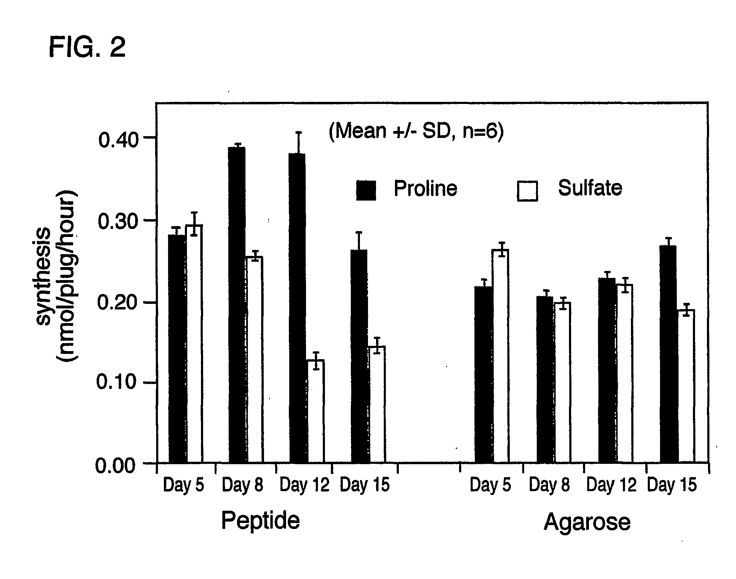

Fig. 2 is a bar graph showing the rates of protein and proteoglycan synthesis in chrondrocytes encapsulated by a peptide scaffold compared to the corresponding rates in chrondrocytes suspended in an agarose gel.

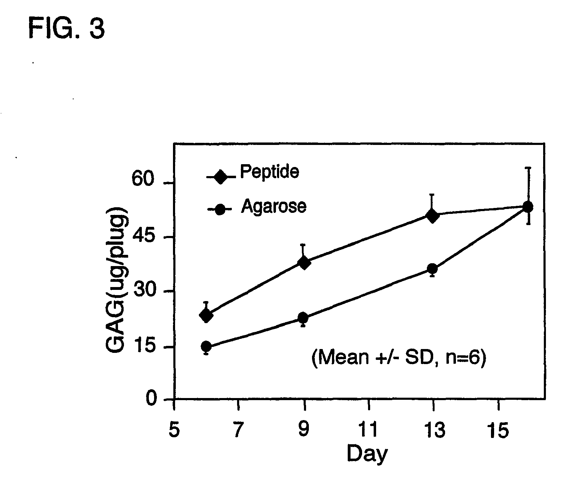

Fig. 3 is a graph showing the amount of glycosaminoclycan (GAG) synthesized by chrondrocytes encapsulated by a peptide scaffold or suspended in an agarose gel.

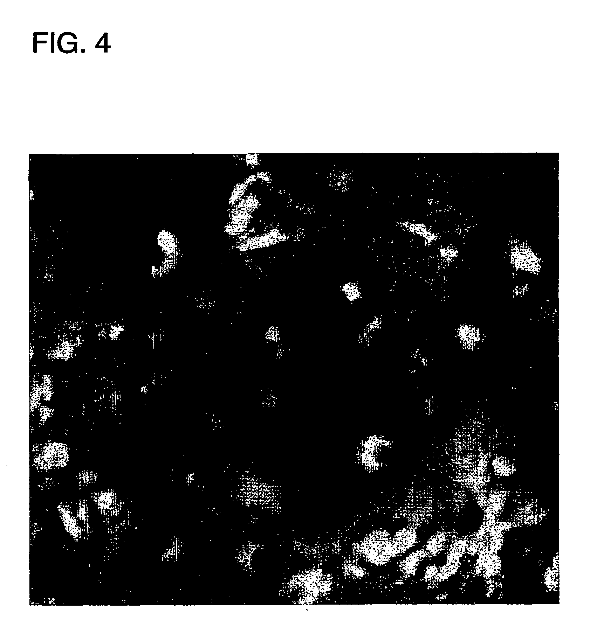

Fig. 4 is a picture of the toluidine blue staining of glycosaminoclycan in a peptide scaffold encapsulating chondrocytes.

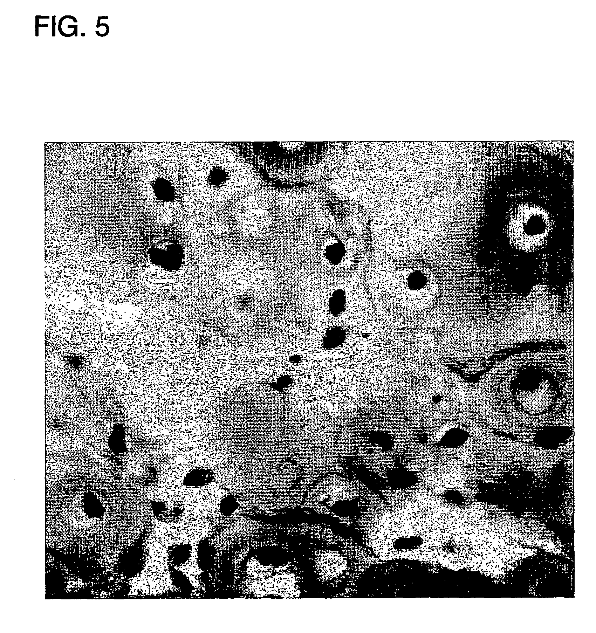

Fig. 5 is a picture of immunohistochemical staining for collagen II in a peptide scaffold encapsulating chondrocytes.

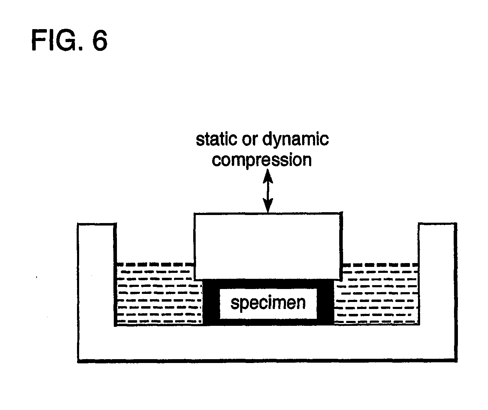

Fig. 6 is a schematic illustration of the apparatus used for mechanical testing of peptide scaffolds and that may be used, if desired, to compress the scaffolds to stimulate the secretion of extracellular matrix components by the encapsulated cells (Buschmann et al., J. of Cell Science 108:1497-1508, 1995)

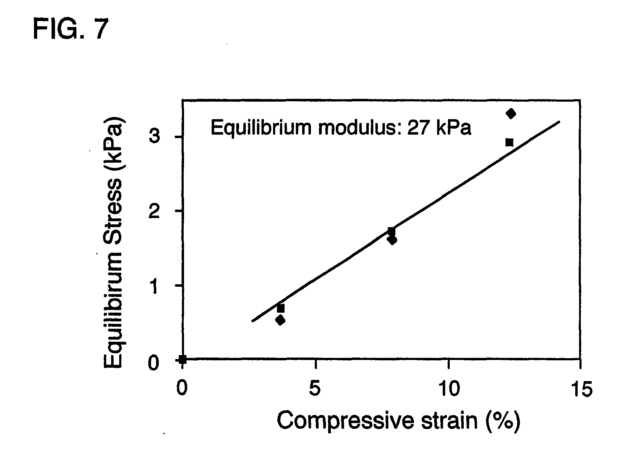

Fig. 7 is a graph of the equilibrium stress values at different compressive strain values for the peptide scaffold encapsulating chrondrocytes. Based on this graph, a equilibrium modulus of 27 kPa was calculated, which is significantly higher than the equilibrium modulus of approximately 0.5 kPa in the absence of cells.

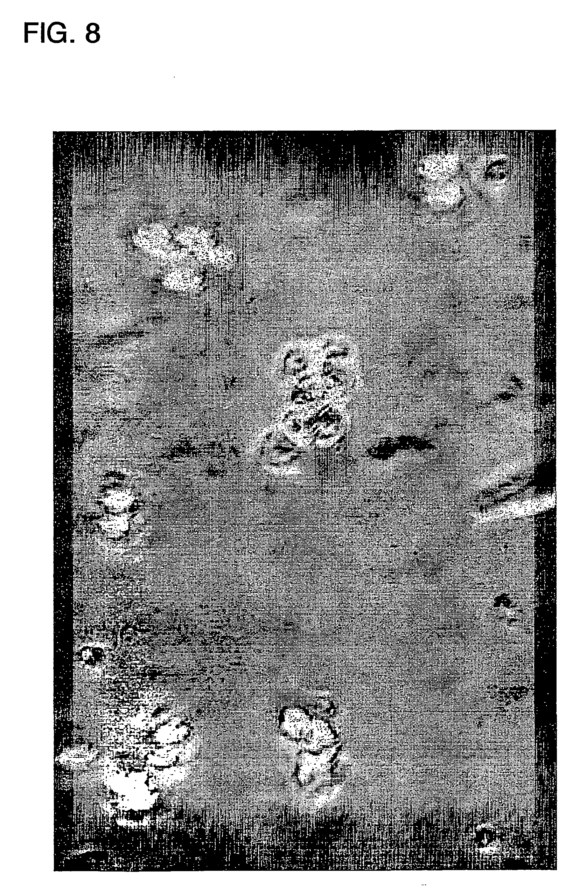

Fig. 8 is a picture showing the distribution of chondrocytes five days after scaffold formation. Each cluster or pair of cells evolved from one cell and is substantially uniformly dispersed.

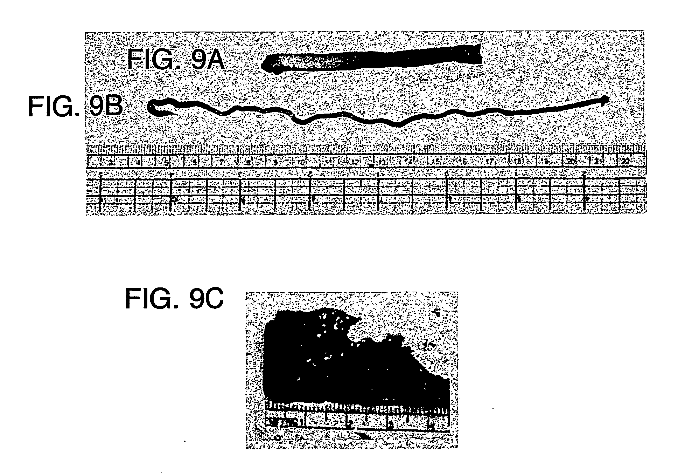

Figs. 9A-9C are pictures of peptide scaffolds formed in a variety of predetermined shapes, including a tape (Fig. 9A), a rope (Fig. 9B), and a sheet (Figs. 9C) (Holmes et al., PNAS 97:6728-6733, 2000). To form a tape-shaped macroscopic peptide scaffold, the RAD16-II peptide was dissolved in water and injected through a device, which consisted of two pieces of thin wire spaced 5 mm apart and sandwiched between two glass slides, into phosphate-buffered saline (PBS). The scaffold was then stained with Congo red. The tape shown in Fig. 9A is approximately 8 cm in length, 0.5 cm in width, and 0.3 mm in thickness. A similar procedure was also used to form a peptide scaffold in the shape of a rope. In this case, the aqueous peptide solution was introduced into PBS using a 3-mL syringe. The peptide scaffold rope shown in Fig. 9B is 18 cm in length and 2 mm in diameter. As illustrated in Fig. 9C, a peptide scaffold was also fabricated in the shape of a sheet. A centimeter scale is shown below the sheet structure in Fig. 9C.

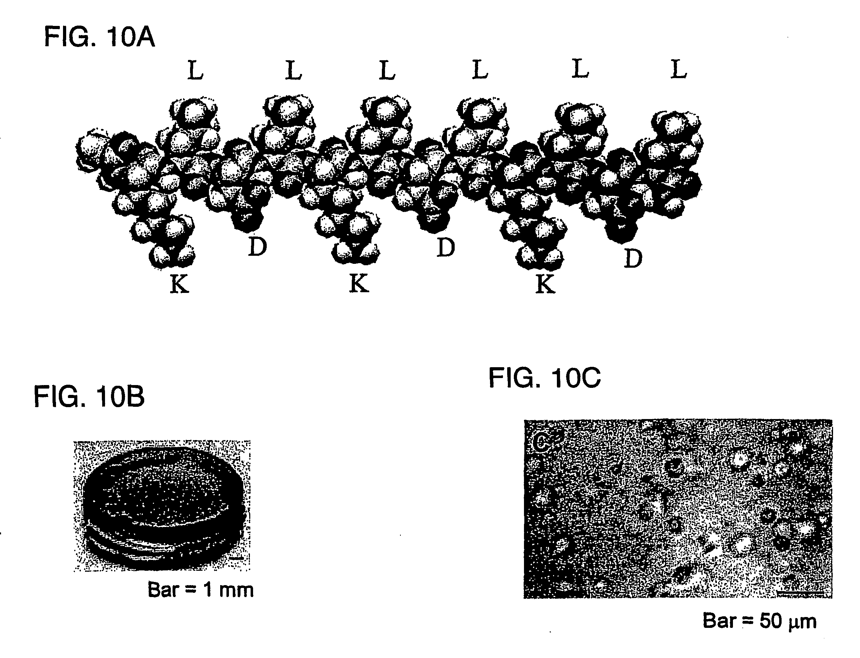

Fig. 10A is a molecular model of a single KLD-12 self-assembling peptide. The alternating hydrophobic and hydrophilic residues on the backbone promote beta-sheet formation. The positively charged lysines (K) and negatively charged aspartic acids (D) are on the lower side of the beta-sheet, and the hydrophobic leucines (L) are on the upper side. This molecular structure facilitates self-assembly through intermolecular interactions. Fig. 10B is a picture of a 12 mm chondrocyte-seeded peptide scaffold plug, punched from 1.6 mm thick slabs. Fig. 10C is a light microscope image of chondrocytes encapsulated in a peptide scaffold.

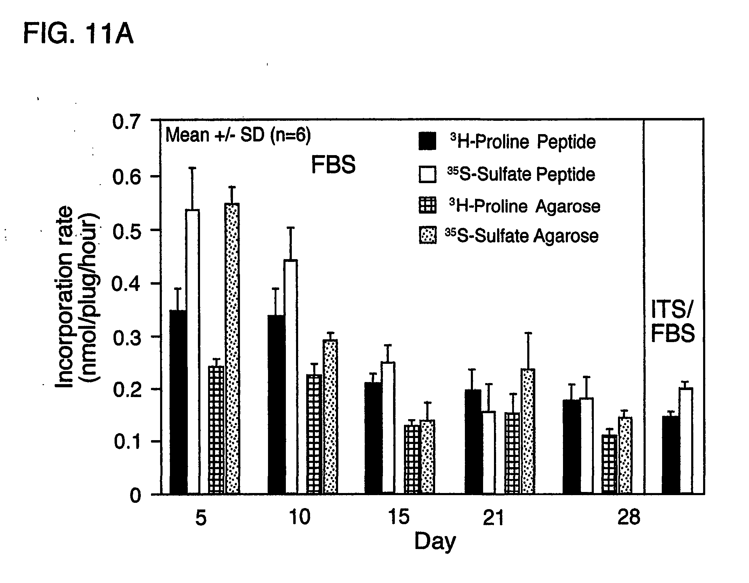

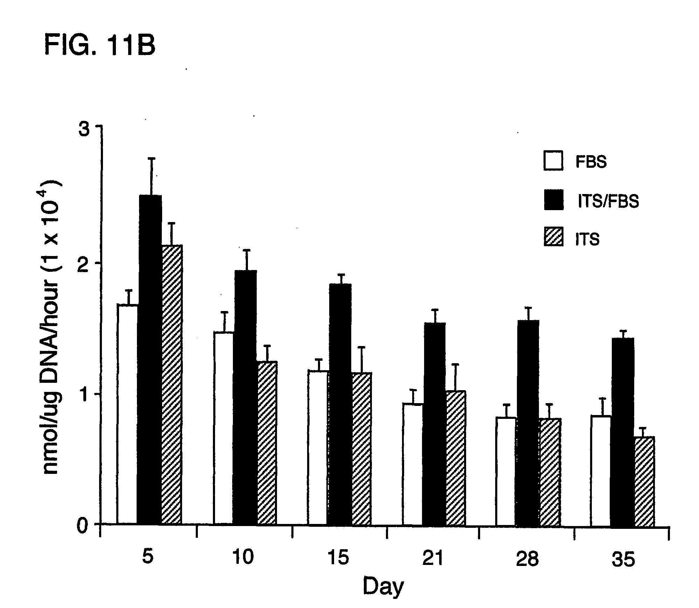

Fig. 11A is a bar graph of the radiolabel incorporation of ³H-proline and ³⁵S-sulfate as measures of the synthesis of proteins and sulfated proteoglycans, respectively, by chondrocytes within cell-seeded peptide scaffolds. In similar experiments using the parent calf cartilage explants from which our chondrocytes were obtained, ∼75-80% of the ³H-proline was incorporated into newly synthesized collagen molecules while essentially all of the macromolecular ³⁵S-sulfate incorporation was associated with sulfated proteoglycans (Sah *et al*., *supra*). Chondrocyte-seeded agarose scaffolds were analyzed in parallel as a well-defined reference chondrocyte culture system. ITS medium supplement is Insulin, Transferrin, and Selenium (Sigma Chemical). Fig. 11B is a bar graph of radioactive proline incorporation in an agarose hydrogel.

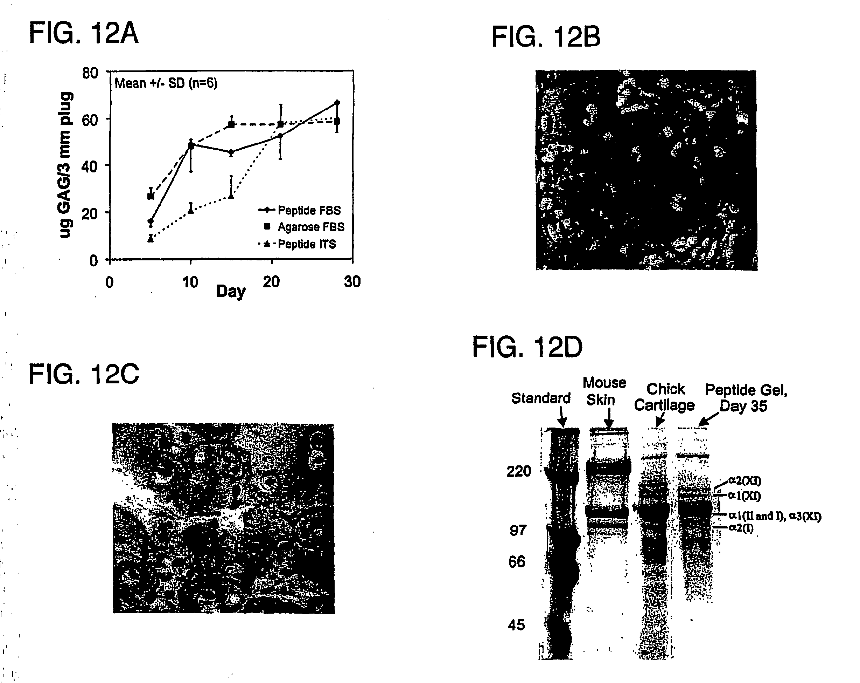

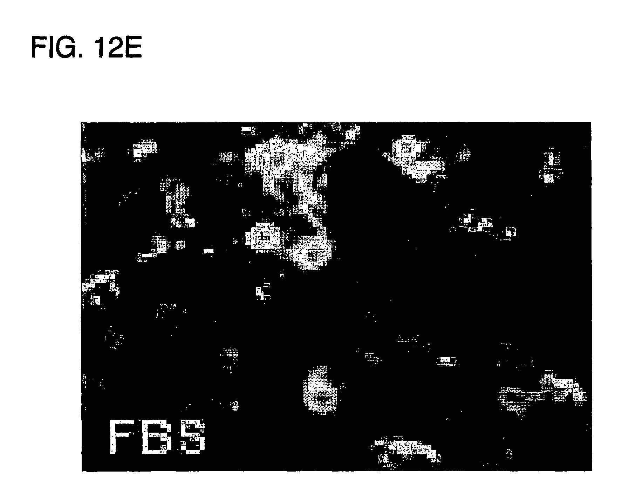

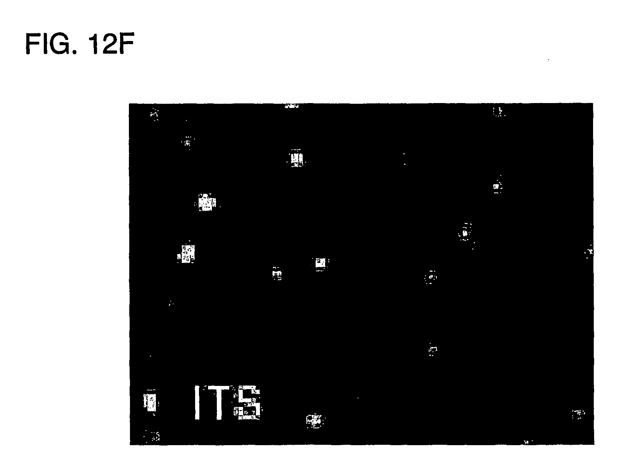

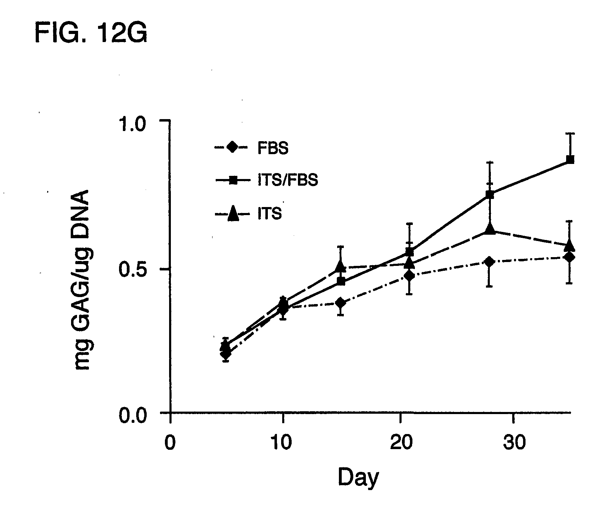

Figs. 12A-12F illustrate matrix accumulation in chondrocyte-seeded peptide scaffold. Fig. 12A is a graph of total glycosaminoglycan (GAG) accumulation in cell-seeded peptide scaffold cultured in FBS and ITS/FBS medium. A chondrocyte-seeded agarose hydrogel was analyzed in parallel as a well-defined reference chondrocyte culture system. Fig. 12B is a picture of toluidine blue staining of a chondrocyte-seeded peptide scaffold cultured in 10% FBS on day 15. Fig. 12C is a picture of immunohistochemical staining for type II collagen in a cell-seeded peptide scaffold cultured in 10% FBS on day 15. Fig. 12D is a picture of an SDS-PAGE gel of collagens extracted from day 35 samples of chondrocyte-seeded peptide scaffold cultured in 1% ITS with 0.2% FBS. Chick cartilage was used as a standard for the migration of collagen II and XI. Mouse skin was used as a standard for collagen I alpha helix 2, which is indicative of collagen expression of a de-differentiated, fibroblastic phenotype rather than the desired differentiated chondrocyte phenotype. Figs. 12E and 12F are pictures of immunohistochemical staining for type II collagen in a cell-seeded peptide scaffold cultured in FBS or ITS medium on day 13, respectively. Fig. 12G is a graph of GAG accumulation in an agarose hydrogel.

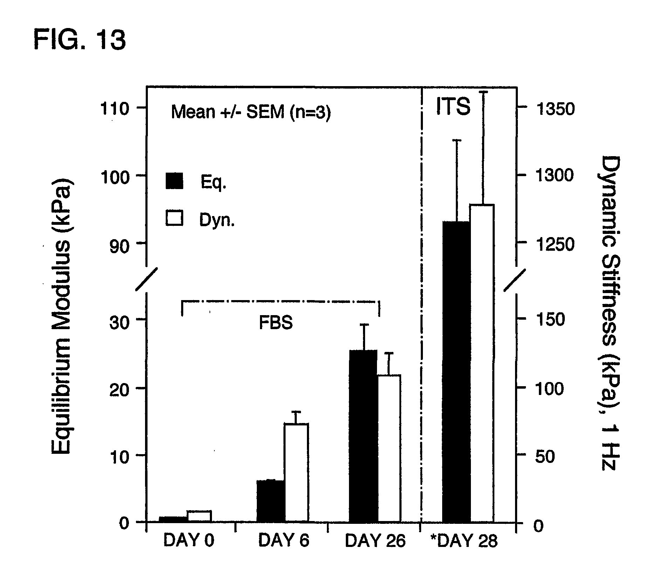

Fig. 13 is a bar graph of the mechanical properties of chondrocyte-seeded peptide scaffolds. The equilibrium modulus and dynamic stiffness were measured in uniaxial confined compression for gels initially seeded at a density of 15 x 10⁶ cells/ml and cultured in 10% FBS medium and for gels seeded at a density of 30 x 10⁶ cells/ml cultured in ITS/FBS medium (*). The later conditions resulted in markedly increased stiffness values, as high as 20-33% that of human and animal cartilages.

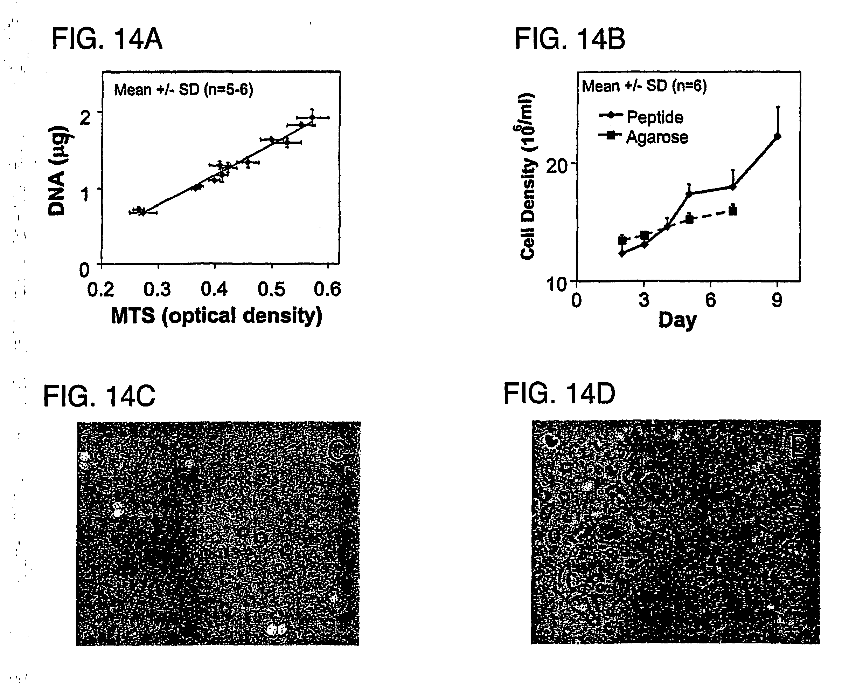

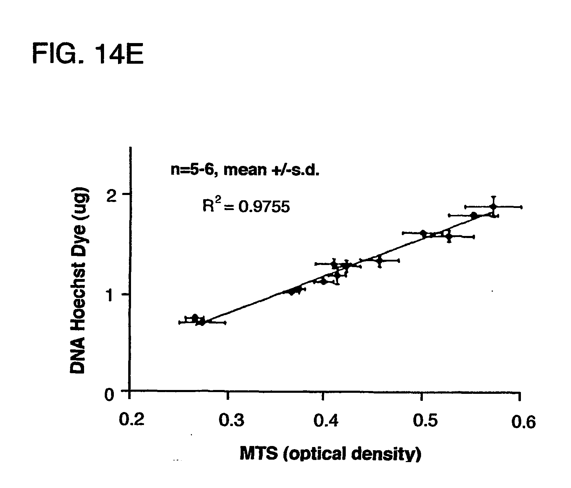

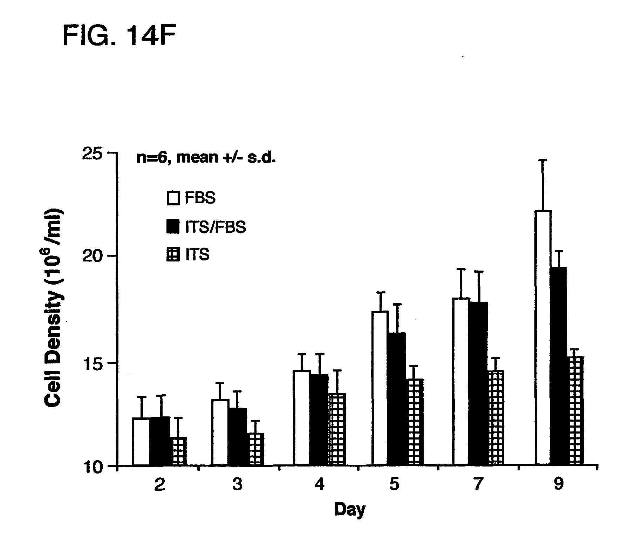

Figs. 14A-14F illustrate the proliferation of chondrocytes seeded in a peptide scaffold. Figs. 14A and 14E are calibration curves for the MTS assay in chondrocyte-seeded agarose hydrogel. Fig. 14B is a graph of the MTS measurement of the density of cells in chondrocyte-seeded peptide scaffolds. A chondrocyte-seeded agarose hydrogel was analyzed in parallel as a well-defined reference chondrocyte culture system. Fig. 14C and 14D are pictures illustrating BrdU incorporation during a 24 hour period ending on 3 days (Fig. 14C) and 7 days (Fig. 14D) after seeding of peptide scaffolds with chondrocytes. Fig. 14F is a bar graph of viable cell density in the peptide scaffold.

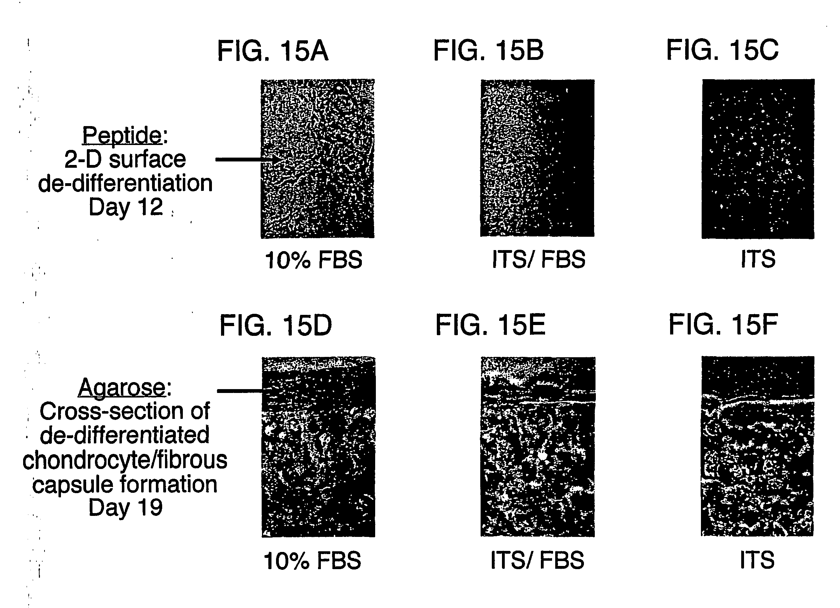

Figs. 15A-15C are pictures illustrating the ability of 1% ITS and 0.2% FBS (ITS/FBS, Fig. 15B) and 1% ITS (ITS, Fig. 15C) medium to limit or eliminate, respectively, chondrocyte migration to the peptide scaffold surface and subsequent de-differentiation to a fibroblast phenotype and proliferation on day 12. Figs. 15D-15F are cross-section pictures of de-differentiated chondrocyte/fibrous capsule formation on day 19 in agarose hydrogels.

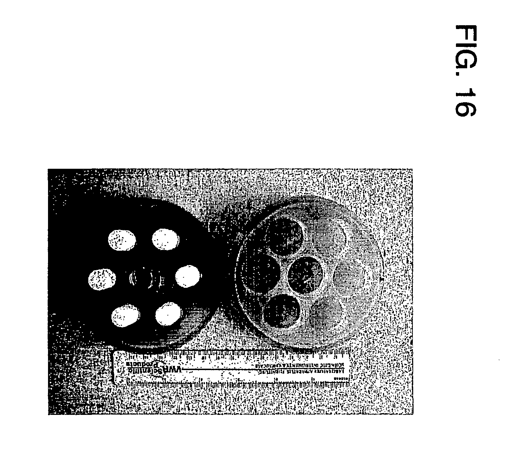

Fig. 16 is a picture of the compression camber (top) and lid (bottom) used for compressive loading.

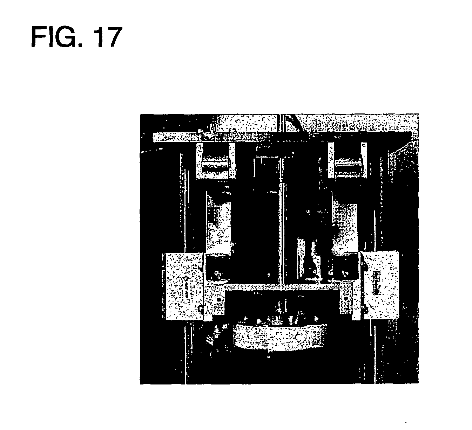

Fig. 17 is a picture of an incubator-housed loading system.

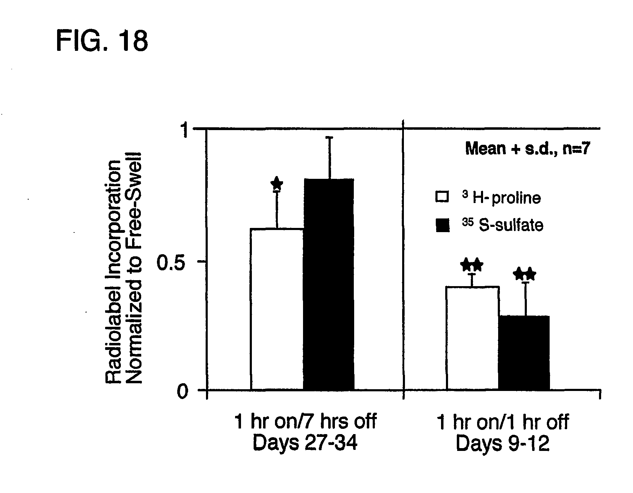

Fig. 18 is a bar graph of radiolabel incorporation in agarose hydrogels after repeated duty cycles for eight or four days.

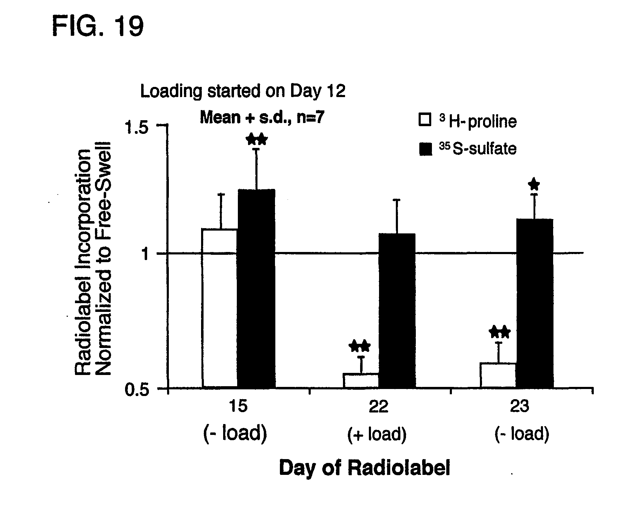

Fig. 19 is a bar graph of radiolabel incorporation in agarose hydrogels using alternate day loading.

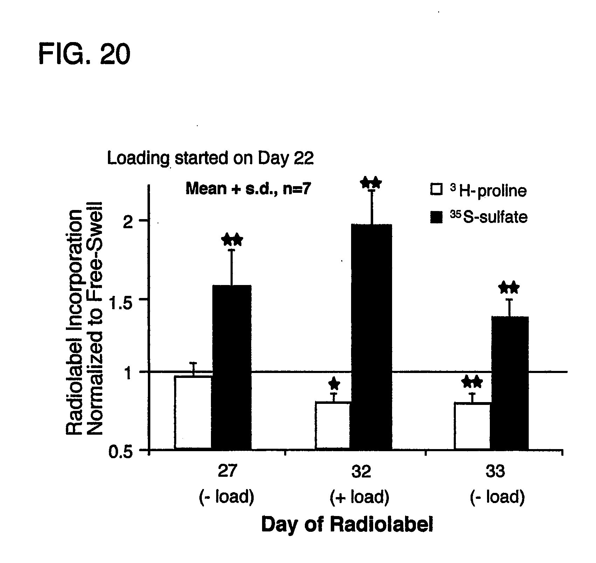

Fig. 20 is a bar graph of radiolabel incorporation in peptide scaffolds using alternate day loading.

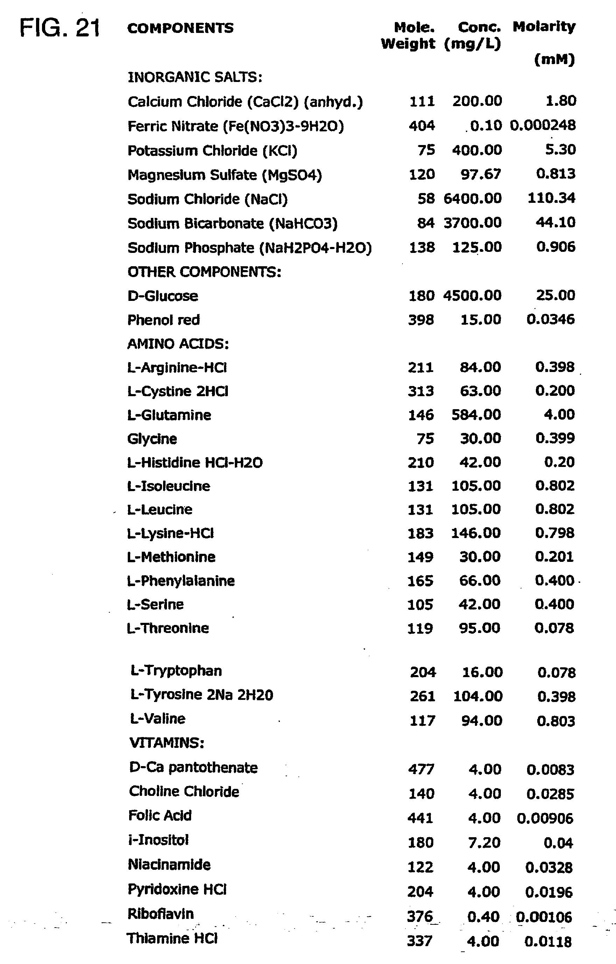

Fig. 21 is a list of components in 1X DMEM medium.

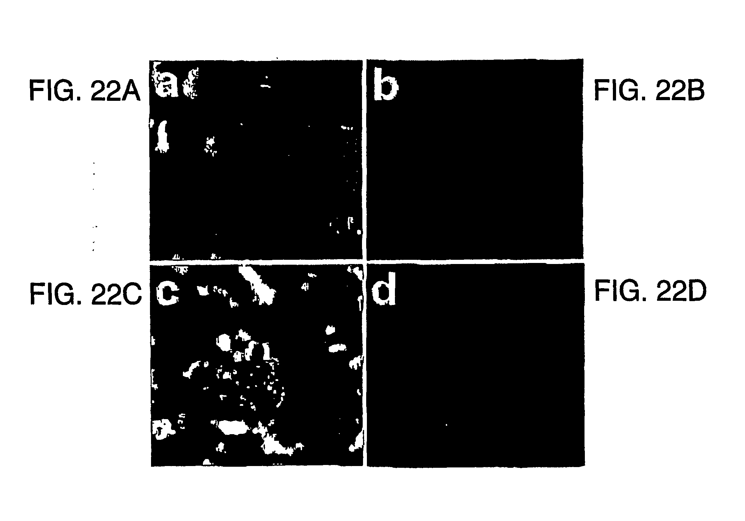

Figs. 22A-22D show encapsulation of adult LPCs in an assembled peptide structure. Fig. 22A shows encapsulated LPCs immediately after encapsulation. Fig. 22B shows encapsulated LPCs two days after encapsulation. Fig. 22C shows spheroid formation by adult LPCs encapsulated in an assembled peptide structure four to five days after encapsulation. Fig. 22D shows the same spheroid as in Figure 3c, after staining for incorporation of BrdU into DNA.

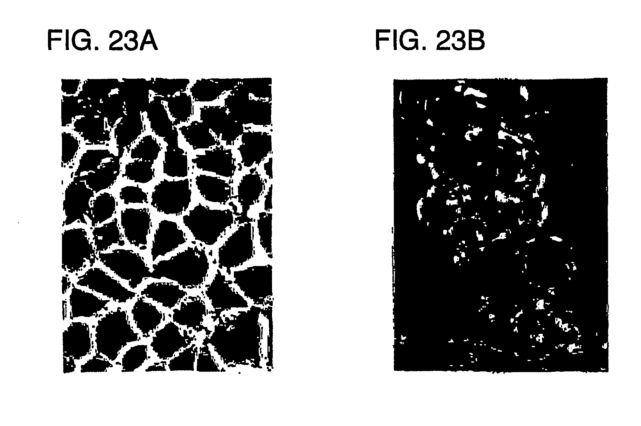

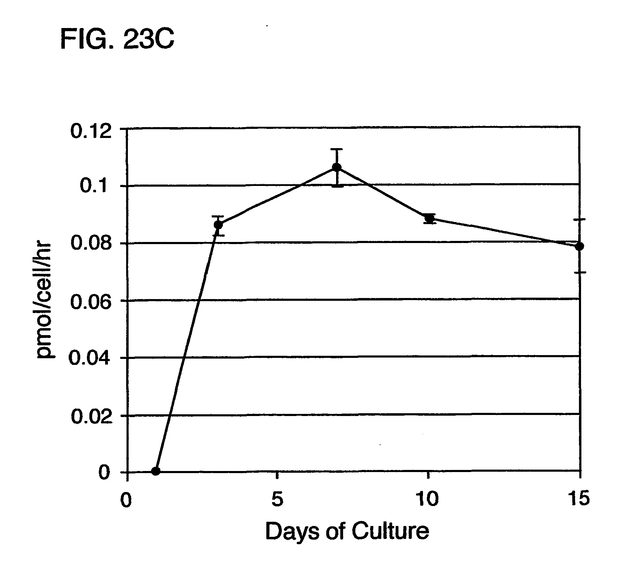

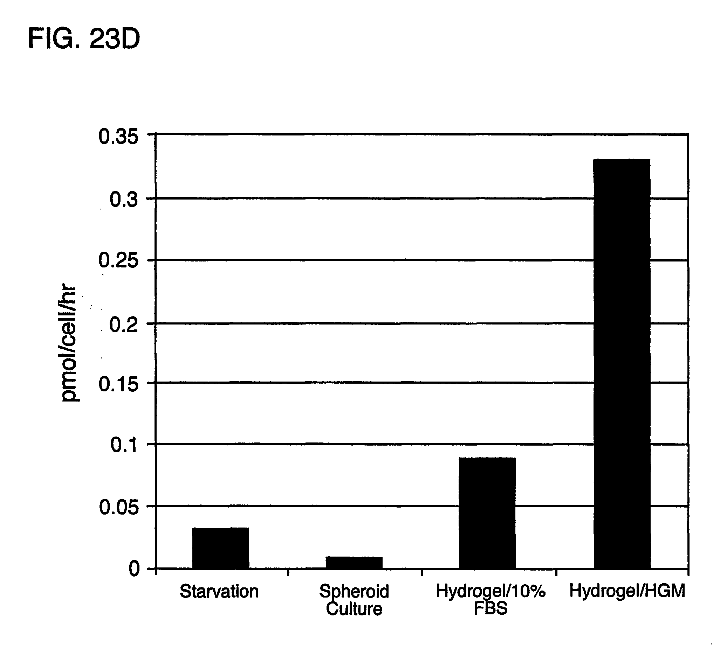

Figs. 23A-23D present data on CYP1A1 activity of LPCs growing under various culture conditions. Fig. 23A shows CYP1A1 activity of LPCs growing as a monolayer on a standard plastic culture dish. Fig. 23B shows CYP1A1 activity of LPCs spheroids growing in an assembled peptide structure two weeks after encapsulation. Fig. 23C is a graph showing CYP1A1 activity of LPC spheroids growing in an assembled peptide structure during a time course of two weeks, starting 24 hours after encapsulation. Fig. 23D is a graph showing comparative CYP1A1 activity ofLPCs maintained under various culture conditions: monolayer on plastic dish with low (1%) serum concentration (serum starvation); spheroid culture obtained by growing LPCs in liquid culture at high density; spheroid culture in assembled peptide structure growing in DMEM with 10% FBS; spheroid culture in assembled peptide structure growing in HGM.

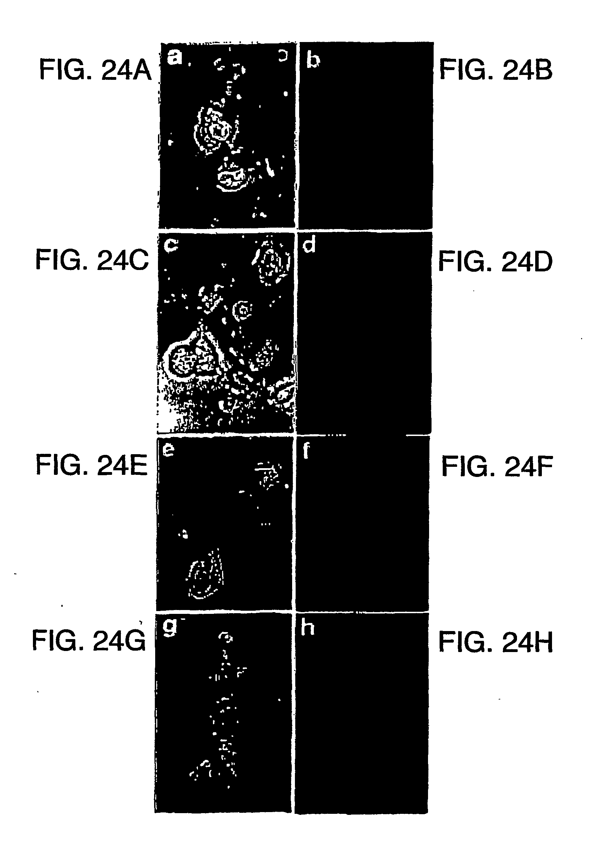

Figs. 24A-24H show LPCs growing in an assembled peptide structure after staining for various neuronal markers. Fig. 24B shows LPCs stained for Nestin. Fig. 24D shows LPCs stained for β-tubulinIII. Fig. 24F shows LPCs stained for NeuN. Fig. 24H shows LPCs stained for GFAP. Figures 24A, 24C, 24E, and 24G are corresponding phase contrast images.

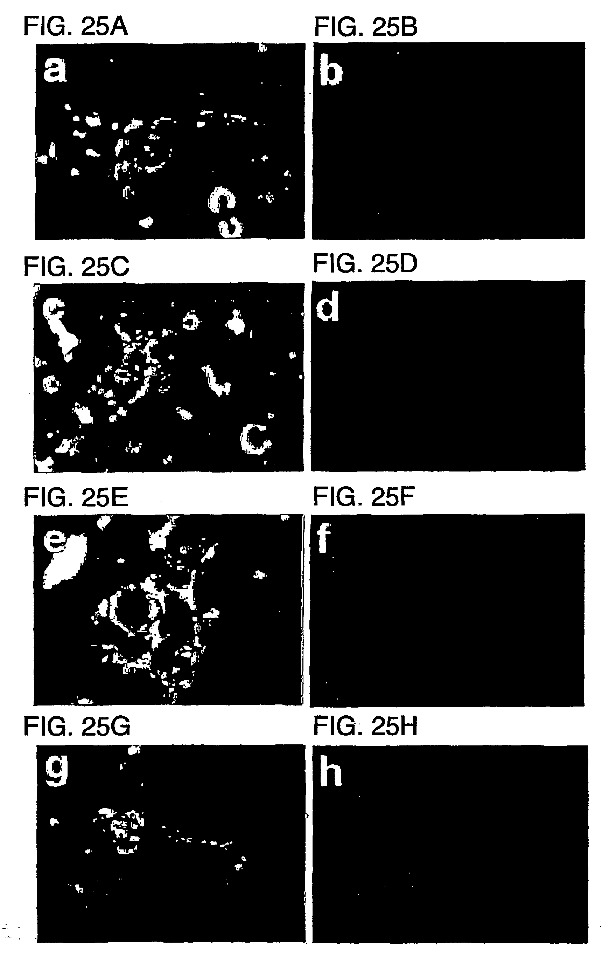

Figs. 25A-25H show Nestin (25B, 25F) and BrdU (25D, 25H) staining of neuronal-precursor-like cells arising from LPC clusters cultured in an assembled peptide structure in HGM in the presence of EGF (25A-25D) or EGF plus NGF (25E-25H). 25A, 25C, 25E, and 25G are phase contrast images of 25B, 25D, 25F, and 25H.

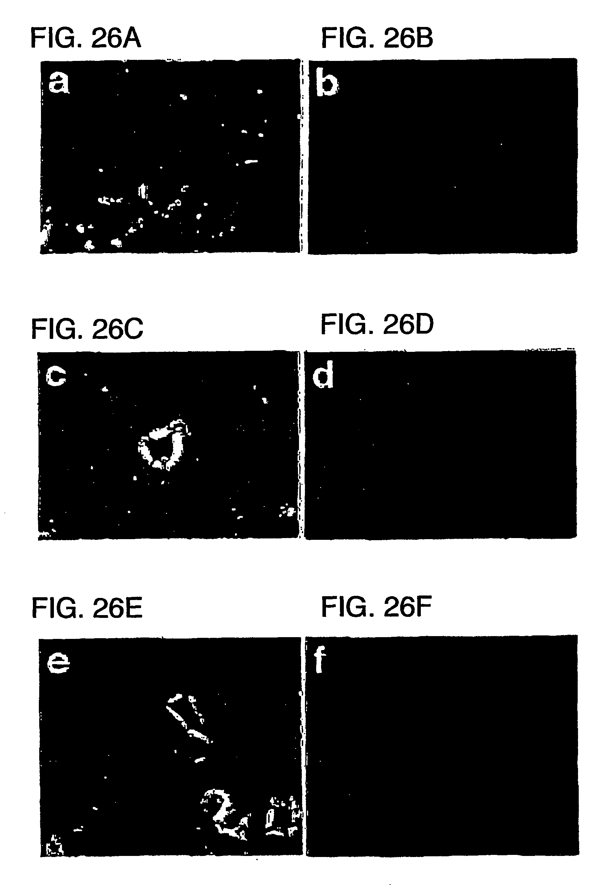

Fig. 26A-26F show the phenotype of LPC cells cultured on laminin-coated plates in HGM containing EGF plus NGF one week after extraction from assembled peptide structures. Fig. 26A shows cells that assumed a classical hepatocyte shape. Fig. 26B shows the same cells as in 26a after staining for CYP1A1 activity. Fig. 26C shows cells that assumed a flat, expanded shape with some processes. Fig. 26D shows the same cells as in 26c after staining for GFAP. Fig. 26E shows another example of cells that assumed a flat, expanded shape with some processes. Fig. 26F shows the same cells as in 26e after staining for GFAP.

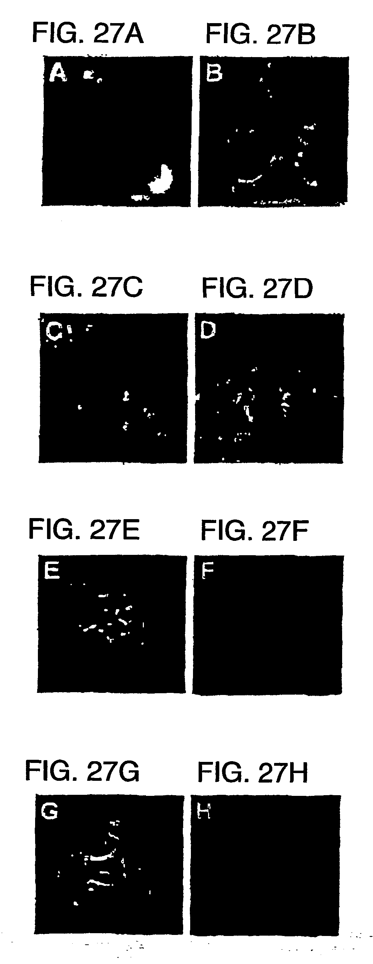

Figs. 27A-27H show analysis of LPC cell division on conventional culture dish or after encapsulation in peptide hydrogel. Fig. 27A is a phase contrast micrograph showing cells immediately after encapsulation. (400X magnification). Fig. 27B is a phase contrast micrograph showing cells 24 hours after encapsulation. (400X magnification). Fig. 27C is a phase contrast micrograph showing cells 48 hours after encapsulation illustrating adoption of a spheroid morphology (approximately 5-6 cells, 400X magnification). Fig. 27D is a phase contrast micrograph showing cells 96 hours after encapsulation illustrating a spheroid containing approximately 10-14 cells. (400X magnification). Fig. 27E shows a phase contrast micrograph of a control colony grown on a conventional tissue culture dish for 48 hours. (200X magnification). Fig. 27F shows the same colony as in 27E immunostained for BrdU. (200X magnification). Fig. 27G shows a phase contrast micrograph of a spheroid colony after 96 hours of growth encapsulated in peptide hydrogel. (200X magnification). Fig. 27H shows the same colony as in 27G immunostained for BrdU. (200X magnification).

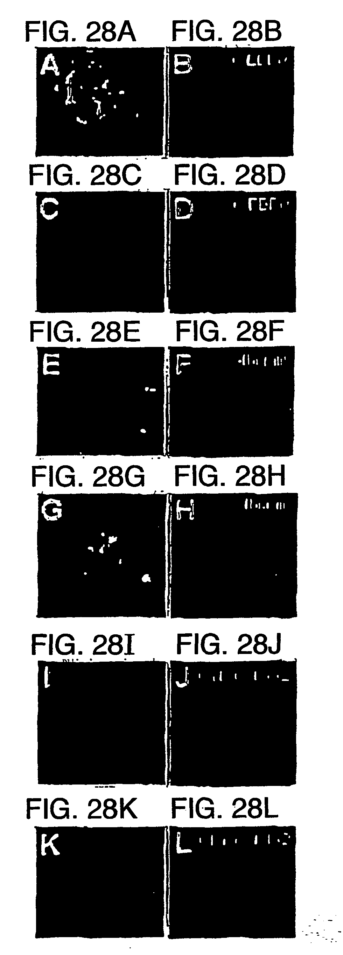

Figs. 28A-28I show phenotypic analysis of LPC cells during exponential growth on conventional culture dish either after isolation from regular culture conditions or after isolation from peptide hydrogel culture. Fig. 28A is a phase contrast micrograph of a control colony. Fig. 28B shows the same cells as in 28A after staining for C/EPBα. Fig. 28C is a phase contrast micrograph of a spheroid containing cells isolated from peptide hydrogel. Fig. 28D shows the same cells as in 28C after staining for C/EPBα. Fig. 28E is a phase contrast micrograph of a control colony. Fig. 28F shows the same cells as in 28E after staining for albumin. Fig. 28G is a phase contrast micrograph of a spheroid containing cells isolated from peptide hydrogel. Fig. 28H shows the same cells as in 28G after staining for albumin. Fig. 28I is a phase contrast micrograph of a control colony. Fig. 28J shows the same cells as in 28G after staining for CYP1A1/1A2. Fig. 28K is a phase contrast micrograph of a spheroid containing cells isolated from peptide hydrogel. Fig. 281L shows the same cells as in 28G after staining for CYP1A1/1A2.

### Detailed Description

We have discovered that the peptide scaffolds described herein surprisingly seem to function for the encapsulation of virtually any cell type. For example, our scaffold systems originally developed for cartilage tissue replacement are capable of supporting a broad range of cell types, such as stem cells, neurons, and liver cells. Thus, these scaffolds are useful for the repair or replacement a variety of tissues and organs.

### Encapsulation of Cells such as Chondrocytes

To maintain their phenotype, chondrocytes are typically cultured in a three-dimensional environment. Within such an arrangement, chrondrocytes develop a mechanically functional extracellular matrix and respond appropriately to static and dynamic compressive loads. Thus, a major challenge in choosing an appropriate scaffold for cartilage repair is the identification of a material that can maintain high rates of proliferation of chondrocytes and high rates of chondrocyte synthesis of phenotypically specific extracellular matrix (ECM) macromolecules until repair evolves into steady state.

We have discovered that a peptide scaffold that encapsulates living cells in a three-dimensional arrangement may be formed by first mixing the cells and the peptides in a solution having the required osmolarity to maintain cell viability, and then adding sufficient electrolytes to initiate self-assembly of the scaffold. Long-term cultures (*e*.*g*., four week cultures) showed that the chondrocytes encapsulated by this scaffold deposited a continuous matrix rich in proteoglycans and type II collagen which are indicative of a stable chondrocyte phenotype, maintained a rounded morphology, and had a significant rate of protein and proteoglycan synthesis. The content of viable differentiated chondrocytes within the peptide scaffold increased at a rate that was 4-fold higher than the corresponding rate for a parallel chondrocyte-seeded agarose culture, a well-defmed reference chondrocyte culture system. These results indicate that a peptide gel scaffold encapsulating chondrocytes may be used to repair or replace cartilage tissue.

Time dependent accumulation of ECM was paralleled by increases in material stiffness, indicative of deposition of mechanically-functional neo-tissue. In particular, secretion of extracellular matrix components by the encapsulated chondrocytes increased the equilibrium modulus, a measure of the strength of the scaffold, by over 50-fold by day 28 after scaffold formation. Dynamic stiffness at 1 Hz also increased with time in culture, with day 26 values about 5% of those of native tissue. Additionally, cells seeded into peptide scaffolds at 30 x 10⁶ cells/ml and cultured in ITS/FBS medium showed an even more profound increase in compressive stiffness. The equilibrium modulus reached 93 kPa by day 28, and the dynamic stiffness at 1 Hz reached 1.28 MPa. Both of these values are approximately 1/5 to 1/3 that of native human and animal articular cartilages.

If desired, the stiffness of the scaffold may be further increased by incorporating cysteines which may be disulfide bonded or by incorporating aromatic residues which may be UV cross-linked into the scaffold. In addition, varying the length or concentration of the peptides may further increase the stiffness of the scaffold. Moreover, forming the scaffolds in the presence of growth factors so that they are encapsulated by the scaffold, adding growth factors to the media surrounding the scaffold so that they diffuse into the scaffold, or using standard molecular biology techniques to modify the encapsulated cells so that they express heterologous growth factors or over-express endogenous growth factors is expected to promote the proliferation of the encapsulated cells and to increase the secretion of extracellular matrix components by the cells (van Osch et al., Osteoarthritis Cartilage 6:187-195, 1998). For example, the synthetic manufacturing of peptides allows for the tethering of growth factors to subpopulations of peptide units, enabling controlled concentrations of growth factors to be directly delivered to encapsulated cells. The stiffness of the scaffold may also be increased by culturing it in a bioreactor that increases nutrient transport (Grande et al., J. Biomed. Mater. Res. 34:211-220, 1997; Mizuno *et al*., *supra*; and Vunjak-Novakovic et al., J. Orthop Res. 17:130-138, 1999). Moreover, subjecting the scaffold to external pressure may further enhance the secretion of extracelluar matrix components by the cells, resulting in an even higher equilibrium modulus (Frank et al., J. Biomech. 33:1523-1527, 2000; and Mauck et al., J. Biomech. Eng. 122:252-260, 2000). Also, the stiffness of the scaffold may further increase after it is implanted *in vivo.* Thus, the equilibrium compression modulus of the peptide scaffold may approach the 500 kPA value of articular cartilage.

Additionally, the interaction between chondrocytes and the peptide matrix may be modulated by designing binding sites in the peptide sequence. Furthermore, peptides can be designed to include specific sites to enable enzymatic degradation by matrix metalloproteinases and aggrecanases that are naturally synthesized by chondrocytes. In this manner, spatial and temporal control of scaffold biodegradability may be obtained. Such flexibility offers a broad spectrum of opportunities to apply multiple approaches for generating the appropriate tissue repair response.

In summary, our study demonstrates the ability of a self-assembling peptide scaffold to maintain differentiated chondrocytes and stimulate the synthesis and accumulation of a mechanically functional cartilage-like ECM in a 3-dimensional cell culture. The peptide used in this study (KLD12) represents one of a class of specially designed self-assembling peptides made through molecular engineering and characterized by a set of unique features including (i) design at the single amino acid level, using individual units that can be tailored to the specific cell and tissue application of interest, as well as cell signaling and biodegradability, (ii) self-assembly initiated by standard culture medium to form a network of 10-20 nm diameter nanofibers with 50-200 nm pores containing 99%-99.9% water (*i*.*e*., a network of nanofibers that are almost 3 orders of magnitude smaller than most polymer microfibers), and (iii) self assembly of the scaffold by addition of standard culture medium without any toxic processing procedures.

### Encapsulation of a diverse range of cell types

Because these peptide scaffolds have been previously shown to be nontoxic to a variety of mammalian cell types, the methods of the present invention may also be applied to other cell types for applications involving other tissue types (Zhang et al., Biomaterials 16:1385-1393, 1995). The strength of scaffolds that is required to repair or replace soft tissues such as young male thigh and forearm skin which have equilibrium compression moduli of 1.99 and 1.51 kPa, respectively, is much lower than that required for cartilage. Additionally, neurons grown in a monolayer on the outside surface of a peptide scaffold have been previously shown to exhibited extensive neurite outgrowth. Thus, neurons that are encapsulated by these peptide scaffolds using the methods of the present invention may project axons that enable cell-to-cell contact between the encapsulated cells and neighboring endogenous neurons.

### Encapsulation and differentiation ofprogenitor cells

Additionally, progenitor cells or stem cells can be encapsulated using any of the peptide scaffolds described herein. If desired, the cells may also be treated with an agent that promotes differentiation of the cells.

As presented in more detail below, a peptide scaffold provided an environment that altered the phenotypes and differentiation pathways adopted by liver progenitor cells (LPCs).cultured therein. For example, when cultured on traditional plastic culture dishes, rat liver progenitor cells maintain a uniform, flat morphology and do not differentiate well into functional hepatocytes. Such cells fail to express significant amounts of cytochrome P4501A1 (CYP1A1), an enzyme produced by mature hepatocytes. In contrast, rat liver progenitor cells cultured in a three-dimensional peptide hydrogel divide to form spheroidal clusters, reminiscent of the behavior of mature hepatocytes under certain culture conditions. Furthermore, a portion of cells maintained in the hydrogels express CYP1A1.

To further investigate this phenomenon, cells in peptide hydrogels were switched to a defined hepatocyte growth medium. Surprisingly, by 24 hours after the medium was changed, a considerable proportion of the cells acquired a dramatic change in cellular morphology, exhibiting very elongated cell bodies with rudimentary processes. The phenotype of these cells resembled that of neuronal lineages. Staining for various markers characteristic of neuronal lineage cells further indicated that the cells were developing along a non-hepatocyte pathway. Results were consistent with a conclusion that these cells possessed features of neuronal precursors.

The following examples are to illustrate the invention. They are not meant to limit the invention in any way.

### Peptide Scaffolds

Certain peptides consisting of alternating hydrophilic and hydrophobic amino acids self-assemble to form an exceedingly stable beta-sheet macroscopic scaffold in the presence of electrolytes, such as monovalent alkaline cations (U.S. Patent Numbers 5,955,343 and 5,670,483; Figs. 1A and 1B). For example, NaCl at a concentration of between 5 mM and 5 M induces the assembly of scaffolds within a few minutes. Lower concentrations of NaC1 may also induce assembly but at a slower rate. The side-chains of the peptides in the scaffold partition into two faces, a polar face with charged ionic side chains and a nonpolar face with alanines or other hydrophobic groups. These ionic side chains are self-complementary to one another in that the positively charged and negatively charged amino acid residues can form complementary ionic pairs. These peptides are therefore called ionic self-complementary peptides, or Type I self-assembling peptides. If the ionic residues alternate with one positively and one negatively charged residue (- + - + - + - +), the peptides are described as "modulus I;" if the ionic residues alternate with two positively and two negatively charged residues (--++--++), the peptides are described as "modulus II."

Many modulus I and II self-complementary peptides with identical compositions and length; such as EAK16, KAE16, RAD16, RAE16, and KAD16; have been analyzed previously (Table 1). Modulus IV ionic self-complementary peptides containing 16 amino acids; such as EAK16-IV, KAE16-IV, DAR16-IV and RAD 1 6-IV; have also been studied. If the charged residues in these self-assembling peptides are substituted (i. *e*., the positive charged lysines are replaced by positively charged arginines and the negatively charged glutamates are replaced by negatively charged aspartates), there are essentially no significant effects on the self-assembly process. However, if the positively charged resides, lysine and arganine are replaced by negatively charged residues, aspartate and glutamate, the peptides can no longer undergo self-assembly to form macroscopic scaffolds; however, they can still form a beta-sheet structure in the presence of salt. Other hydrophilic residues, such as asparagine and glutamine, that form hydrogen-bonds may be incorporated into the peptides instead of, or in addition to, charged residues. If the alanines in the peptides are changed to more hydrophobic residues, such as leucine, isoleucine, phenylalanine or tyrosine, these peptides have a greater tendency to self-assemble and form peptide matrices with enhanced strength. Some peptides that have similar compositions and lengths as the aforementioned peptides form alpha-helices and random-coils rather than beta-sheets and do not form macroscopic structures. Thus, in addition to self-complementarity, other factors are likely to be important for the formation of macroscopic scaffolds, such as the peptide length, the degree of intermolecular interaction, and the ability to form staggered arrays.

**Table 1. Representative Self-Assembling Peptides**

| Name | Sequence (n-->c) | Modulus | Structure |
|---|---|---|---|
| RADA16-I | n-RADARADARADARADA-c | I | β |
| RGDA16-I | n-RADARGDARADARGDA-c | I | r.c. |
| RADA8-I | n-RADARADA-c | I | r.c. |
| RAD16-II | n-RARADADARARADADA-c | II | β |
| RAD8-II | n-RARADADA-c | II | r.c. |
| EAKA16-I | n-AEAKAEAKAEAKAEAK-c | I | β |
| EAKA8-I | n-AEAKAEAK-c | I | r.c. |
| RAEA16-I | n-RAEARAEARAEARAEA-c | I | β |
| RAEA8-I | n-RAEARAEA-c | I | r.c. |
| KADA16-I | n-KADAKADAKADAKADA-c | I | β |
| KADA8-I | n-KADAKADA-c | I | r.c. |
| EAH16-II | n-AEAEAHAHAEAEAHAH-c | II | β |
| EAH8-II | n-AEAEAHAH-c | II | r.c. |
| EFK16-II | n-FEFEFKFKFEFEFKFK-c | II | β |
| EFK8-II | n-FEFKFEFK-c | I | β |
| ELK16-II | n-LELELKLKLELELKLK-c | II | β |
| ELK8-II | n-LELELKLK-c | II | r.c. |
| EAK16-II | n-AEAEAKAKAEAEAKAK-c | II | β |
| EAK12 | n-AEAEAEAEAKAK-c | IV/II | α/β |
| EAK8-II | n-AEAEAKAK-c | II | r.c. |
| KAE16-IV | n-KAKAKAKAEAEAEAEA-c | IV | β |
| EAK16-IV | n-AEAEAEAEAKAKAKAK-c | IV | β |
| RAD16-IV | n-RARARARADADADADA-c | IV | β |
| DAR16-IV | n-ADADADADARARARAR-c | IV | α/β |
| DAR16-IV* | n-DADADADARARARARA-c | IV | α/β |
| DAR32-IV | n-(ADADADADARARARAR)-c | IV | α/β |
| EHK16 | n-HEHEHKHKHEHEHKHK-c | N/A | r.c. |
| EHK8-I | n-HEHEHKHK-c | N/A | r.c. |
| VE20* | n-VEVEVEVEVEVEVEVEVEVE-c | N/A | β |
| RF20* | n-RFRFRFRFRFRFRFRFRFRF-c | N/A | β |

| | | | |
|---|---|---|---|
| "β" denotes beta-sheet; " α" denote alpha-helix; "r.c." denotes random coil; "N/A" denotes not applicable. *Both VE20 and RF20 form a beta-sheet when they are incubated in a solution containing NaCl; however, they do not self-assemble to form macroscopic scaffolds. | | | |

Other self-assembling peptides may be generated by changing the amino acid sequence of any self-assembling peptide by a single amino acid residue or by multiple amino acid residues. Additionally, the incorporation of specific cell recognition ligands, such as RGD or RAD, into the peptide scaffold may promote the proliferation of the encapsulated cells. *In vivo* these ligands may also attract cells from outside a scaffold to the scaffold, where they may invade the scaffold or otherwise interact with the encapsulated cells. To increase the mechanical strength of the scaffolds, cysteines may be incorporated into the peptides to allow the formation of disulfide bonds, or residues with aromatic rings may be incorporated and cross-linked by exposure to UV light. The *in vivo* half-life of the scaffolds may also be modulated by the incorporation of protease cleavage sites into the scaffold, allowing the scaffold to be enzymatically degraded. Combinations of any of the above alterations may also be made to the same peptide scaffold.

Peptides capable of being cross-linked may be synthesized using standard f-moc chemistry and purified using high pressure liquid chromatography (Table 2). The formation of a peptide scaffold may be initiated by the addition of electrolytes as described herein. The hydrophobic residues with aromatic side chains may be crossed linked by exposure to UV irradiation. The extent of the cross-linking may be precisely controlled by the predetermined length of exposure to UV light and the predetermined peptide concentration. The extent of cross-linking may be determined by light scattering, gel filtration, or scanning electron microscopy using standard methods. Furthermore, the extent of cross-linking may also be examined by HPLC or mass spectrometry analysis of the scaffold after digestion with a protease, such as matrix metalloproteases. The material strength of the scaffold may be determined before and after cross-linking, as described herein.

**Table 2. Representative Peptides for Cross-Linking Study**

| Name | Sequence (N-->C) |
|---|---|
| RGDY16 | RGDYRYDYRYDYRGDY |
| RGDF16 | RGDFRFDFRFDFRGDF |
| RGDW16 | RGDWRWDWRWDWRGDW |
| RADY16 | RADYRYEYRYEYRADY |
| RADF16 | RADFRFDFRFDFRADF |
| RADW16 | RADWRWDWRWDWRADW |

Aggrecan processing sites, such as those underline in Table 3, may be added to the amino- or carboxy-terminus of the peptides or between the amino-and carboxy-termini. Likewise, other matrix metalloproteases (MMPs) cleavage sites, such as those for collagenases, may be introduced in the same manner. Peptide scaffolds formed from these peptides, alone or in combination with peptides capable of being cross-linked, may be exposed to various protease for various lengths of time and at various protease and scaffold concentrations. The rate of degradation of the scaffolds may be determined by HPLC, mass spectrometry, or NMR analysis of the digested peptides released into the supernatant at various time points. Alternatively, if radiolabeled peptides are used for scaffold formation, the amount of radiolabeled peptides released into the supernatant may be measured by scintillation counting. Cross-linking and cleavage studies are described further in pending U.S. Patent Application Serial No. 09/778200, filed February 6, 2001, Entitled "Peptide Scaffold Encapsulation Of Tissue Cells And Uses Thereof'.

**Table 3. Representative Peptides for Enzymatic Cleavage Study**

| Name | Sequence (N-->C) |
|---|---|
| REEE | RGDYRYDYTFREEE-GLGSRYDYRGDY |
| KEEE | RGDYRYDYTFKEEE-GLGSRYDYRGDY |
| SELE | RGDYRYDYTASELE-GRGTRYDYRGDY |
| TAQE | RGDYRYDYAPTAQE-AGEGPRYDYRGDY |
| ISQE | RGDYRYDYPTISQE-LGQRPRYDYRGDY |
| VSQE | RGDYRYDYPTVSQE-LGQRPRYDYRGDY |

If desired, peptide scaffolds may also be formed with a predetermined shape or volume (Figs. 9A-9C). To form a scaffold with a desired geometry or dimension, an aqueous peptide solution is added to a pre-shaped casting mold, and the peptides are induced to self-assemble into a scaffold by the addition of an electrolyte, as described herein. The resulting geometry and dimensions of the macroscopic peptide scaffold are governed by the concentration and amount of peptide solution that is applied, the concentration of electrolyte used to induce assembly of the scaffold, and the dimensions of the casting apparatus. When the peptide structure or scaffold is to be implanted into the body, the shape may be selected, for example, based upon the intended implantation site.

If desired, the peptide scaffolds formed from any of the above peptides may be characterized using various biophysical and optical instrumentation, such as circular dichroism (CD), dynamic light scattering, Fourier transform infrared (FTIR), atomic force microscopy (ATM), scanning electron microscopy (SEM), and transmission electron microscopy (TEM) (see, for example, Leon *et al*., *supra*; Holmes *et al*. (2000), *supra*). For example, biophysical methods may be used to determine the degree of beta-sheet secondary structure in the peptide scaffold. Additionally, filament and pore size, fiber diameter, length, elasticity, and volume fraction may be determined using quantitative image analysis of scanning and transmission electron microscopy. The scaffolds may also be examined using several standard mechanical testing techniques to measure the extent of swelling, the effect of pH and electrolyte concentration on scaffold formation, the level of hydration under various conditions, and the tensile strength.

The encapsulated cells are present in the macroscopic structure in a three-dimensional arrangement. The density of the cells may be approximately 10⁵/ml, between 5 x 10⁵/ml and 5 x 10⁶/ml, inclusive, between 5 x 10⁴/ml and 5 x 10⁵/ml, between 5 x 10⁵/ml and 5 x 10⁶/ml. Other ranges may also be used. Conditions for culturing should be close to physiological conditions. The pH of the culture medium should be close to physiological pH, desirably between pH 6-8, for example about pH 7 to 7.8, in particular pH 7.4. Physiological temperatures range between about 30° C to 40°C. Mammalian cells are desirably cultured at temperatures between about 32° C to about 38°C, *e*.*g*., between about 35° C to about 37° C.

Cells may be cultured within the peptide scaffold for any appropriate time, depending upon the cell number and density desired, the proliferation rate of the cells, and the time required for differentation and/or transdifferentiation to occur, if desired. These parameters may vary depending upon the particular progenitor cells and purposes for which the invention is to be used. One of ordinary skill in the art is able to vary these parameters and to observe the effects of doing so in order to determine the optimal time for maintaining cells in culture within the scaffold. In certain embodiments, the cell are cultured for approximately 3 days, 7 days, 14 days, 21 days, 28 days, 56 days, or 90 days. In certain embodiments, the cells are cultured for between 1 and 3 days inclusive, between 4 and 7 days inclusive, between 8 and 14 days inclusive, between 15 and 21 days inclusive, between 22 and 28 days inclusive, between 29 and 56 days inclusive, or between 57 and 90 days inclusive. Longer or shorter culture periods may also be used.

If desired, differentiation-enhancing agents may be added to the peptide solution or to the electrolyte solution prior to initiation of self-assembly. In this case the concentration of the agent will likely be substantially uniform within the assembled scaffold. In certain embodiments, the agent is added to media with which the peptide scaffold is incubated after encapsulation of cells. After addition to the media, a portion of the differentiation-enhancing agent enters the peptide scaffold, e.g., through diffusion. This process may create a gradient of the differentiation-enhancing factor. Cells in different regions of the scaffold may exhibit different responses to the agent depending upon the concentration of the agent at the location of the cell.

In certain embodiments, the peptide structure renders the encapsulated cells permissive to instruction by the differentiation-enhancing agent. In these embodiments, encapsulated progenitor cells and/or their progeny are induced to differentiate and/or transdifferentiate in the presence of the differentiation-enhancing agent. Growth factors are typically used at concentrations ranging between about 1 fg/ml to 1 mg/ml. Frequently growth factors are used at concentrations in the low nanomolar range, *e*.*g*., 1 - 10 nM. In certain embodiments, growth factors are used at concentrations that are not typically used in the prior art or that are not typically found *in vivo* under normal conditions. For example, growth factors may be used at concentrations that are 5 fold greater, 10 fold greater, 20 fold greater, 100 fold greater, *etc*., than is typically required to produce effects or than typically occurs *in vivo.* Titration experiments can be performed to determine the optimal concentration of a particular differentiation-enhancing agents, such as a growth factor, depending upon the particular growth, differentiating, and/or transdifferentiating effects desired.

In certain embodiments, progenitor cells and/or their progeny that have proliferated, differentiated, and/or transdifferentiated within the peptide scaffold are extracted from the scaffold. The extraction may be accomplished by any suitable means, including mechanical disruption of the scaffold, enzymatic degradation of the scaffold *in vitro*, *etc*. In certain embodiments, the method selected results in extraction of approximately 25%, between 25% and 50% of the cells inclusive, between 51% and 75% of the cells inclusive, or between 76% and 100% of the cells inclusive. Of course methods that result in any convenient range may be selected. The method selected may depend upon the purposes for which the cells are to be used, the number of cells required, *etc.* In certain embodiments, the viability of the extracted cells is approximately 10% of cells, between 10% and 25% inclusive, between 26% and 50% of cells inclusive, between 51 and 75% of cells, inclusive, or between 76% and 100% of cells inclusive. Of course methods that result in any convenient range may be selected. The method selected may depend upon the purposes for which the cells are to be used, the number of cells required, *etc.*

The extracted cells may be further cultured *in vitro,* either in a peptide hydrogel structure or in any other culture vessel. The extracted cells may be administered to a subject by any appropriate route, including intravenous, subcutaneous, oral, percutaneous, intramuscular, or surgical. The administered cells may be used to fill or repair a tissue defect or otherwise supplement an organ or body structure. The administered cells may synthesize or otherwise supply a therapeutic agent. For example, the administered cells may supply a protein, *e*.*g*., an enzyme, that the individual lacks. The administered cells may be genetically modified and thus used as a means to deliver gene therapy.

### Peptide Scaffold Encapsulating Chondrocytes

A peptide with the amino acid sequence n-KLDLKLDLKLDL-c (KLD12) was synthesized using a peptide synthesizer (Applied Biosystems) and lyophilized to a powder. A 0.5% peptide casting solution was obtained by dissolving KLD12 in a solution of 295 mM sucrose and 1 mM HEPES. Freshly isolated chondrocytes from bovine calf femoropatellar groove cartilage were re-suspended in the casting solution at a concentration of 15 x 10⁶ cells/ml. The suspension was injected into a casting frame consisting of a 40 x 40 x 1.5 mm window supported on both faces by filter paper and a porous mesh. The casting frame was placed in a 1 X phosphate-buffered saline (PBS, which contains 150 mM NaCl and 10 mM sodium phosphate at pH 7.4) bath for 15 minutes to induce the self-assembly of the peptides into a scaffold. Desirably, the cells are incubated in the sucrose solution for less than 5 minutes, or more desirably for less than 1 minute, before PBS is added. If desired, formation of a peptide scaffold may be confirmed using phase-contrast microscopy. As a control, cells were also suspended into warm agarose (2% solution, w/w), injected into the casting frame, and placed into a cold 1 X PBS bath for 5 minutes. Both the peptide and control agarose gels were maintained in DMEM media (Gifco) plus 10 % FB S, which was changed every other day.

Initial cell viability was determined based on ethidium bromide staining using a standard FDA assay (Jones et al., Journal of Histochemistry and Cytochemistry 33(1):77-79, 1985; Beletsky et al., Journal of Immunological Methods 134(2):201-205, 1990). For both the peptide scaffold and the agarose gel, initial cell viability was comparable (80-95% after two hours and approximately 75% after 24 hours).

For the following studies of protein and proteoglycan synthesis, glycosaminoglycan (GAG) accumulation, and immunohistochemistry, a 3 mm diameter by 1.5 mm thick cylindrical plug was punched immediately prior to addition of a radiolabel, digestion, or fixation. Extracellular protein production in a plug from the scaffold was measured by addition of [³H]-proline to the media. The radiolabeled proline was taken up by the cells and incorporated into newly synthesized proteins. After 16-24 hours in the radiolabeled media, the plug was rinsed with buffer to remove free [³H]-proline. The extracellular protein was digested by incubation in a proteinase K solution overnight at approximately 60 °C, and the radioactivity present in the digested protein was quantitated by scintillation counting. Proteoglycan production was measured similarly, except that [³⁵S]-sulfate was added to the media instead of [³H]-proline. The total accumulation of GAG, a proteoglycan component, was measured based on fluorometric analysis of the amount of DMMB dye bound (Chandrasekhar et al., Analytical Biochemistry 161(1): 103-108, 1987). The rates of protein and proteoglycan synthesis by the cells in the peptide scaffold were similar to the rates by cells in the agarose gel (Fig. 2). As total GAG accumulation increased (based on measurements of DMMB binding, Fig. 3), the rate of GAG synthesis decreased (based on radiolabel incorporation, Fig. 2), as seen previously.

For histological analysis of GAG, collagen I, and collagen II, samples were fixed at day 21. To visualize GAG, toluidine blue dye was applied using standard procedures (Fig. 4). Based on this staining, proteoglycan deposition is present throughout the gel, with higher intensity in the pericellular regions. Immunohistochemical staining of collagen I using standard procedures resulted in light background staining throughout the gel, with no increase in the pericellular region (Ioannidis et al., Cell Tissue Res. 297:141-147, 1999; Domm et al., Orthopäde 29:91-99, 2000). Collagen II staining with DMP showed a similar deposition pattern as that of GAG staining but with less defined pericellular staining (Ioannidis *et al*., *supra*; Domm *et al*., *supra*) (Fig 5). This result is consistent with the known lower pericellular deposition of collagen.

For mechanical testing of the peptide scaffold, a 6 mm diameter by 1.5 mm thick cylindrical plug was taken from the scaffold at day 28. The plug was subjected to various levels of compression and the level of stress was measured, as described previously (Buschmann *et al*., *supra*). (Figs. 6 and 7). Based on these results, the equilibrium modulus for the scaffold containing chondrocytes was 27 kPa compared to only approximately 0.5 kPa for a scaffold without cells or a scaffold immediately after encapsulation of chondrocytes.

If desired, the stiffness of the peptide scaffolds may be further increased by subjecting the scaffold to static or dynamic compression using standard methods, such as those described by Buschmann *et al*. (*supra*). For example, dynamic compression at 0.01 to 3 Hz, superimposed on a static offset compression may be used. Typically, the dynamic strain amplitude is between 0.01 and 10%, and the static offset compression is between 5 and 15%.

The above method for encapsulating chondrocytes in a peptide scaffold was also repeated using a lower initial cell density of approximately 0.5 x 10⁶ cells/mL. After formation of the peptide scaffold, the cells were substantially evenly dispersed in the scaffold, and cell viability was approximately 80% at 24 hours after scaffold formation. As illustrated in Fig. 8, pairs and clusters of cells that originated from a single cell were also substantially uniformly dispersed in the scaffold five days after its formation. In addition, the total number of cells increased approximately three-fold by day 5.

Other self-assembling peptides may be used in this method to encapsulate living chondrocytes or other cell types. If desired, the potential cytotoxicity of various peptide scaffolds may also be determined by measuring the rate of ³H-thymidine incorporation due to DNA replication or the rate of RNA expression of genes, such as actin or tubulin. Alternatively, a specific marker gene, such as enhanced green fluorescent protein under a specific promoter control, may be introduced into the cells to facilitate monitoring of gene expression and cell viability. The expression of proteins (*e*.*g*., fibronectin and fibronectin receptors) may also be analyzed using specific antibodies (see, for example, Ausubel et al., Current Protocols in Molecular Biology, Chapter 9, John Wiley & Sons, New York, 2000).

### Alternative Mediums and Conditions for Encapsulation of Cells

Insulin-Transferrin-Selenium (ITS, which contains insulin, transferrin, and selenium) medium supplement allows a reduced serum concentration while maintaining normal cell functions. To develop optimal conditions for both cell proliferation and matrix synthesis and accumulation, mediums supplemented with ITS were used to culture chondrocytes encapsulated in peptide scaffolds. In particular, cell proliferation and type II collagen production were assessed at early times in seeded peptide scaffolds cultured in ITS medium +/- 0.2% FBS relative to 10% FBS. As a control, cell biosynthesis in the well-defined agarose culture system in ITS media was evaluated relative to 10% FBS culture over a five week period. In the process of this investigation, we developed a novel method for quantifying the number of viable cells in the peptide scaffold.

### Formation of peptide scaffolds and control agarose gels

Cartilage was harvested from the femoropatellar grooves of 1-2 week-old calves within several hours after slaughter. The tissue was minced, and chondrocytes were extracted by sequential treatment with pronase and collagenase in feed medium (high glucose DMEM supplemented with 10 % FBS (Hyclone, Logan UT), 0.1 mM nonessential amino acids, 0.4 mM proline, 20 µg/ml ascorbate, 100 U/ml penicillin, and 100 µg/ml streptomycin). The tissue was first incubated in 20 U/ml pronase (Sigma Chemical, St. Louis, MO) medium for two hours, washed twice in PBS, and then incubated in 200 U/ml collagenase (Worthington, type 2) overnight. Cells were isolated by passing the digest through a 40 µm cell strainer, and then centrifugation for eight minutes at 100 x g. The cells were washed twice in PBS, resuspended in feed medium minus ascorbate, and stored at 4°C for several hours prior to gel casting. Cell counting and viability were determined using Trypan Blue exclusion on a hemocytometer.

The peptide KLD-12 was synthesized using a peptide synthesizer (Applied Biosystems Peptide synthesizer 431A) and lyophilized to a powder, or customized-synthesized commercially (Research Genetics, AL) (Figs. 10A-10C). KLD12 powder was dissolved in 295 mM sucrose solution at a peptide concentration of 0.56% (w/v). Isolated chondrocytes were resuspended in a volume of sucrose solution equal to 10% of the final peptide/cell suspension volume. Peptide solution was added to obtain a final peptide concentration of 0.5% and a cell density of 15 x 10⁶ cells/ml. The sucrose in this situation was used to maintain physiologic osmotic pressure. The suspension was lightly vortexed and injected into a stainless steel casting frame consisting of a 40 x 40 x 1.6 mm window, supported on both faces by filter paper and porous mesh, similar to that described by Ragan *et al*. (Ragan et al., Arch. Biochem. Biophys. 383:256-264, 2000). The casting frame was placed in a 1X PBS bath to initiate self-assembly of the peptide scaffold into a slab structure. After 25 minutes, the 1.6-mm thick seeded peptide scaffold was transferred to a petri dish for long-term culture.

Chondrocyte-seeded agarose gel slabs were cast in a similar manner. Centrifuged cells were re-suspended in feed medium, and mixed with 3% low melting temperature agarose'(SeaPlaque agarose, FMC Bioproducts, Rockland, ME) to obtain a fmal agarose concentration of 2% and a cell density of 15 x 10⁶ cells/ml. The casting frame was placed in a room temperature 1X PBS bath to initiate gelation. After five minutes the gel slab was transferred to a petri dish containing
4° C medium and subsequently placed in a 37°C incubator. Peptide and agarose gels were cultured in 10% FBS-supplemented DMEM medium ("FBS medium"). Additional gel preparations were maintained in DMEM medium (Fig. 21) supplemented with either 1% ITS (0.1 mg/L insulin, 0.055 mg/L transferrin, and 0.05 mg/L selenium; Sigma Chemical; "ITS medium") or 1% ITS plus 0.2% FBS ("ITS/FBS medium"). The slabs were seeded at a cell density of 15 x 10⁶ or 30 x 10⁶ cells/ml. Each slab was fed 12 ml of medium every other day.

### Cellular biosynthesis of extracelluar matrix macromolecules

To measure the rate of cellular biosynthesis of extracellular matrix macromolecule, groups of six 3-mm diameter by 1.6-mm thick plugs were punched from cell-seeded peptide and agarose gel slabs using a stainless steel dermal punch on days 5, 10, 15, 21 and 28 of culture. Each group of plugs was transferred to a single well of a 12 well dish containing 2 ml feed medium plus 5 µCi/ml ³⁵S-sulfate and 10 µCi/ml ³H-proline. As described above, incorporation of ³⁵S-sulfate and ³H-proline are measures of the rate of synthesis of sulfated proteoglycans and total protein, respectively (Hascall et al., Arch. Biochem. Biophys. 224:206-223, 1983). After 20 hours the plugs were removed from radiolabel medium, and washed five times over 90 minutes in standard PBS plus 1 mM unlabeled proline and sulfate. Each cell-seeded plug was then transferred to 1 ml proteinase K (Roche) TRIS HCl solution and digested overnight at 60°C. Radiolabel incorporation was assayed by mixing 100 µl of the digest with 2 ml scintillation fluid (Ecolume-LCN) and counting the radioactivity (Rack-Beta 1211 scintillation counter, LKB, Turku, Finland) with corrections for spillover and dilution quenching. Total accumulated sulfated GAG content was assessed separately by reacting 20 µl digest with 200 µl Dimethylmethylene Blue dye (DMB) solution in 96-well microplates and analyzing via spectrophotometry (Vmax plate reader, Molecular Devices, Menlo Park, CA). A standard curve was created using chondroitin sulfate (shark cartilage, Sigma Chemical).

Separate control studies on day 35 of culture were performed to assess (i) the fraction of the radioactivity within the peptide scaffold plugs that was in macromolecular versus low molecular weight form and (ii) the fraction of total newly-synthesized ECM macromolecules that was retained within the peptide scaffold versus that released to medium. Radiolabeled species obtained from peptide scaffold extracts and medium fractions were separated into macromolecular and low molecular weight components on a PD10 gel filtration column of Sephadex G-25 (molecular weight cutoff of 1-5,000; Pharmacia, Piscataway, NJ) in 0.5 ml portions of elution buffer containing 1% SDS, 10 mM dithiothreitol, 50 mM Tris-HCl, pH 8.5 (Sah et al., J. Orthop. Res. 7:619-623, 1989). Macromolecular components released into the medium were ∼2% and ∼11% of total peptide scaffold-accumulated ³⁵S-sulfate and ³H-proline, respectively. Proteinase K digested peptide scaffold plugs contained 95% macromolecular ³⁵S-sulfate. Scaffold-accumulated ³H-proline was analyzed following SDS extraction. Cell-seeded peptide scaffold was incubated in 2% SDS, 10 mM dithiothreitol, 50 mM Tris-HCl, pH 8.5 at 100°C for 15 minutes. Extracted ³H-proline molecules were 89% macromolecular. Together, these data demonstrated that the hydrogel scaffold retained ∼93% and 78% of newly-synthesized proteoglycan and protein macromolecules, respectively, similar to that observed in native cartilage organ culture. Due to the high retention of macromolecules in the hydrogel scaffold, radiolabel medium was not routinely analyzed for biosynthetic products.

As a measure of the rate of biosynthesis of extracellular matrix components and the rate of biosynthesis of proteins, ³⁵S-sulfate and ³H-proline incorporation was measured as described above during four weeks in culture in both cell-seeded peptide scaffolds and agarose cultures (Fig. 11A). Proteoglycan (³⁵S-sulfate) and protein (³H-proline) synthesis rates by cells in peptide scaffolds with 10% FBS medium were initially high at early times (day 5) and then decreased by day 15 to values on the order of that in native cartilage tissue. Subsequent incorporation through day 28 remained relatively constant. Radioisotope incorporation in cell-seeded agarose hydrogels (Fig. 11A) revealed biosynthesis levels similar to those in peptide scaffolds, and comparable to values previously reported in the literature for agarose culture using bovine calf chondrocytes. For agarose hydrogels, 15-125% more ³⁵S-radiolabel was incorporated in ITS/FBS cultures than in FBS and ITS cultures. Proline incorporation was highest in the ITS/FBS cultures at all time points tested (Fig. 11B). Similar levels of proline incorporation were observed in FBS and ITS cultures of agarose hydrogels. Significantly, cell-seeded peptide plugs cultured in minimal medium (ITS/FBS) and radiolabeled on day 28 showed biosynthesis rates similar to those in agarose and peptide scaffolds using 10% FBS-supplemented rich medium.

As another measure of extracellular matrix biosynthesis, total glycosaminoglycan (GAG) accumulation was quantified in all radiolabeled cell-seeded plugs, and in unlabeled plugs from ITS/FBS cultured peptide scaffolds during four weeks in culture (Fig. 12A). GAG accumulation in peptide scaffolds using 10% FBS medium increased with time in culture, reaching ∼70 µg/plug by day 28. Agarose cultures showed similar GAG accumulation, consistent with previously reported data for agarose culture. In ITS/FBS medium, GAG accumulation in peptide scaffolds was initially lower than that in both agarose culture and 10% FBS peptide scaffold cultures through day 15; however, by days 21 and 28, GAG accumulation was similar in all three cases. For agarose hydrogels, total GAG accumulation (Fig. 12G) in all medium conditions was similar through 21 days of culture. By day 35, GAG accumulation in ITS/FBS samples was ∼50% higher than the GAG accumulation in ITS and FBS cultures (p<.001).

Toluidine blue staining of peptide scaffolds cultured in FBS medium revealed GAG accumulation throughout the peptide scaffold matrix on day 26 (Fig. 12B). The majority of the chondrocytes showed a rounded morphology and was fully encapsulated within a continuous GAG-rich matrix, with a higher pericellular staining relative to the interterritorial matrix.

### Analysis of collagen formed by chondrocytes and newly synthesized ECM with peptide scaffolds

For histological examination of the chondrocytes, selected 3-mm cell-seeded peptide plugs were punched and fixed in 4% paraformalydehyde solution in PBS, pH 7 overnight at 4°C. The plugs were washed with PBS, dehydrated with sequential concentrations of isopropanol (75%, 96% and 100%), transferred to xylol, and embedded in paraffin. Sections (7µm thick) were made using a sledge microtome (Leitz, Wetzlar, Germany) and spread on slides coated with chromealaun-gelatine [4.5% (w/v) gelatine, 300 mM potassium-chrome-sulfate-12-hydrate]. Selected paraffin sections of the peptide scaffold were deparaffmated in xylol, and transferred into aqua dest using decreasing concentrations of ethanol. These sections were incubated for six minutes with toluidine blue dye solution [0.0714% toluidine blue (Merck, No. 15930), 0.0714% pyronin Y (Fluka, No. 83200), and borax (0.143% di-sodium-tetraborate, Merck, No. 6306)], washed with aqua dest, 96% ethanol, 3x propanol (two minutes each), 3x xylol (15 minutes each), and mounted on microscopic slides using DePeX (Serva, No. 18243).

For collagen immunohistochemistry, selected deparaffmated sections of cell-seeded peptide scaffold were treated with pepsin (3.9 kU per ml 0.5% acetic acid, Sigma Chemical) for 30 minutes, and then washed with TBS buffer (0.14 M NaCl in 20 mM Tris/HCl-Buffer, pH 7.4). Nonspecific peroxidases were blocked by treatment with 0.6% H₂O₂ in methanol for 20 minutes. Samples were rinsed with TBS and treated with the primary antibody for 60 minutes (mouse anti-collagen-type II, Clone CII Cl, DSHB, Iowa, 1:1000 in TBS; negative controls were incubated with TBS without the first antibody). After rinsing in TBS, samples were treated with the second antibody for 30 minutes (rabbit anti-mouse IgG, HRP-conjugated, Dako P-0260, Hamburg, Germany; 1:200 in TBS containing 1% bovine serum), rinsed, and incubated with the third antibody for 30 minutes (goat anti-rabbit IgG, HRP-conjugated, Dako P-0448; 1:100 in TBS containing 1% bovine serum). The samples were stained with diaminobenzidine (DAB-Kit, Vector Laboratories, Burlingame, USA). Cell nuclei were counterstained using Meyer's hemalum (Merck, Darmstadt, Germany), and the stained samples were embedded on microscopic slides using Aquatex (Merck). Sections of bovine articular cartilage were used as positive controls.

For analysis of collagen, selected 3 mm cell-seeded peptide plugs (initial seeding density 15 x 10⁶ cells/ml) cultured in ITS/FBS medium were punched on day 35 and lyophilized. Collagens were extracted with pepsin (Sigma Chemical, 1 mg/ml in 0.2M NaCl, 0.5M acetic acid) for 48 hours at 4° C. The extract was neutralized with 10N NaOH, and the sodium concentration raised to 1M with NaCl. The extract was stirred overnight at 4° C, then centrifuged at 6000 x g for 20 minutes. Collagen was precipitated with 4.5M NaCl, and centrifuged at 16,000 x g for one hour at 4° C. The precipitate was washed twice in washing solution (3 parts 95% ethanol, 1 part 0.4M NaCl, and 0.1M Tris-HCl, pH 7), lyophilized, resuspended in Tris buffer, and analysed by sodium dodecyl sulfate-polyacrylaminde gel electrophoresis (SDS-PAGE; Mini-gel, Bio-Rad, Hercules, CA). Collagens extracted from chick cartilage and mouse skin (generously donated by P. Bruckner, University of Muenster) were run in parallel to the cell-seeded peptide extract as standards for type II collagen synthesized by native chondrocytes and type I collagen synthesized by de-differentiated fibroblastic phenotypes, respectively (Bruckner P., personal communication).

As described above, immunohistochemical and SDS-polyacrylamide denaturing gel electrophoresis analyses for collagen type I and type II were used to explore chondrocyte phenotypic expression. In particular, cell-seeded peptide scaffolds cultured in FBS medium were fixed on day 15 for Collagen II immunostaining. Collagen II (Fig. 12C) showed a staining pattern similar to that of GAG deposition, with positive staining throughout the matrix, especially in pericellular environments. Collagens from cell-seeded peptide scaffolds cultured in ITS medium were extracted on day 35 and analyzed using SDS gel electrophoresis (Fig. 12D). Proteins extracted from cell-seeded peptide scaffold showed bands for collagen type II α1 chains, as well as α1 and 2 chains for type XI collagen. No type I α 2 chains were present, as seen in skin collagen.

On day 13, collagen II immunostaining in FBS medium was largely in the cell associated region (Fig. 12E) and sometimes formed forming continuous networks between cells. In ITS/FBS and ITS (Fig. 12F) medium, collagen II was found only within cells. No collagen II was found in or around cells that incorporated BrdU.

These results indicated unambiguously that cells seeded into peptide scaffolds maintained their chondrocyte phenotype throughout the 4-week *in vitro* culture period.

### Mechanical properties of peptide scaffolds and agarose cultures

For measurement of material properties, groups of three 6-mm diameter cell-seeded peptide plugs were punched on days 0, 6, and 26 from the 10% FBS culture. Additionally, a peptide scaffold was seeded 30 x 10⁶ cells/ml and maintained in ITS/FBS medium, and groups of three 3-mm diameter plugs were punched for evaluation of material properties. The equilibrium and dynamic compressive modulus of each plug was measured in radially confined uniaxial compression using a Dynastat mechanical spectrometer (IMASS, Hingham, MA) as previously described (Frank et al., J. Biomech. 20:615-627, 1987). Briefly, each specimen was placed in the well of a confining cylindrical chamber, which was clamped in the jaws of the Dynastat. A porous platen attached to the upper jaw was used to apply a ramp-and-hold compressive strain of 3% to the plug (3% compression over 10 seconds followed by one to five minutes of hold), resulting in an initial increase and subsequent relaxation of the compressive stress. This sequence was repeated four to six times (up to 12-30% strain), and the ratio of the relaxed equilibrium stress to the engineering strain was used to compute the equilibrium modulus. At 18% compressive offset strain, a 1% amplitude sinusoidal strain was applied at 1.0 Hz. The sinusoidal stress and strain signals were continuously recorded through a computer, and a discrete Fourier transform was implemented to calculate the amplitudes of the fundamental and four higher order harmonics. The dynamic compressive stiffness was calculated as the ratio of the fundamental amplitudes of stress to strain.

The confined compression equilibrium modulus and dynamic stiffness of cell-seeded peptide scaffold plugs cultured in FBS medium were measured on days 0, 6, and 26 (Fig. 13). Day 0 values for equilibrium modulus were less than 1 kPa, which corresponds to the very weak compressive resistance of the acellular peptide scaffold itself. Development of a continuous GAG matrix was reflected in a dramatic increase in mechanical properties over time in culture. By day 26, the equilibrium modulus increased to 26 kPa, a value about 10% of the modulus of native tissue. Dynamic stiffness at 1 Hz also increased with time in culture, with day 26 values about 5% of those of native tissue. Interestingly, cells seeded into peptide scaffolds at 30 x 10⁶ cells/ml and cultured in ITS/FBS medium showed an even more profound increase in compressive stiffness. The equilibrium modulus reached 93 kPa by day 28, and the dynamic stiffness at 1 Hz reached 1.28 MPa (Fig. 13), both values being approximately 1/5 to 1/3 that of native human and animal articular cartilages.

Consistent with the biosynthesis results for agarose cultures, the equilibrium modulus of FBS (44 kPa) and ITS agarose gels (42.8 kPa) were significantly lower than that of ITS/FBS agarose gels (59.8 kPa) (p=.012).

### Proliferation of chondrocytes encapsulated by peptide scaffolds

Proliferation of chondrocytes within peptide scaffold was also measured. DNA quantification of the cell-seeded peptide scaffold using Hoechst dye analysis was ambiguous using the method of Kim et. al. (Anal. Biochem. 174:168-176, 1988), due to interference associated with the peptide scaffold. As an alternative, a viable cell kit based on the compound (3-(4,5-dimethylthiazol-2-yl)-5-(3-carboxymethoxyphenyl)-2-(4-sulfophenyl)-2H- tetrazolium (MTS) (Promega Corp., Madison, WI) was used to determine viable cell density in seeded peptide scaffolds. In order to interpret the values obtained with the MTS kit, a calibration curve for 3-dimensional cultures was first established using agarose cultures (Fig. 14 E). Five agarose slabs were seeded at cell densities ranging between 10-30 x 10⁶ cells/ml. Samples were punched and analyzed on days 2, 3, and 5. For each group of 11 plugs, six were incubated in MTS medium for approximately two hours on a shaker table. SDS was added at a final concentration of 2% to stop the MTS reaction. After 30 minutes, medium samples were tested for optical density. The remaining five plugs were digested and analyzed for DNA content. Mean MTS output was plotted against mean DNA content to establish the calibration curve. MTS data were then obtained for cell-seeded peptide plugs using the established method. Viable cell counts were determined from MTS content, and cell densities are reported as viable cell counts/plug volume.

To further examine and verify the MTS results, cell-seeded peptide samples were analyzed using 5'-bromodeoxyuridine (BrdU) incorporation for 24 hours on days 3, 7, and 13. BrdU was added to the culture media to a final concentration of 10 µM for a period of 24 hours. Following BrdU exposure, the samples were washed with PBS several times, and fixed in 2% paraformaldehyde in PBS at pH 7.4 at room temperature for two hours with slow shaking. Samples were washed several times in PBS and subsequently treated with 0.1% Triton X-100 in PBS for two hours at room temperature. To denature the DNA, the samples were treated with 2M HCl in PBS for 30 minutes at 37° C. Following HCl treatment, several washes were performed with PBS until pH 7.4 was recovered. At this time, blocking buffer (20% bovine calf serum, 0.1 % Triton X-100, and 1% DMSO in PBS) was added to the samples, which were then incubated for four hours at 4° C with slow shaking. Anti-BrdU mouse monoclonal antibody IgG₁ FITC-conjugated (BD PharMingen, catalog number 33284X) was preincubated in blocking buffer for one hour at 4° C (1:40 dilution) and then added to the samples overnight at 4° C with slow shaking. After the incubation period, the samples were washed three times with blocking buffer over the course of two hours, and a final wash in PBS was also performed for an extra hour. The samples were monitored under a Nikon TE 300 inverted microscope with phase contrast and fluorescence, using an OpenLab data acquisition system mounted with a Hamamatsu digital camera. The pictures obtained represent a single optical layer observed with phase contrast and fluorescence.

Calibration of the MTS viable cell assay in cell-seeded agarose plugs showed linear behavior over a range of cell densities (Fig. 14A, Fig. 14E). Cell-seeded peptide scaffolds and agarose gels cultured in FBS medium were evaluated for viable cell density using the MTS assay (Figs. 14B and 14F). In agarose gels, viable cell density increased by 20% between day 2 and day 7 of culture, an increase similar to that previously reported using DNA (Hoechst dye) analysis. In contrast, peptide scaffolds showed a more dramatic 80% or 60% increase in viable cell density between days 2 and 9 of culture for FBS and ITS/FBS mediums, respectively. Proliferation was slowest using the ITS medium, with day 9 values ∼30% higher than day 2 for peptide scaffolds. BrdU incorporation in cell-seeded peptide scaffolds further delineated MTS results (Figs. 14C and 14D). A significant fraction of cells within the peptide scaffold labeled positively for BrdU incorporation during the 24 hour incubation period on days 2-3 and 7-8, further indicating the high content of proliferating cells in these cultures. Because cells tended to de-differentiate and proliferate at the surface of FBS gels, FBS values may overestimate the size of the intragel chondrocyte population. Little or no de-differentiation was visible on the surface of ITS/FBS and ITS gels (Figs. 15A-F). On day 13, BrdU incorporation was sparse in samples in all media conditions.

DNA analysis also showed similar cell densities in all mediums through 35 days of agarose culture. H&E sections on day 35 showed similar populations of single cell and doublets. DNA values for the FBS cultures may also overestimate the intragel chondrocyte population, as discussed above for peptide scaffolds in FBS, due to differentiation and proliferation on the gel surface.

### Cell morphology

Cell pairs in peptide scaffold each gave the appearance of the typical chondrocyte spherical morphology. In contrast, cell pairs in 2% agarose, a material that is about 20 times stiffer than peptide scaffold, assumed hemispheric morphologies approximately within the volume of a single cast cell. During culturing of agarose hydrogels, a continuous monolayer had formed which partially peeled off the gel surface during sample punching by day 35. No macroscopic monolayer was observed in either ITS medium.

### Summary of results

The casting procedures described herein enable rapid and efficient encapsulation of chondrocytes within peptide scaffolds while maintaining high cell viability (>80%). Peptide dissolved in sucrose did not significantly increase the viscosity of the solution, and chondrocytes could be quickly and evenly resuspended without the formation of air bubbles. Consequently, encapsulated cells were evenly distributed throughout the scaffold. In this study, cell-seeded scaffolds were cast in a flat slab geometry to enable quantification of biomechanical properties and normalization of ECM biosynthesis and accumulation to a constant plug volume. However, this casting technique is generally applicable for scaffolds of arbitrary geometry and for other cell types, a very important feature for applications in tissue repair. Our approach also avoids difficulties that may be encountered when seeding cells into an already preformed scaffold. Here, peptides self assemble into well ordered nanofibers with inter-fiber spaces ∼50-200 nm and, simultaneously, into a microscale scaffold network around each individual chondrocyte in the cell-peptide solution. This intimate cell-scaffold architecture may offer unique advantages regarding peptide-cell signaling and cell-mediated peptide biodegradability.

The addition of small concentrations of FBS to the ITS medium increased proliferation to nearly that of 10% FBS medium, while still limiting de-differentiation on the scaffold surface. Collagen II immunostaining (day 13) showed less deposition of collagen II in both ITS media, relative to 10% FBS medium. On day 13, handling properties of FBS-cultured scaffold had increased significantly over that of a scaffold without cells. ITS/FBS samples showed a marginal increase in strength, while ITS samples were as delicate as an acellular scaffold. ITS and ITS/FBS medium supplements are acceptable alternatives to 10% FBS for culture of chondrocytes in agarose gels. DMEM + 1% ITS is a fully-defined medium that allows for biosynthesis similar to 10% FBS. Addition of 0.2% FBS to the ITS medium increased biosynthesis over both single-component media. As observed in peptide cultures, surface de-differentiation is limited with both ITS media, reducing the potential artifact for normalization of biosynthesis to plug DNA. The agarose results indicated that ITS medium is potentially equivalent or superior to 10% FBS for biosynthesis.

### Cellular Biosynthesis of Extracellular Matrix and Proteins

A major challenge in choosing an appropriate scaffold for cartilage repair is the identification of a material that can stimulate high rates of proliferation of chondrocytes and, simultaneously, high rates of chondrocyte synthesis of phenotypically specific ECM macromolecules. The peptide scaffold matrix appeared favorable for both. The proliferation of encapsulated chondrocytes was substantially higher in peptide scaffolds relative to agarose control cultures. Abundant cell pairs undergoing cell division were observed within the scaffold as early as day 3 in culture (Figs. 15B-15D). The higher proliferation in peptide scaffold relative to agarose may be attributed to cell-peptide interactions, physical environment, or other factors. Proliferating chondrocytes were able to displace the peptide scaffold matrix to a greater extent than agarose.

### Cellular biosynthesis of extracelluar matrix macromolecules

Radiolabel incorporation and total GAG accumulation data (Figs. 11A and 12A) were normalized on a per-plug basis, as punched from parent slab gels. In this manner, biosynthesis is reported as representative of a repair response for a given initial scaffold geometry and cell-seeding density. Direct normalization on a per cell basis via DNA content was ambiguous due to interference of peptide digest with the Hoechst dye assay. Radiolabel incorporation (Fig. 11A) showed that synthesis of proteoglycans and total protein by primary calf chondrocytes was similar in 3-D agarose and 3-D peptide scaffold culture. Thus, specific differences in scaffold composition and micro-physical environment between peptide and agarose scaffolds did not significantly affect the net rates of cell synthesis of ECM macromolecules.

Total GAG accumulation in cell-seeded peptide scaffold was also similar to that in agarose culture (Fig. 12A), despite the differences in scaffold concentration (0.5% vs. 2% w/w). For comparison with other studies in the literature, data from day 15 (Fig. 12A) were normalized to DNA content (7.7 pg DNA/cell (Kim *et al., supra*) based on an estimate of the cell density at day 15, which was assumed to be approximately twice the initial seeding density from the trends of Fig. 14B. Thus, the computed GAG density on day 15 was ∼22 µg/µg DNA. This value is less than that reported in alginate culture (∼50 µg/µg) (Ragan *et al., supra*) but greater than that in type I collagen sponge (4.8 µg/µg) (Mizuno et al., J. Biomed. Mater. Res. 56:368-375, 2001), PGA (∼ 8 µg/µg) and PLA (∼5 µg/µg) (Freed et al., J. Biomed. Mater. 27:11-23, 1993). In addition, the percent GAG normalized to wet weight in peptide scaffold (estimate ∼0.9%, week 4) compared to PEO (∼0.23%, week 6, 50 x 10⁶ cells/ml initial seeding density) (Bryant et al., Biomaterials 22:619-626, 2001). While direct comparisons are difficult due to inconsistent factors such as seeding density and culture medium, GAG accumulation in our peptide scaffold appears to be within the range of other polymer and scaffold scaffolds.

In this study the majority of experiments were conducted at peptide and agarose hydrogel seeding density of 15 x 10⁶ cells/ml in culture medium containing 10% FBS. Such conditions are convenient for comparison to previously published studies but are not necessarily optimal for *in vitro* development of repair tissue. Studies using different medium and cell densities were also conducted to demonstrate options for *in vitro* culture conditions. ITS-based, low serum medium (0.2% FBS) was found to be comparable to 10% FBS + DMEM in terms of total GAG accumulation and radioisotope incorporation (on day 28, Fig. 11A). However, a 2-fold increase in cell seeding density in combination with culture in ITS/FBS medium resulted in a 4-fold increase in equilibrium compressive modulus and an over 10-fold increase in dynamic stiffness, much more than would be expected from a proportional increase in seeding density alone. Thus, even higher cell seeding densities may be used to further optimize a chondrocyte/peptide-scaffold system for clinical use in cartilage repair.

### Analysis of collagen formed by chondrocytes and newly synthesized ECM with peptide scaffolds

Immunohistochemical analysis for type II collagen showed positive staining throughout the peptide scaffold in a continuous manner, while type I collagen staining appeared at only background levels around cells within the gel. With 10% FBS supplemented medium, positive type I collagen staining was observed within a thin layer of fibroblastic-like cells that showed increasing cell numbers with time in culture only on the outer surfaces of the gel. Interestingly, cell-seeded peptide scaffolds cultured in ITS/FBS medium were observed to have much less de-differentiation and fibroblast-like cell accumulation on the surface (Figs. 15A-F). Therefore, cell samples were chosen from ITS/FBS culture for collagen content analysis. SDS-gel electrophoresis confirmed accumulation of predominantly type II collagen in the cell-seeded peptide scaffold culture (Fig. 12D).

### Mechanical properties of peptide scaffolds and agarose cultures

GAG accumulation in the seeded peptide scaffold was distributed throughout the scaffold, in both cell-associated and interterritorial space (Fig. 12B). The development of continuous ECM was reflected in the increase in compressive stiffness as a function of time in culture (Fig. 13). The day 0 mechanical properties of the peptide scaffold seeded with 15 x 10⁶ cells/ml were very fragile, with extremely low compressive resistance (100-1000 times lower than native cartilage). After six days in culture, the handling properties of the plugs were noticeably improved, with equilibrium modulus increasing by almost an order of magnitude. By day 26 the test samples could be freely handled, and attained an equilibrium modulus of 26 kPa (approximately 30-fold increase compared to day 0). For comparison, the equilibrium modulus of PGA scaffolds seeded with primary calf chondrocytes at ∼125 x 10⁶ cells/ml and cultured in free-swelling conditions was ∼52 kPa after 42 days (Vunjak-Novakovic et al., J. Orthop. Res. 17:130-138, 1999), and the modulus of peptide scaffold scaffolds with 30 x 10⁶ cells/ml in ITS/FBS was 93 kPa (Fig. 13). For agarose hydrogels, the equilibrium modulus of FBS (44 kPa) and ITS cultures (42.8 kPa) were significantly lower than that of ITS/FBS cultures (59.8 kPa) (p=.012).

### Exemplary Compression Schemes for Increasing the Stiffness of Peptide Scaffolds

*In vitro* conditioning of cell-seeded tissue engineered scaffolds prior to implantation may benefit from long-term (*e*.*g*., over 24 hours) mechanical loading that increases biosynthesis and accelerates matrix accumulation relative to free-swell cultures. Various duty cycles of dynamic compressive loading were tested in a chondrocyte-seeded self-assembling peptide scaffold and in an agarose culture system.

### Formation and treatment of-peptide scaffolds encapsulating chondrocytes

For the formation of chondrocyte-encapsulated peptide scaffolds, bovine chondrocytes were isolated from 1-2 week old calves by sequential pronase and collagenase digestion. Cells were seeded into 2% agarose or self-assembling peptide scaffolds as 1.6 mm thick flat slab structures at a concentration of 15 x 10⁶ cells/ml, as described herein. The peptide scaffold consisted of the 12 amino acid sequence n-KLDLKLDLKLDL-c at a concentration of 0.5%. Seeded gels were cultured in DMEM supplemented with 1% ITS + 0.2% FBS.

A new chamber was designed for long-term mechanical compression of the seeded peptide scaffolds (Fig. 16). In the base component, six wells were designed to each hold one 12 mm diameter sample. Porous platens (40% void, 120 µm pore size, 13 mm diameter) were attached to the lid, aligned co-axially with the center of the wells. The lid contained a center-mounted spring that aligns with a non-culture well in the base component. In this manner, the spring created a 400-800 µm gap between the platens and samples when the lid was unloaded. The chamber was placed in an incubator-housed loading system (Frank et al., J. Biomech. 2000) to apply dynamic compressive loading to seeded peptide scaffolds (Fig. 17).

Experiments used sinusoidal dynamic compression protocols with a 2.5-3% strain amplitude superimposed on 5-7% static offset strain at a frequency of 1.0 Hz. Loading was applied for 45 minutes to one hour, after which samples were maintained in free-swelling conditions for a designated "off" time by backing the platens several hundred micrometers off the gels. Such duty cycles are designated as "on" during loading, and "off" during non-loading periods.

### Results for agarose cultures

Studies investigated repeated duty cycle protocols of one hour "on"/one to seven hours "off" using agarose cultures. For each duty cycle, samples were maintained in free-swelling conditions for 0-28 days after casting prior to loading. Loading was applied for 4-8 days, with 20 hours of radiolabel incorporation (³H-proline or ³⁵S-sulfate radiolabel incorporation) at the end of the loading period.

For analysis of each 12 mm sample, up to seven 3-mm plugs were punched. Total GAG accumulation (DMMB dye binding) and total protein and proteoglycan synthesis (³H-proline and ³⁵S-sulfate radiolabel incorporation) were measured following proteinase K digestion, as described herein. Radiolabel medium was analyzed for macromolecular content by fractionating on Sephadex G25 columns. Seeded peptide scaffolds were analyzed for scaffold-incorporated macromolecule and low-molecular weight content by applying sample digest and SDS- extracted macromolecules to a Sephadex G25 columns (Sah et al., J. O. R. 7(5), 1989). For all results, loaded samples were compared to free-swell control cultures.

All repeated duty cycles decreased radiolabel incorporation to ∼30-80% of free-swelling controls. For example, Fig. 18 shows radiolabel incorporation into the agarose gel for a duty cycles of one hour on/seven hours off, and one hour on/one hour off (control value=1). Incorporation increased when loading was applied every other day (Fig. 19). Here, three samples were loaded on day 12 after casting. A loading protocol consisting of four cycles of 45 minutes on/five hours 15 minutes off, followed by 24 hours of no loading was applied for up to 12 days. Samples were radiolabeled for 20 hours on days 15 (non-loading period), 22 (loading period), and 23 (non-loading). Non-loading periods showed increased sulfate incorporation and equivalent or decreased proline incorporation normalized to free-swell control values (control=1). Radiolabel during loading showed similar sulfate and decrease proline incorporation. On day 15, labeled ³⁵S-macromolecules released to the medium during the labeling period were only 1-2% of the label incorporated into the agarose in both loaded and control samples. Despite the higher sulfate incorporation (Fig. 19), total GAG accumulation was similar at all time points.

### Results for peptide scaffolds

Chondrocyte-seeded peptide scaffolds were loaded using the alternate day loading protocol of Fig. 19 after 21 days of free-swell culture. Samples were radiolabeled during loading (day 32) and non-loading (days 27 and 33) periods. Sulfate incorporation was higher than free-swell control samples at all time points, especially during loading. Proline incorporation was similar or less than control values. Total GAG content was measured on days 32 and 33. In both cases, GAG content was 9% higher in loaded samples (p=0.005, 0.035). This difference represents a ∼20-30% increase in GAG content during the 12 and 13 day loading periods. The medium was analyzed for release of sulfate and proline-labeled macromolecules at all time points. For both loaded and control samples, ³⁵S-macromolecule release was ∼1-3% of scaffold-incorporated sulfate label. Release of ³H-macromolecules was 4-6% of scaffold-incorporated label in loaded samples, and 10-12% of that in free-swelling controls. Scaffold-accumulated radiolabel analysis was performed on day 33. The measured values were similar in both loaded and control groups, with ∼95% macromolecular sulfate and ∼86% macromolecular proline in the scaffolds (Fig. 20).

### Summary of results

As a model system of chondrocyte-seeded peptide scaffold culture, agarose gels required alternate day loading in order to increase sulfate incorporation over free-swelling cultures. However, proline incorporation was at best similar to control samples, and up to ∼50% less, consistent with Lee et al. (J. Orthop. Res. 15(2), 1997). In contrast results for agarose cultures, sulfate incorporation into seeded peptide scaffolds was maximized during periods of loading, and total GAG accumulation was higher relative to free-swell controls. Proline incorporation decreased with loading, although to a lesser extent than in agarose cultures. Release of ³⁵S-macromolecules was minimal and unaffected by loading. However, release of ³H-proline macromolecules was lower in loaded cultures, further reducing total protein biosynthesis (scaffold incorporated + macromolecule released) relative to free-swell controls. The percentage of labeled macromolecules incorporated into the scaffold is similar to that in agarose (Buschmann et al., J. Cell Sci. 108, 1995) and explant culture (Sah *et al., supra*). Cell-seeded peptide scaffolds may respond favorably to more frequent or daily loading cycles to further increase proteoglycan synthesis and to increase or maintain protein synthesis.

### Alternative Compression Schemes

To increase the stiffness of scaffolds, shear loading may also be applied (Jin et al., Arch Biochem Biophys 395(1):41-8, 2001). In order to apply shear loads and create shear deformation, sufficient friction must exist between the sample and loading platen to create a traction force. The traction force then displaces the surfaces, creating shear deformation. The application of compressive loading increases friction between the sample and platen, which may be necessary in order to prevent slipping between sample and platen. In particular, shear loading is typically applied after a compressive offset is reached for appropriate friction between sample and platen. The frequencies are in the same range for shear or compressive loading. Dynamic shear loading can be applied without dynamic compressive loading, alternating periods of dynamic compressive and dynamic shear, or simultaneously with a certain systems.

For example, the macroscopic scaffold can be compressed for a static offset of 0 to 45%, 0 to 40%, 0 to 30%, 0 to 20%, 0 to 10%, or 10 to 20%. A shear strain (*e*.*g*., a shear strain of 0.01 to 30%, 0.05 to 20%, 0.1 to 10%, 1 to 10%, or 2 to 5%) is applied to the macroscopic scaffold. In various embodiments, the frequency is 0.001 to 20Hz, 0.001 to 10 Hz, 0.02 to 10 Hz, 0.1 to 10 Hz, or 1 to 5 Hz. The shear loading can be continuous or variable.

### In vivo Immune and Inflammatory Responses for Scaffolds Encapsulating Chondrocytes or Other Cell Types

The *in vivo* immune and inflammatory responses to two self-assembling peptides were analyzed. Neither the RAD16 or EAK16 peptides, alone or conjugated with other highly immunogeneic proteins such as BSA, elicited a detectable immunological response when injected into rabbits or goats (Holmes *et al*. (2000), *supra*). Also no significant titers of antibodies were obtained. To measure the inflammatory response elicited by these peptides, the peptides were injected into the leg muscle and brain of rats (Holmes *et al*. (2000), *supra*). No inflammation in these or neighboring areas was observed during the two weeks following the injection. Other self-assembling peptides may be tested similarly to measure the immune and inflammatory responses that they generate.

The lack of an immune or inflammatory response to these peptides in a variety of mammals suggests that the peptides may not elicit an adverse immune or inflammatory response when administered to humans. Furthermore, structural modeling and theoretical analysis of peptide presentations by class I and class II MHC molecules also suggest that the self-assembling peptides of the present invention are not likely to elicit strong immune response due to their alternating distribution of charged and uncharged residues.

### In vivo Animal Model Studies for Scaffolds Encapsulating Chondrocytes or Other Cell Types

Several previous studies have employed a canine model to compare the reparative tissues formed in defects in articular cartilage (see, for example, Brittberg et al., New England J. of Med. 331:889-894, 1994; Breinan et al., J. Bone Jt. Surg. 79-A: 1439-1451, 1997; Breinan et al. Tiss. Engr. 4:101-114, 1998; Nehrer et al., Biomaterials 19:2313-23128, 1998). For initial studies, four dogs are tested for each peptide scaffold. Two defects are made in each knee, and each defect is filled with a peptide scaffold encapsulating chondrocytes. Because previous studies have generated data on untreated control groups, an untreated control group is not needed for this study (Breinan *et al*. (1997), *supra*). However, if desired, dogs in which one or more of the knee detects are not filed with a scaffold or are filed with a scaffold that does not contain cells may be used as controls.

The power calculation for determining the required sample size for these experimental groups is based on detecting a 30% difference in the mean values of total fill, the areal percentage of hyaline cartilage, and the values of specific mechanical properties, assuming a 25% standard deviation. A 30% change in the outcome variable is expected to be a meaningful indication of the benefit of one treatment group over another. For a power of 0.80 (β=0.20) and a level of significance of α=0.05, n = 8 specimens are required. The statistical analysis is performed by averaging the values for the two defects in the same knee and counting the average value as one observation. However, in our previous work with this animal model, there were no systematic relationships between the two defects in the same knee of an animal. Thus, if desired, the individual defects may be treated as independent observations.

For this study, adult mongrel or hound dogs, each weighing approximately 25-30 kilograms, are used. Prior to the operation, the knee joints are examined roentgenographically to exclude animals with degenerative joint disease. All operations are performed under general anesthesia and sterile conditions, as described previously (Breinan *et al*. (1997), *supra*). Two 4 mm diameter defects are created in the trochlear groove of the right stifle (knee) joint. These defects are placed approximately 1.25 and 2.25 centimeters proximal to the intercondylar notch, each slightly lateral or medial to the midline. A 4 mm diameter dermal punch is used to outline the defect. Using loupe visualization, an attempt is made to remove all non-calcified cartilage from the defect by scraping the calcified cartilage surface with a customized curette, without fissuring the calcified cartilage. The objective is to remove all of the articular cartilage and to gently scrape the calcified cartilage to facilitate the integration of the reparative tissue with the calcified cartilage. A peptide scaffold with or without encapsulated chondrocytes is placed in each defect. Before closing the capsule, bleeding vessels are clamped and cauterized. The knee joint is closed by zero point suturing. Postoperatively, the operative knee are immobilized by external fixation (IMEX Veterinary, Longview, Texas) for ten days (Breinan *et. al*. (1997), *supra*). It is also contemplated that the operative knee may be mobilized for a longer time period if required for the peptide scaffold to increase in stiffness as extracelluar matrix components are secreted *in vivo* by the encapsulated cells and/or nearby cells. Six months after the first surgical procedure, two defects are made in the left knee using the same procedure. The dogs are sacrificed 12 months after the initial surgical procedure, producing a postoperative evaluation period of 12 months for the right knees and six months for the left knees. It is also contemplated that other postoperative periods may be used, such as a few hours, a few days, or even a few years. Additionally, the defects in other dogs may be analyzed at earlier time points, such as after 30 minutes, a few hours, or a few days, to determine if early displacement of the graft is occurring.

After formalin fixation, specimens are immersed in a 15% disodium ethylenediamine tetracetate decalcifying solution at pH 7.4. The samples are placed on a shaker at 4 °C for four weeks, and during this incubation the decalcifying solution is changed every week. Samples are rinsed thoroughly, dehydrated, and embedded in paraffin at 60 °C. Seven-micrometer thick sections are stained with hematoxylin, eosin, and/or safranin O/fast green. Selected paraffin sections are stained with antibodies to type I collagen and type II collagen.

The specific tissue types filling the defects are determined by evaluating the percentage of the area of the central section through the defect occupied by each tissue type: articular cartilage, non-articular hyaline cartilage, fibrocartilage, and fibrous tissue (Breinan *et al*. (1997), *supra*). These percentages refer only to the representative histological cross-section through the middle portion of the defect. They do not imply values equivalent to the actual volume percentages of tissues in the defects. Due to edge effects (regenerating tissues tend to form at the periphery of the defect), only sections representing 60% or more of the defect diameter are analyzed. Sections taken too close to the edge of the defect may preferentially show regeneration, which could yield misleading data. The effects of treatment and time on the areal percentages of specific tissue types are determined by two-way ANOVA. Group comparisons are made using the Student t test with the appropriate corrections.

Additionally, any degradation of adjacent tissues and the bonding of the repaired tissue to the subchondral plate and the adjacent cartilage may be evaluated. The presence of new tissue formed in the remodeling subchondral bone underlying the defects may also be determined, and the area surrounding the defects may be analyzed for signs of inflammation. If desired, the rate of scaffold degradation may be measured. For this determination of the *in vivo* degradation rate, radiolabelled peptides, such as ¹⁴C-, ³H-, or ³⁵S-labelled peptides, may be assembled into a radiolabelled peptide scaffold and administered to a mammal using the methods of the present invention. At one or more time points after administration of the radioactive scaffold, urine or blood samples are obtained from the mammal. The amount of radioactivity in the sample is measured to determine the amount of degradation products that have been released from the scaffold.

Other animal models may be used to test peptides scaffolds encapsulating living cells for the ability to repair or replace tissues *in vivo.* For example, scaffold encapsulating chrondrocytes may also be tested using rabbit models of cartilage defects (see, for example, Perka et al. Clinical Orthopaedics 378:245-254, 2000; Solchaga et al., Journal of Orthopaedic Research 18(5):773-780, 2000). Standard bone tissue engineering animal models may be used for *in vivo* studies of scaffolds encapsulating cells such as osteocytes (see, for example, Lennon et al., Experimental Cell Research 219(1):211-222, 1995; Solchaga *et al., supra;* Boyan et al., Journal of Orthopaedic Research 17(2):246-55, 1999). Examples of ligament tissue engineering animal models that may be used to test peptide scaffolds for the ability to repair or replace ligament tissue *in vivo* include those described by Awad et al. (Tissue Engineering 5(3):267-277, 1999) and Kato et al. (Journal of Bone and Joint Surgery (Am) 73(4):561-574, 1991). Peptides scaffolds encapsulating any other cell type may also be routinely tested in an appropriate animal model. Standard medical procedures may be used to adapt the methods used to repair or replace tissues in these animal models for the treatment of other mammals, such as humans.

### Encapsulation and/or Differentiation of Progenitor Cells

### Overview

Stem cells have been defined as cells that are able to both self-renew, *i*.*e*., to divide and create additional stem cells, and also to give rise to cells that can undergo differentiation along a specified pathway or pathways (See, *e*.*g*., Fuchs, E. and Segre, J., "Stem Cells: A New Lease on Life", Cell, 100, 143-155, 2000; Weissman, I., "Stem Cells: Units of Development, Units of Regeneration, and Units in Evolution", Cell, 100, 157-168, 2000). Thus stem cells themselves are undifferentiated but are able to generate one or more specialized cell types with specific functions in the body. Differentiation refers to a qualitative change in cellular phenotype that is the consequence of the synthesis of new gene products (Loeffler, M. and Potten, C., "Stem cells and cellular pedigrees - a conceptual introduction", in Stem Cells, Potten, C. (ed.), Academic Press, San Diego, 1997).

Differentiation may be recognized by a morphological change in the cell or by detecting changes in enzyme activity or protein composition. Proteins (including enzymes) and the mRNAs that encode such proteins that may be used to characterize a particular differentiation pathway or state are referred to herein as markers. Such markers may not be unique to a particular differentiated cell type but may be found in a variety of differentiated cell types. For example, cytochrome P450 enzymes are produced by mature hepatocytes and are thus markers for hepatocyte differentiation. However, some of these enzymes are also produced by cells in the intestine and are thus also markers for these intestinal cells. One of ordinary skill in the art will readily be able to select an appropriate marker or combination of markers that are sufficient to identify a cell as being of a particular differentiated cell type.

Two different types of stem cells have been recognized. Embryonic stem cells, present in the embryonic blastocyst, are sufficiently undifferentiated that they are able to give rise to any cell type. Somatic stem cells are found in specific adult tissues, where they are thought to be able to divide indefinitely. Until recently it was widely believed that somatic stem cells possessed a restricted *in vivo* differentiation capability, e.g., that they were only able to give rise to differentiated cells characteristic of the tissues in which they were found. Recent results, however, have suggested that somatic stem cells may have a much broader range of differentiation possibilities than previously thought. For example, neural stem cells appear able to give rise to hematopoietic cells *in vivo* (Bjornson, C., et al., Science, 283:534-537), while hematopoietic stem cells can differentiate into hepatocytes (Lagasse, E., et al., "Purified hematopoietic stem cells can differentiate into hepatocytes in vivo. Nature Medicine, 6:1229-1234, 2000). These and other similar results, based largely on *in vivo* studies, have highlighted the importance of the phenomenon of cellular plasticity.

As used herein, the term "progenitor cell" refers to a cell that is not fully differentiated but that has the capacity to give rise to a daughter cell or cells that are able to do so. Thus a stem cell is one type of progenitor cell. As is known in the art, a stem cell is generally considered to have broader differentiation potential than other types of progenitor cells. Stem cells are generally considered to have the ability to self-renew. The term "progenitor cell" also includes cells that may have undertaken one or more steps along a differentiation pathway, *e*.*g*., that express one or more differentiation markers. The terms "precursor cell" and "progenitor cell" are used interchangeably herein.

In order to more fully exploit the potential of progenitor cells and stem cells it is desirable to be able to control and manipulate their differentiation. In particular, it would be desirable to control cellular transdifferentiation. As used herein, the term "transdifferentiation" refers to (1) the capacity of a progenitor cell isolated from a particular body tissue or organ, either to differentiate into cells of a type normally found in a different body tissue or organ or to give rise to a daughter cell or cells that are able to do so or (2) the capacity of a cell that exhibits a cellular morphology characteristic of a particular cell lineage or expresses one or more differentiation markers characteristic of a particular cell lineage to alter its developmental fate and to adopt an alternate differentiation pathway. These alternative definitions are not mutually exclusive and will frequently overlap. Altering the differentiation or transdifferentiation potential of a cell may be referred to as cellular reprogramming.

The present invention provides compositions and methods for manipulating the extracellular environment of a progenitor cell or stem cell including compositions and methods for enhancing differentiation and transdifferentiation. Such compositions and methods include growing cells under physical conditions that render the cells permissive for instruction along particular differentiation pathways. The physical conditions can include, in particular, encapsulating progenitor cells within three-dimensional peptide hydrogels. The instruction can comprise exposing the cells to one or more growth or differentiation factors. The compositions and methods of the present invention allow harnessing of the inherent regenerative capacity of progenitor cells in new and useful ways.

The regenerative capacity of the body is particularly striking in the case of certain organs such as the liver, which is well-known to possess extensive capacity to regenerate after insults ranging from partial hepatectomy to toxin-induced injury. Unlike other regenerating tissues such as bone marrow and skin, liver regeneration is thought not to depend on a small group of progenitor cells. Instead, regeneration occurs through the division of mature cell populations including hepatocytes (the main functional cells of the liver, which synthesize a wide range of proteins including enzymes that serve key roles in metabolism of exogenous and endogenous compounds); biliary epithelial cells (which line biliary ducts); endothelial cells; Kupffer cells (macrophages); and stellate cells, also referred to as Ito cells (which are located under liver sinusoids, surround hepatocytes with long processes, and secrete extracellular matrix proteins and growth factors). Liver regeneration is extensively reviewed in Michalopoulos, G. and DeFrances, M., "Liver Regeneration", Science, 276, 60-66, 1997.

Nevertheless, cells with stem cell or precursor cell properties do appear in large numbers when mature hepatocytes are prevented from proliferating. These cells are able to give rise *in vivo* to some or all of the mature cell types found in the liver. While it is possible to culture liver-derived cells *in vitro* success in maintaining long-term cultures of fully differentiated, functional hepatocytes has been limited. Cultured hepatocytes tend to lose certain aspects of the mature hepatocyte phenotype. This feature has hampered the development of cell culture systems in which to study hepatotropic viruses such as hepatitis B virus, which replicate in mature hepatocytes. In addition, it limits the utility of liver-derived cells in medical applications such as tissue engineering (*e*.*g*., artificial livers), cell transplantation therapies, and gene therapy.

Unlike the central nervous system, the liver represents an organ that is readily accessible for the removal of tissue. The inherent regenerative capacity of the liver and the fact that even a fraction of the normal liver mass is able to fulfill the body's needs suggest that the liver can serve as a convenient source of cells for *in vitro* culture and medical applications, not necessarily limited to the treatment of liver-related conditions.

The present invention represents a convergence of research in the fields of stem and progenitor cell biology and technology and research in the field of biological materials. The development of new biological materials, particularly biologically compatible materials that serve as permissive substrates for cell growth, differentiation, and biological function has broad implications for advancing medical technology and for understanding basic biological characteristics of cells. The inventors have previously described a class of biomaterials that are made through self-assembly of ionic self-complementary peptides (Zhang, S., et al., Proc. Natl. Acad. Sci. USA, 90, 3334-3338, 1993; Zhang, S., et al., Biomaterials, 16, 1385-1393, 1995; U.S. Patent Numbers 5,955,343 and 5,670,483). These materials are hydrogels, which in certain embodiments contain approximately 99% or greater water content. They self-assemble into membranes or three-dimensional structures upon exposure to a sufficient concentration of ions. The sequences, characteristics, and properties of the peptides and the structures formed by them upon self-assembly are further discussed herein.

The inventors have shown that these peptide structures are able to support cell attachment, viability, and growth when cells are cultured on the surface of the structure. In addition, the structures (also referred to herein as scaffolds) are able to serve as substrates for neurite outgrowth and synapse formation when neurons are grown on their surface (Holmes, T., et al., Proc. Natl. Acad. Sci., 97(12), 2000). In addition, inventors have shown that it is possible to encapsulate cells within the peptide hydrogels, thus placing the cells in a three-dimensional arrangement within the peptide structure, and that the cells maintain viability and function when so encapsulated (see pending U.S. Patent Application Serial No. 09/778200, filed February 6, 2001, Entitled "Peptide Scaffold Encapsulation Of Tissue Cells And Uses Thereof"). Inventors showed, for example, that chondrocytes encapsulated within peptide structures are able to synthesize extracellular matrix components, as described herein.

In order to further explore the phenomenon of cellular plasticity and to develop ways to modulate and control the division, differentiation, and transdifferentiation of progenitor cells *in vitro*, the inventors decided to examine the effects of encapsulation within peptide hydrogel structures on these parameters. Inventors have discovered now discovered new and unexpected properties of the peptide structures in that they are able to promote differentiation and transdifferentiation of encapsulated cells. The peptide structures are able render progenitor cells permissive for instruction by differentiation-enhancing factors to which they would not respond under standard culture conditions or to alter the differentiation response of cells to such factors.

Somatic cells (LPCs) were isolated from liver as described herein and cultured in three dimensional peptide structures (peptide hydrogels) in various media and in the presence of different growth factor(s). Because of the cellular plasticity exhibited by these cells, they are referred to as "liver progenitor cells" (LPCs). The name LPC was chosen to indicate the tissue of origin, while not restricting expectations for their differentiation and/or self-renewal potential. The peptide hydrogel structures are described in further detail below. As presented in more detail below, the peptide hydrogel provided an environment that altered the phenotypes and differentiation pathways adopted by cells cultured therein. For example, when cultured on traditional plastic culture dishes, rat liver progenitor cells maintain a uniform, flat morphology and do not differentiate well into functional hepatocytes. Such cells fail to express significant amounts of cytochrome P4501A1 (CYP1A1), an enzyme produced by mature hepatocytes. In contrast, rat liver progenitor cells cultured in a three-dimensional peptide hydrogel divide to form spheroidal clusters, reminiscent of the behavior of mature hepatocytes under certain culture conditions. Furthermore, a portion of cells maintained in the hydrogels express CYP1A1.

To further investigate this phenomenon, cells in peptide hydrogels were switched to a defined hepatocyte growth medium. Surprisingly, by 24 hours after the medium was changed, a considerable proportion of the cells acquired a dramatic change in cellular morphology, exhibiting very elongated cell bodies with rudimentary processes. The phenotype of these cells resembled that of neuronal lineages. Staining for various markers characteristic of neuronal lineage cells further indicated that the cells were developing along a non-hepatocyte pathway. Results were consistent with a conclusion that these cells possessed features of neuronal precursors. Acquisition of neuronal-like phenotypes was dependent on the presence of various growth factors (EGF or EGF/NGF) in the culture medium and did not occur when cells were cultured on plates in identical medium. Thus within the hydrogel liver progenitor cells are being instructed to divide and differentiate both in hepatocyte-like pathways and in pathways distinct from those assumed by normal hepatocytes. Thus encapsulation within the peptide hydrogel structures rendered cells permissive for instruction by various growth factors. In the presence of either EGF or EGF/NGF, the liver progenitor cells cultured within the peptide hydrogel structure exhibited the ability to differentiate along a hepatocyte-like lineage and to transdifferentiate along a neuronal lineage. Cells grown in soft agar did not proliferate or exhibit either hepatocyte-like or neuronal-like phenotypes, suggesting that the mere arrangement of the cells in three dimensions is not sufficient to allow these effects.

The data presented herein indicates that a three-dimensional nanoscale environment comprising a peptide hydrogel is able to support the growth and differentiation of cells having properties of liver cell precursors, liver stem cells, hepatocytes, and oval cells. In addition, the hydrogel is able to support the growth, trans-differentiation, and differentiation of cells having properties of neural lineage cells. Cell types such as these exist in a wide variety of three-dimensional environments within the body. Thus the hydrogel is able to support the growth, trans-differentiation, and/or differentiation of a wide range of cell types, *e*.*g*., cell types that may exist in a variety of different three-dimensional environments within the body. Such cells include cells derived from liver tissue and cells derived from neural tissue.

The invention thus provides a composition comprising a peptide hydrogel structure encapsulating progenitor cells and a differentiation-enhancing factor such as a growth factor, which may be present or added to medium in which the structure is cultured. In certain embodiments, the progenitor cell is a liver progenitor cell, *i*.*e*., a cell that does not express a fully differentiated liver-specific phenotype but that has the capacity, under appropriate conditions, to give rise to cells that assume a liver-specific cellular morphology and/or express a marker characteristic of a liver-specific cell. In certain embodiments, the liver progenitor cell is isolated from the liver. In certain other embodiments, the liver progenitor cell may be isolated from another organ, *e*.*g*., from the bone marrow.

The invention further provides methods for enhancing cellular differentiation comprising encapsulating a progenitor cell within a peptide hydrogel structure and culturing the encapsulated cells in the presence of one or more differentiation-enhancing factors such as a growth factor for a time sufficient to allow differentiation or transdifferentiation to occur. In certain embodiments, the progenitor cell is a liver progenitor cell. The process of differentiation or transdifferentiation may occur within the cell or within progeny or descendants of the cell. The differentiation may be along a liver-specific lineage pathway, *e*.*g*., towards a hepatocyte-like phenotype. The transdifferentiation may be along a neuronal-like pathway, *e*.*g*., towards a neuronal precursor, a neuron, or a glial cell. In other embodiments, the progenitor cell is a progenitor cell for one of the following cell types: chondrocytes, heart cells, bone marrow cells, peristeal cells, perichondrial cells, fibroblasts, neuronal cells, hippocampal cells, epidermal cells, endothelial cells, keratinocytes, basal cells, spinous cells, granular cells, non-human embryonic stem cells, ovarian cells, pancreatic cells, cervical cells, liver cells, or foreskin cells. Both transdifferentiation and differentiation may occur among the progeny of the encapsulated cells, and different cells within the peptide structure may transdifferentiate along different lineage pathways.

While inventors have not yet identified the precise mechanism by which the peptide structures exert their effect, and while not wishing to be bound by any theory, inventors propose a number of possibilities that may be systematically explored and parameters that may be varied in order to refine and expand upon the discoveries and inventions described herein. For example, the peptide sequence, length, and concentration may be varied, which may in turn affect the stiffness, oxygen tension, fraction of cell surface in contact with the gel, and/or growth factor gradients within the structure. All such improvements and refinements are within the scope of the invention.

The sections below address various aspects of the invention including peptide structures, cells that may be used in the practice of the invention, methods of encapsulating cells within peptide structures of the invention, and inventive culture techniques that may be employed in the context of cells cultured in or on peptide structures, in order to achieve various effects on cell division and phenotype. Methods for monitoring the effects of the peptide structures and culture conditions on cell division and phenotype are also presented. Among the embodiments of the invention are (i) an *in vitro* culture system for studying stem and progenitor cells and their differentiation and transdifferentiation properties; (ii) an *in vitro* culture system for growing hepatocyte-like cells and/or mature hepatocytes, which may be used, for example, to propagate hepatotropic viruses; (iii) a system for controlling and manipulating cell differentiation and transdifferentiation *in vitro,* from which cells can be extracted and then either maintained *in vitro* or administered to a subject; and (iv) a system for controlling and manipulating cell differentiation and transdifferentiation *in vitro* within a structure that is to be implanted within a subject. Various other embodiments of the invention such as cell culture kits and assay systems are also described.

### Exemplary cell types for encapsulation and/or differentiation

Any type of progenitor cell is appropriate for use in the present invention. Sources of the cells may also include fetal or adult mammals or established cell lines. Numerous established cell lines are known in the art, many of which are available through the American Type Culture Collection (http://www.atcc.org), which also provides references describing these cell lines. In discussing cells, progenitor cells, stem cells, and cell lines, the phrase "derived from" indicates that a cell is obtained from a particular source, or that the cell is a descendant of a cell obtained from that source. For example, a liver-derived cell is a cell that is obtained from the liver or the progeny or descendant of such a cell. When the term "progeny" is used herein, it refers not only to the immediate products of cell division but also to the products of subsequent cell divisions, *i*.*e*., to cells that are descendants of a particular cell. A cell that is derived from a cell line is a member of that cell line or is the progeny or descendant of a cell that is a member of that cell line. A cell derived from an organ, tissue, individual, cell line, *etc*., may be modified *in vitro* after it is obtained. Such a cell is still considered to be derived from the original source.

Although the examples describe isolation and encapsulation of liver progenitor cells, the invention is not limited to those embodiments. Stem cells and progenitor cells are known to exist in a wide variety of tissues (see, for example, references cited above). Cells may be obtained from any body tissue, organ, or structure. Thus cells may be derived, for example, from brain, nervous tissue, bone marrow, esophagus, fallopian tube, heart, pancreas, intestines, gallbladder, kidney, liver, lung, ovaries, prostate, bladder, spinal cord, spleen, stomach, testes, thymus, thyroid, trachea, ureter, urethra, or uterus. Non-human Embryonic stem cells may be used. As discussed above, mature hepatocytes and other mature liver cell types are able to divide and give rise to daughter cells of the same type. However, the existence, identity, and origin of liver stem cells or liver progenitor cells and their role in liver regeneration remains unclear (See, *e*.*g*., Peterson, B. et al., "Bone Marrow as a Potential Source of Hepatic Oval Cells", Science, 284, May 14, 1999; Paku, S., et al., "Origin and Structural Evolution of the Early Proliferating Oval Cells in Rat Liver", Am. J. Path., 158(4), 2001.) As described in herein, liver progenitor cells may be conveniently harvested from rat liver. In the case of a human, liver cells may be harvested surgically or using a less invasive liver biopsy technique. Liver progenitor cells may be isolated from a mixed population of cells residing in the liver. The bone marrow is likely to be a good source of progenitor cells having broad transdifferentiation potential (*i*.*e*., potential to differentiate into multiple different cell types). Thus the invention specifically contemplates the use of progenitor cells derived from bone marrow.

Cells harvested from an individual may be used either with or without a period of expansion in culture. Alternately, cells that have been propagated in culture as a stable cell line may be used. In certain embodiments, the cells are autologous or allogeneic. In certain embodiments, cells are harvested from a subject, *e*.*g*., a patient, and a clonal cell line is derived from one or more of these cells. Clonal lines of progenitor cells, including somatic tissue stem cells may be obtained by limiting dilution plating or single cell sorting. Methods for deriving clonal cell lines are well known in the art and are described for example in Puck, T. T. and Marcus, P. I., J. (1956) Experimental Medicine 103, 653; Nias, A. H. W. and Lajtha, L. G. (1965)" Clone size distribution in the study of inhomogeneity of growth rates in tissue culture" in Cell Culture, C. V. Ramakrishnan, ed. (Dr. W. Junk Publishers, Netherlands), and Leong, P.-M., Thilly, W. G., and Morgenthaler, S. (1985) Variance estimation in single-cell mutation assays: comparison to experimental observations in human lymphoblasts at 4 gene loci. Cells from the cell line may be used in the practice of the invention. When intended for treatment of a particular patient, cells from a matched donor may be advantageously used. Cells isolated from an individual or maintained as a cell line may be cultured according to any appropriate technique including standard cell culture techniques prior to their use in the practice of the present invention.

It may be desirable to genetically modify the cells prior to their use in the invention. Numerous methods for introducing exogenous genetic material into cells are well known in the art. In certain embodiments, it may be desirable to introduce a selectable marker into the cells. In certain embodiments, it may be desirable to introduce a gene that encodes a selectable marker (*e*.*g*., a gene encoding a protein that confers drug resistance) or a detectable marker (*e*.*g*., GFP) under the control of a tissue-specific promoter. Expression of the detectable marker may then be used as a means to determine whether the cell or its progeny has differentiated or transdifferentiated along a particular cell lineage pathway characteristic of that tissue. The marker may also be used as a means of isolating cells that have differentiated or transdifferentiated along a particular pathway, *e*.*g*., by using immunological methods, FACS, *etc*., such other methods as are well known in the art. Numerous selectable and detectable markers are known in the art. In addition, tissue-specific, organ-specific, and lineage-specific promoters are well known. Genes may be introduced under the control of either a constitutive or an inducible promoter of which many are known in the art.

In certain embodiments, a therapeutically desirable genetic modification may be made. For example, in a case where an individual harbors a mutation in a particular gene it may be desirable to introduce a wild-type copy of the gene into the progenitor cell for gene therapy purposes. This approach may be particularly useful in the case of certain liver diseases (See, *e*.*g*., Grompe, M., "Liver repopulation for the treatment of metabolic diseases", J. Inherit. Metab. Dis., 24, 231-244, 2001 for a discussion of some of these diseases.) In certain embodiments, it may be desired to introduce a gene encoding a particular receptor, *e*.*g*., a growth factor receptor, in order to confer or enhance a particular differentiation or transdifferentiation potential by allowing cells to respond to the growth factor.

In certain embodiments, it is desirable to enrich for progenitor cells. Various methods of enrichment may be used. For example, the presence of particular markers may be used to remove or otherwise exclude cells that have differentiated and reached a point at which they do not qualify as progenitor cells. Techniques for removing cells or sorting cells are well known in the art and include the use of flow cytometry (*e*.*g*., FACS) and various other methods employing antibodies that recognize particular cell types.

In certain embodiments, it may be desirable to employ cells in which asymmetric cell kinetics have been suppressed, as described in detail in the U.S. provisional patent application entitled "Methods for Ex Vivo Propagation of Somatic Stem Cells", filed July 10, 2001, on which one of the present inventors (Dr. James Sherley) is a co-inventor. Suppression of asymmetric cell kinetics (SACK) allows the expansion of a stem cell population without the dilution that inevitably occurs under conditions in which a stem cell gives rise to both another stem cell and a more fully differentiated cell. The technique may be applied to cells harvested from an individual or to a cell line. A clonal cell line may be established from cells to which SACK has been applied.

### Differentiation-enhancing factors and agents to promote differentiation of progenitor cells

As is well known in the art, numerous environmental factors are likely to play key roles in cell differentiation and transdifferentiation. These may include physical or mechanical factors such as compressive forces, contact with substrate, *etc.* The extracellular matrix is known to exert profound effects on cell development. In addition, cell-cell contacts may play an important role. In addition, a large number of specific growth and/or differentiation factors have been described. Among these are epidermal growth factor (EGF), nerve growth factor (NGF), platelet-derived growth factor (PDGF), hepatocyte growth factor (HGF), brain-derived neurotrophic factor (BDNF), insulin-like growth factor-I (IGF-I), transforming growth factor β (TGF- β), *etc*. The foregoing list is merely representative. Over 2,000 growth factors have been identified, and one of ordinary skill in the art will be able to select and to test these factors appropriately depending upon the differentiation or transdifferentiation properties desired. The growth factors may be provided in a pure form or as components of a more complex biological mixture such as serum. The growth factors may be present within the culture medium of a peptide hydrogel structure in which cells are encapsulated and/or may be encapsulated within the structure itself. In addition, it is well known in the art that different concentrations of a particular growth factor may exert different effects on target cells. One of ordinary skill in the art will be able to test a range of concentrations and combinations in order to achieve the desired effects.

In addition to growth factors, various other chemical stimuli or conditions may influence cell differentiation or transdifferentiation, and any of these may be used in the context of the present invention. Among such stimuli are activators of the phosphatidyl inositol pathway, or other factors that increase levels of inositol trisphosphate and/or intracellular Ca concentrations, activation of protein kinase C and/or other cellular kinases, *etc.* The presence of small molecules including small organic molecules or metal ions may also influence cell differentiation or transdifferentiation and may be used in the practice of the invention.

### Methods for monitoring cell division and phenotype

As is well known in the art, there are a number methods for monitoring cell division and for assessing various aspects of cell behavior and phenotype. In general, any appropriate method may be employed to investigate and assess the effects of culturing cells in or on the peptide structures described herein. In addition, the effects of the cells on the overall composition and properties of the cell/hydrogel assembly may be monitored. Such features as protein content, strength, *etc*., can be examined.

Cell viability may be assessed by examining vital dye exclusion (*e*.*g*., trypan blue exclusion). Cell division may be observed by light microscopy and is indicated by the presence of mitotic figures. mRNA and/or protein synthesis may also be measured by techniques well known in the art. An increase in cell number accompanying division may also be observed, *e*.*g*., by counting with a hemacytometer. Morphological changes such as cell rounding may also accompany division. DNA synthesis may be monitored by detecting and/or measuring incorporation of various substances such as radiolabeled nucleotides (*e*.*g*., ³[H] thymidine), bromodeoxyuridine (BrdU), *etc*., into DNA.

Cell differentiation and transdifferentiation can be assessed based on a number of parameters, including morphology. Cell differentiation and transdifferentiation may also be assessed by monitoring gene expression (*e*.*g*., by detecting and/or measuring mRNA by Northern blot analysis, microarray analysis, *etc*.) or by detecting and/or measuring the presence of certain polypeptides commonly known as markers. The latter approach is widely used, and markers characteristic of numerous different cell types have been identified. In some cases such markers identify a cell as belonging to one of a restricted number of cell types but do not uniquely identify the exact cell type. In other cases the presence of a marker is believed to identify a cell as being of a particular cell type and no other. In addition to allowing identification of cell type, certain markers are characteristic of cells that lack or possess a particular feature or functional capability (*e*.*g*., cells that are post-mitotic).

The variety of markers is immense, and new markers are routinely being identified. Of particular significance in the context of the present invention are markers that may be used to identify cells that are able to differentiate or transdifferentiating into cell types characteristic of the mature liver and markers that may be used to identify cells that have differentiated into such cells. Also of particular significance in the context of the present invention are markers that may be used to identify cells capable of differentiating or transdifferentiating along a neuronal lineage pathway, *i*.*e*., a pathway that ultimately results in the production of neurons or glia. A variety of markers for liver precursor cells and for cells that are found in the mature liver are known in the art and are described, for example, in Grisham, J. and Thorgeirsson, S., "Liver stem cells", in Potten, C. (ed.), Stem Cells, Academic Press, San Diego, 1997. A selection of markers appropriate for assessing differentiation along a liver cell pathway include the embryonic liver and oval cell marker alpha-fetoprotein (Shiojiri et al., Cancer Res. 51, 2611-2620, 1991); the hepatocyte and oval cells marker albumin (Houssaint, E., Cell Differ. 9, 269-279, 1980); various cytochrome P450 enzymes including CYP1A1 and CYP1A2 (present in hepatocytes, but nonspecific); C/EBPα, a CCAAT enhanced-binding protein highly expressed in hepatocytes and other endodermic tissues (Wang et al., Science 269, 1108-1112, 1995); a marker for all hepatic cells (hepatocytes, biliary duct and oval cells) cytokeratin 18 (CK18) (Van Eyken, P.et al., Lab. Invest. 59, 52-59, 1988); and a specific biliary duct marker cytokeratin 19 (CK19) (Bouwens, L. et al., Diabetes 43, 1279-1283, 1994). The presence of binucleated cells is indicative of hepatocytes. A variety of markers for neuronal lineage cells are mentioned in Woodbury, D., et al., J. Neurosci. Res. 61: 364-370, 2000 and in Mahendra S. Rao (ed.) Stem cells and CNS development, Totowa, N.J. : Humana Press, 2001. Among the markers appropriate for assessing differentiation or transdifferentiation along a neuronal lineage pathway are nestin, GFAP, and NeuN. These markers are discussed further below.

Nestin is an intermediate filament protein expressed in neuroepithelial neuronal precursor stem cells, and its expression decreases with neuronal maturation (Lendahl, U., et al., "CNS stem cells express a new class of intermediate filament protein", Cell, 60:585-595, 1990. NeuN is a neuron-specific marker expressed in postmitotic cells (Sarnat, H., et al., "Neuronal nuclear antigen (NeuN): a marker of neuronal maturation in early human feta nervous system", Brain Research, 20:88-94, 1998). Glial fibrillarary acidic protein (GFAP) is a classic glial astrocyte marker. These markers have been employed to demonstrate the applicability of certain embodiments of the invention. Numerous other markers for neuronal lineage cells are known in the art.

### Kits and methods for enhancing differentiation

The invention provides kits that may be used for enhancing cell differentiation and/or transdifferentiation. The kits comprise a peptide hydrogel of the invention, which may be provided in dry or lyophylized form. The kits may further comprise one or more of the following elements: instructions for encapsulating cells within a peptide hydrogel structure and for other uses of the system, instructions for inducing cells to differentiate or transdifferentiate within the scaffold, a vessel in which the encapsulation may be performed, a liquid in which the peptide can be dissolved, an electrolyte for initiating peptide self-assembly, medium for tissue culture, cells that may be encapsulated, differentiation-enhancing agents, *etc.* Additional elements may also be included.

In addition, the invention provides assay systems and methods for testing compounds. Since the liver is the major organ that metabolizes a wide range of foreign and endogenous compounds including drugs, it is great importance to determine the effects of such compounds on the liver, *e*.*g*., their ability to induce liver enzymes, and the ability of the liver to metabolize such drugs. Traditionally such assessments have initially been performed using *in vitro* systems such as microsomes, or *in vivo* animal models. However, these approaches have significant drawbacks. The microsome system does not allow for tests that include the possible effects of cell membrane barriers or other features of an intact cell. Animal models are expensive and time-consuming to employ. In addition, animal liver cells may metabolize compounds differently to human liver cells due, for example, to a different cytochrome P450 profile. The difficulties of culturing hepatocytes *in vitro* have precluded the development of cell-based assay systems.

The present invention provides an assay system for a compound comprising a peptide hydrogel structure encapsulating liver progenitor cells (non-human animal or human), wherein the liver progenitor cells have been induced to differentiate along a hepatocyte pathway and exhibit features of a mature hepatocyte such as expression of metabolic enzymes such as cytochrome P450 enzymes. According to the invention a compound is applied to the system, and various parameters are tested. For example, the ability of the encapsulated cells to metabolize the compound may be tested. The ability of the compound to induce or inhibit P450 enzymes may be assessed, *e*.*g*., using a fluorescent or otherwise conveniently detectable substrate such as that as described herein. The effect of the compound on other liver parameters, *e*.*g*., the synthesis of proteins such as albumin, alanine transaminase, aspartate transaminase, *etc*., may be assessed. Methods for measuring a wide variety of proteins synthesized by the normal liver are well known in the art. Many drugs and other foreign compounds are known to induce or inhibit liver enzymes, and such effects present significant safety concerns in terms of drug development. It is desirable to test the effects of new medication candidates upon liver enzymes. The present invention provides a way of performing such tests *in vitro,* which may be used to predict potential dangers posed by new drug candidates, drug interactions, *etc.*

### Encapsulation of Adult Rat Liver Precursor Cells in Peptide Hydrogels

The methods described below were used to encapsulate adult rat liver precursor cells in peptide scaffolds. These methods can be applied to any other cell type.

### Rat somatic liver progenitor cells methods

The isolation and preparation of the liver progenitor cells is described in detail in U.S. provisional patent application "Methods for Ex Vivo Propagation of Somatic Stem Cells", filed July 10, 2001, on which one of the present inventors (Dr. James Sherley) is a co-inventor. Briefly, xanthosine (Xs) was used as a pharmacological agent allowing for a switch from the default asymmetric kinetics normally exhibited by tissue stem cells to exponential kinetics. When xanthosine was removed, clonal rat liver epithelial stem cell lines that retained the ability to divide by asymmetric cell kinetics were isolated. Rat liver epithelial cells found in the low-speed supernatant of centrifuged cells from *in situ* collagenase-perfused livers were isolated by limiting dilution cloning in the absence or presence of Xs. The cell line used in this work, which is termed LPC-8, LPC-8.1 (a sub-clone of LPC-8), or Xs-D8, or D8 elsewhere herein and in the above-mentioned provisional patent application, has now been maintained continuously in culture for more than 80 cell doublings. Early passage cells were cryo-preserved in liquid nitrogen and can be reestablished in culture after thawing.

### Methods for encapsulation of LPC-8 Cells

Cells were maintained in plastic culture dishes with DMEM supplemented with 10% dialyzed fetal bovine serum (FBS), penicillin, and streptomycin. When cells reached 80% confluence they were harvested by treatment with trypsin. The cell suspension was washed with DMEM/10%FBS/pen/strep and then resuspended in an acqueous solution of 10% sucrose. Cells were counted using a hemacytometer.

RAD16-I peptide (sequence AcN-RADARADARADARADA-CNH₂) [theoretical MW=1712.74 and MW by Mass spectra=1712.64] was obtained from ResGen Invitrogen Corporation. A RAD16-I peptide solution was prepared by dissolving peptide in deionized, distilled, sterile water at a concentration of 0.5% w/v. The RAD16-I solution was equilibrated with PBS (phosphate buffered saline) to a final concentration of 1X prior to mixing with the cell suspension in order to bring the pH within a physiologic range.

Cells were mixed with RAD16-I solution at a final concentration of approximately 100,000 cells/ml. The cell/peptide mixture was loaded into multiwell (96-well) plates at 50 µl/well. Immediately after loading, 200 µl of culture medium (DMEM/10%FBS/pen/strep) was added to each well, thereby providing an electrolyte concentration sufficient to allow self-assembly of the gel into a three-dimensional structure. After gel self-assembly, the media was changed three to four times to allow proper equilibration of the cell/hydrogel assembly. The final cell density was between 1-2 x 10⁵ cells/ml of hydrogel. The multiwell plates were cultured at 37° C in a standard incubator containing a humidified chamber equilibrated with 5% CO₂. Cell viability was measured by staining with trypan blue according to standard techniques. Viability 24 hours after encapsulation was typically greater than 85%.

### Control Cells

Control cultures of LPC-8.1 cells were initiated at the same time as the encapsulation using a portion of the cell/sucrose suspension. Cells were maintained on plastic culture dishes in DMEM/10%FBS/pen/strep at 37° C in a standard incubator containing a humidified chamber equilibrated with 5% CO₂. Cell viability was measured by staining with trypan blue according to standard techniques. Viability 24 hours after plating was typically greater than 90%.

LPC-8 cells were also grown on the surface of plates coated with assembled RAD 16-1 hydrogel in DMEM/10%FBS/pen/strep and maintained at 37°C in 5% CO₂.

LPC-8 cultures in soft agar were also prepared according to standard protocols.

### BrdU Staining methods

Cell division within the assembled peptide hydrogel was assessed by monitoring incorporation of 5'-bromodeoxyuridine (BrdU). BrdU was added to the culture medium at a final concentration of 10 µM for a period of 18 hours. Following incubation, hydrogel cultures were incubated in BrdU-free medium for two hours. Hydrogels were then washed in PBS, followed by fixation in 2% paraformaldehyde in PBS (pH 7.4) at room temperature for 2 hours. Following fixation, hydrogels were washed several times in PBS and then treated with 0.1% Triton X-100 in PBS for 2 hours at room temperature. To achieve DNA denaturation, hydrogels were then treated with 2N HCl in PBS for 30 minutes at 37° C . Following this treatment, several washes with PBS were performed until a pH of 7.4 in the wash solution was reached. Hydrogels were then incubated in blocking buffer (20% FBS, 0.1% Triton X-100, 1% DMSO in PBS) for four hours at room temperature with slow shaking.

FITC-conjugated, anti-BrdU mouse monoclonal antibody IgG₁ (BD Pharmingen, catalog number 33284X) was preincubated in blocking buffer for one hour at room temperature at a dilution of 1:400 and then added to samples overnight at 4° C with slow shaking. Following incubation, hydrogels were washed three times with blocking buffer for two hours, following which a final one hour wash with PBS was performed.

### Light microscopy and photography methods

Control cells and hydrogels were observed under a Nikon microscope TE300 with phase contrast and fluorescence using an Openlab acquisition system mounted with a Hamamatsu video camera. Pictures obtained represent a single optical plane observed with phase contrast and fluorescence.

### Results

Encapsulated and control cells were observed daily by light microscopy. The transparency of the assembled peptide structure readily allowed observation and photography of encapsulated cells. Immediately after encapsulation, the cells were substantially uniformly dispersed throughout the gel as isolated single cells rather than in groups or clumps. Fig. 22A shows encapsulated LPCs immediately after encapsulation, illustrating uniform, single-cell dispersion. When cells divide symmetrically in three dimensions, they tend to form compact, spheroidal clusters. However, if cell division is asymmetric, since only one cell of the initial cluster divides, a linear or sometimes branched cluster is formed. Fig. 22B shows encapsulated LPCs two days after encapsulation. As shown in Fig. 22B, during the first two days of culture encapsulated cells started forming small linear clusters suggesting some asymmetric mitotic activity. Over the next two to three days the clusters enlarged and adopted a spherical shape. Fig. 22C shows spheroid formation four to five days after encapsulation.

Cells cultured on plates coated with RAD16-I peptide hydrogel behaved similarly to those cultured directly on the plastic plate surface, continuing to divide and showing no evidence of differentiation or cluster formation. No formation of clusters or other evidence of cell division was observed in the soft agar cultures, indicating that a three-dimensional environment alone is not permissive for cell division.

Incorporation of BrdU indicated that a fraction of the cells in clusters that developed in the peptide structures were actively dividing. Fig. 22D shows the same spheroid as in Figure 3c, after staining for incorporation of BrdU into DNA. Clusters typically reached an average cell number of approximately 20-30, at which time further increase in cluster size was not observed. However, the possibility that one or two cell within the cluster continued to divide at a low rate cannot be excluded and is, in fact, suggested by the facts that (as described below), not all cells in the cluster are positive for CYP1A1, and when clusters are extracted from hydrogels and plated on flat dishes where the morphological changes that accompany cell division are readily observed, one or two cells continue to divide.

### Differentiation Properties of Encapsulated Cells

As described below, the differentiation properties of the encapsulated cells were assessed.

### Cell culture methods

Cells were cultured in standard plastic culture dishes and encapsulated in peptide hydrogels as described in the previous section. Controls and encapsulated cells were initially maintained in identical media (DMEM/10%FBS/pen/strep) and under identical culture conditions. For some experiments cells that had been cultured in DMEM/10%FBS/pen/strep for one week were switched to a defined hepatocyte growth medium (HGM: Base medium: DMEM from Gibco, #11054-020 (500ml), 0.015 g L-Proline, Sigma # P-4655, 0.05 g L-Ornithine, Sigma # O-6503, 0.305 g Niacinimide, Sigma # N-0636, 0.5 g D-(+)-Glucose, Sigma # G-7021, 1 g D-(+)-galactose, Sigma # G-5388, 1 g Bovine Serum Albumin, fraction V, Sigma # A-9647; 500 ml of trace metal solutions: ZnCl2: 0.0272 g, ZnSO4-7 H2O: 0.0375 g, CuSO4-5 H2O: 0.01 g, MnSO4: 0.00125 g; 12.5 ml L-Glutamine, final concentration [5 mM], Gibco # 25030-081, 0.5 ml Insulin-Transferin-Sodium Selenite, Roche # 1074547, 0.4 ml Dexamethasone, final concentration [0.1 microM], Sigma # D-8893, 5 ml penicillin/streptomycin (100 x solution), Epidermal Growth Factor (EGF) final concentration [20 ng/ml], Collaborative # 40001). For other experiments, cells that had been cultured in DMEM/10%FBS/pen/strep for one week were switched to DMEM/pen/strep containing 1% FBS. For some experiments LPC-8.1 cells were induced to form floating spheroids by resuspending trypsinized cells in glass spinner flasks at a density of 10⁶ cells per 30 mL.

### Assessment of Cytochrome P450 1A1 activity.

After two weeks, cultures were incubated in the presence of 7-ethoxyresofurin, a specific substrate for the enzyme whose cleavage produces the fluorescent residue resofurin (excitation: 574 nm, emission: 596 nm). The incubations were carried out at 37 °C for 30 minutes. After incubation the hydrogels were washed three times with culture media. Cells were then monitored under a Nikon microscope TE 300 with phase contrast and fluorescence using an Openlab data acquisition system mounted with a Hamamatsu video camera. Pictures obtained represent a single optical plane observed with phase contrast and fluorescence. For all experiments multiple clusters were observed, and results represent typical observations.

For quantitative measurement of CYP1A1 activity, 7-ethoxyresofurin O-deethylase activity (EROD) was measured in culture supernatants as previously described (Tokudome, S., Yamamoto, T., and Kuroiwa, Y. Involvement of CYP1A2 in mexiletine metabolism. Br. J Clin Pharmacol 46, 55-62, 1998). Briefly, the two types of culture were incubated in the presence 0.1 mM of 7-ethoxyresofurin at 37°C for 30 minutes, following which the supernatant was harvested. The presence of the product resofurin was measured fluorometrically. Known concentrations of the standard (resofurin) were used to generate a standard curve. The total number of cells present in both culture dishes and in the peptide hydrogels was determined using a hematocytometer. EROD activity was expressed as pM of resofurin/cell/hr. Approximately 100 clusters were analyzed.

### Results

In order to determine whether the cells were differentiating into hepatic lineages, Cytochrome P450 1A1 (CYP1A1) enzyme activity, characteristic of fully differentiated hepatocytes, was analyzed over time both visually and using a quantitative assay in cells grown on plastic culture plates and in cells encapsulated within the peptide hydrogel structure. As shown in Fig. 23A, LPCs growing as a monolayer on a standard plastic culture dish did not display detectable EROD activity two weeks after plating. In contrast, as shown in Fig. 23B, LPCs in spheroids growing over the same time period in an assembled peptide structure exhibited abundant EROD activity at two weeks as evidenced by the red staining seen within cells. All spheroids examined contained EROD positive cells. The percentage of positive cells within a cluster ranged between approximately 50 and 80 percent.

EROD activity in cultures of encapsulated LPC-8.1 cells was undetectable one day after encapsulation but rose dramatically by three days, reaching a maximum of approximately 0.14 pmol/cell/hr by day seven. Activity declined slightly thereafter but remained at approximately 0.08 pmol/cell/hr on day 15. Fig. 22C is a graph showing EROD activity of LPC spheroids growing in an assembled peptide structure during a time course of two weeks, starting 24 hours after encapsulation. The slope of the graph suggests that EROD activity was reaching a plateau by two weeks following encapsulation.

Switching control cells growing on standard tissue culture plates to hepatocyte growth medium had no effect on EROD activity, which remained at essentially undetectable levels after one week of culture in HGM. In contrast, switching encapsulated cells to HGM resulted in a 3 to 4 fold increase in EROD activity after one week of culture relative to the level of EROD activity in encapsulated cells maintained in DMEM/10%FBS/pen/strep. Switching encapsulated cells to HGM also resulted in a dramatic change in cellular morphology in a fraction of the cells, as described in the section below. EROD activity was also examined in LPC-8.1 cells grown on standard tissue culture dishes under serum starvation conditions (DMEM with 1%FBS) and in LPC-8.1 cells induced to form floating spheroids. EROD activity in serum-starved cells and cells that formed floating spheroids remained at extremely low levels when measured one week after plating. Fig. 22D is a graph summarizing the data on comparative CYP1A1 activity of LPC-8.1 cells maintained under these various culture conditions: monolayer on plastic dish with low (1%) serum concentration (serum starvation); spheroid culture obtained by growing LPCs in liquid culture at high density; spheroid culture in assembled peptide structure growing in DMEM with 10% FBS; spheroid culture in assembled peptide structure growing in HGM.

To the inventors' knowledge, the LPC-peptide hydrogel system is the first described *in vitro* model for transdifferentiation by a clonal stem cell that retain differentiation properties after long-term growth in culture.

### Effects of Growth Factors on Cells Encapsulated in Peptide Hydrogel Structures

The effect of growth factors on the differentiation of encapsulated cells was also determined, as described below.

### Cells and cell culture methods

LPC-8.1 cells were grown either in DMEM/10% FBS/pen/strep as above or in hepatocyte growth medium. For some experiments human Epidermal Growth Factor (EGF) at final concentration of 20ng/mL (R&D Systems, catalog number: 236-EG-200), rat Beta Neural Growth Factor (β-NGF) at final concentration of 5 ng/mL (R&D, Systems, catalog number: 556-NG-100), and human Platelet Derived Growth Factor (PDGF) at final concentration of 10ng/mL (R&D, System, catalog number: 120-HD-001) was added to the medium (either DMEM or HGM).

P19 and NIH3T3 cells were cultured according to standard protocols as previously described by the American Type Culture Collection (ATCC) (see http://www.atcc.org). Retinoic acid treatment of P19 cells to induce neuron/glia differentiation was performed as previously described (Bain, G., et al., "Neuronlike Cells Derived in Culture from P19 Carcinoma Embryonal Stem Cells", in Culturing Nerve Cells, 2nd edition, Banker, G. and Goslin, K, (eds.), 1998).

### Immunostainin methods

Immunostaining experiments were performed on hydrogel cultures or in cell cultures on laminin-coated cover slips using the following neuronal markers: Nestin and β-tubulin III for neuronal precursors, NeuN for post-mitotic neurons, and GFAP for glia (astrocytes). The samples (hydrogels or laminin-coated cover slips containing cultured cells) were first fixed in 2% paraformaldehyde in PBS (pH 7.4) for 2 hours at room temperature and subsequently washed several times in PBS. They were then treated with 0.1% Triton X-100 in PBS for two hours at room temperature. Following the treatment, the samples were incubated for a minimum of two hours in blocking buffer with slow shaking (20% Fetal Bovine Serum; 0.1% Triton X-100; 1% DMSO in PBS). The primary antibody in each case was preincubated in blocking buffer for one hour at room temperature (normally at final concentration of 1µg/ml) and then added to the samples and incubated overnight at 4 C with slow shaking. The primary antibodies used were: Goat polyclonal IgG anti-GFAP (Santa Cruz Biotechnology, CA, catalog number: sc-6170); Mouse monoclonal IgG₁ anti-NeuN (CHEMICON International, Inc., catalog number: MAB377); Mouse monoclonal IgG₁ anti-Nestin (CHEMICON International, Inc., catalog numberMAB353); and Mouse monoclonal anti-β-tubulin III (CHEMICON International, Inc.). Following the incubation, the samples were washed several times with blocking buffer and subsequently incubated with the appropriate secondary antibodies (1/500 dilution in blocking buffer) overnight at 4 C with slow shaking. The secondary antibodies used were: Goat anti-mouse IgG Rhodamine-conjugated (Santa Cruz Biotechnology); Goat anti-mouse IgG FITC-conjugated (Santa Cruz Biotechnology), and donkey anti-goat -FITC-conjugated (Santa Cruz Biotechnology). The samples were then washed three times with blocking buffer for 2 hours. One final wash with PBS for another hour concluded the treatment. The hydrogels were then monitored under a Nikon microscope TE 300 with phase contrast and fluorescence using an Openlab data acquisition system mounted with a Hamamatsu video camera. Three independent clusters including a total of approximately 100 cells were observed for each staining. Pictures obtained represent one single optical plane observed with phase contrast and fluorescence for FITC and Rhodamine. *BrdU staining methods*

BrdU staining was performed as described above.

### Results

By 24 hours after switching encapsulated LPC-8.1 cells to hepatocyte growth medium, a significant proportion of the cells (10-20%) acquired a dramatic change in cellular morphology, consisting of very elongated cell bodies with rudimentary processes resembling neuronal lineages. Figs. 24A-24G show LPC-8.1 cells growing in an assembled peptide structure after staining for various neuronal markers. The neuronal-like cellular morphology is clearly visible in the phase contrast micrographs in the panels on the left side of the figure (24A, C, D, and G). In contrast, cells maintained in culture on standard tissue culture plates exhibited no similar change in morphology when switched to HGM.

To further explore the neuronal-like phenotype, cells growing in peptide hydrogel structures were stained with a variety of antibodies to markers characteristic of neuronal-lineage cells. Fig. 24B and 24D show that encapsulated LPC 8.1 cells stained positively for the neuronal progenitor markers Nestin and β-tubulinIII respectively. Most of the cells exhibiting a neuronal morphology stained positive for Nestin and β-tubulinIII. However, encapsulated LPC 8.1 cells stained very poorly for NeuN, a marker for differentiated, post-mitotic neurons (Fig. 24F). Encapsulated LPC 8.1 cells were negative for GFAP, a marker for mature, differentiated glial cells (astrocytes). These results indicated that cells in the hydrogel with neuronal morphology have a phenotype more closely related to early neuronal progenitors than to mature neurons of glia. Cells maintained on standard tissue culture plates in HGM were negative for all four markers.

The morphological analysis and marker staining results suggested that encapsulation within the peptide hydrogel structure provided an environment that allowed the cells to alter their differentiation potential in response to extracellular factors present within HGM. The results suggested that the environment of the hydrogel rendered the cells permissive to instruction by differentiation-inducing factors, e.g., growth factors. Since the defined HGM contains 20 ng/ml of epidermal growth factor (EGF), the possibility that the morphological changes were being induced by EGF was explored by comparing encapsulated cells cultured in HGM with encapsulated cells cultured in either HGM without EGF, DMEM/10%FBS without added EGF (*i*.*e*., standard medium) or DMEM/10%FBS with 20 ng/ml EGF added.

When encapsulated cells were cultured in HGM lacking EGF, no change in cellular morphology was observed and most of the cells died within the first 48 hours (see Table 4, which summarizes the results described above, including effects on cell viability, morphology, and staining for neuronal markers when cells were cultured either on plates or in peptide hydrogel structures in either DMEM or HGM with a variety of added factors). This result indicated that the cells were dependent on EGF for survival and suggested that the low concentration of EGF known to be present in FBS is adequate to support survival but not to induce neuronal differentiation when encapsulated cells are cultured in DMEM/10% FBS.

Neither NGF or PDGF was able to support cell viability or induce morphological changes when encapsulated cells were cultured in HGM lacking EGF or in DMEM lacking serum. However, when NGF alone was added to encapsulated hydrogel cultures growing in DMEM with 10% FBS, a similar dramatic change in cell morphology as occurred in HGM medium was observed, *i*.*e*., within 24 hours approximately 10-20% of the cells exhibited a neuronal-like appearance with elongated cell bodies with rudimentary processes. Furthermore, when NGF was present in either DMEM/10% FBS, DMEM/EGF, or HGM/EGF it clearly promoted the number of cells and clusters exhibiting a neuronal morphology, suggesting that the NGF/EGF combination exerts greater neuronal-inducing effects than EGF alone. The presence of PDGF did not induce similar morphological changes either in the presence or absence of EGF. No change in cell morphology was observed when LPC 8.1 cells were cultured in parallel on plastic tissue culture plates in either HGM or DMEM/10% FBS with or without added EGF, NGF, or both.

These results suggested that in the context of the peptide hydrogel structure EGF, while necessary at a low concentration to support cell survival, was able to act as a differentiation-inducing factor at high doses. In contrast, NGF was not able to support cell survival, but in the presence of sufficient EGF to permit cell viability, was able to act as a differentiation-inducing factor. The fact that identical combinations of growth factors and media conditions did not induce either morphological changes or expression of markers for neuronal progenitors when cells were grown on standard culture dishes suggested that the peptide hydrogel structure provided an environment that rendered the cells permissive to instruction by EGF, NGF, or a combination of the two.

In many cases, cells with a neuronal morphology were associated with other cells not exhibiting a neuronal phenotype in small clusters, suggesting the presence of self-renewing neural progenitors. To investigate the possibility of ongoing cell division, cells cultured in HGM in the presence of EGF or EGF plus NGF were independently stained for either Nestin or BrdU. As shown in Figs. 25A-25H, clusters containing cells with neuronal morphology stained positive for both Nestin (25B, 25F) and BrdU (25D, 25H). Nestin positive staining was associated mainly with cells that exhibited a neuronal phenotype (Figures 25A, B, E, and F) while most BrdU positive cells were not Nestin positive. BrdU positive cells tended to be randomly located within the clusters and exhibited a more spherical morphology. In some cases BrdU staining was observed in cells showing a neuronal morphology. The presence of NGF resulted in an enhancement of the number of cells exhibiting a neuronal phenotype.

**Table 4. Peptide hydrogel cultures permit differentiation of somatic liver progenitor cells.**

| Test | Media | Growth Factor | Viability (%) | Morphology * | Phenotype** |
|---|---|---|---|---|---|
| a. | Hydrogel | | | | |
| 1 | 10% FBS | - | > 85 | Spheroid | CYP1A1 |
| 2 | 10% FBS | EGF | > 94 | Spheroid/Neura | CYP1A1/Nestin/β |
| | | | | 1 | -tubulin III |
| | | | | ∼60/40 | |
| 3 | 10% FBS | NGF | > 84 | Neural | Nestin/β-tubulin III |
| 4 | 10% FBS | EGF + NGF | > 95 | Spheroid/Neura | CYP1A1/Nestin/β |
| | | | | 1 | -tubulin III |
| | | | | ∼30/70 | |
| 5 | HGM | - | < 1 | - | |
| 6 | HGM | EGF | > 85 | Spheroid/Neura | CYP1A1/Nestin/β |
| | | | | 1 | -tubulin III |
| | | | | ∼50/50 | |
| 7 | HGM | NGF | < 5 | - | - |
| 8 | HGM | PDGF | < 5 | - | - |
| 9 | HGM | EGF + NGF | > 86 | Spheroid/Neura | CYP1A1/Nestin/β |
| | | | | 1 | -tubulin III |
| | | | | ∼20/80 | |

| b. | Culture Dish | | | | |
|---|---|---|---|---|---|
| 10. | 10% FBS | - | >90 | Flat/Small | None |
| 11. | 10% FBS | EGF | > 95 | Flat/Small | None |
| 12. | 10% FBS | EGF + NGF | > 95 | Flat/Small | None |
| 13 | HGM | - | < 1 | - | - |
| 14 | HGM | EGF | > 90 | Flat/Small | None |
| 15 | HGM | EGF + NGF | > 90 | Flat/Small | None |

| | | | | | |
|---|---|---|---|---|---|
| *By microscopic observation on phase contrast. **By immunostaining with different antibodies against the specific neuronal markers (Nestin, β-tubulin III) or by analyzing the CYP1A1 activity in vivo with 7-Ethoxyresofurin. | | | | | |

### Analysis of Cells Extracted from Peptide Hydrogel Structures

Cells may be recovered from peptide hydrogels as described below.

### Methods for cell and spheroid extraction from the hydrogels

In order to isolate cells and spheroids from the hydrogel cultures they were disrupted mechanically with a Pasteur pipette by several up and down aspirations until about 50% of the cells/clusters were extracted as judged by visual inspection under microscope. The extracted mix was placed on Laminin-coated cover slips (Becton & Dickinson) and incubated overnight in the same media in which they had been cultured. The day after extraction, the remaining pieces of hydrogel were removed by washing the cover slips with fresh media and the attached cells/clusters were incubated for a week in an incubator at 37 °C equilibrated with 5% CO₂. Typically about 50% of the cells/clusters were removed from the hydrogels as judged by counting the number of cells remaining in the hydrogel (using a hemacytometer) and counting the attached cells on the cover slips.

### Results

To study the behavior and phenotype of the peptide-encapsulated cells in more detail and to explore whether the effects of the hydrogel were reversible, LPC-8 cells that had been growing for one week within a peptide hydrogel structure in HGM containing EFG and NGF were extracted from the hydrogels with mechanical disruption and plated onto laminin-coated plates with the same media and growth factor combinations used with the hydrogel. Cells on the plates acquired two basic morphologies over the course of a week: (1) classical hepatocyte shape with expanded cell bodies and mono- or bi-nuclear appearance (Fig. 26A); (2) flat, expanded, with some processes (Fig. 26B). At one week after plating, all cells on the plate stained negative for both Nestin and β-tubulinIII. Cells of class 1 (hepatocyte-like morphology) stained negative for both NeuN and GFAP but did exhibit CYP1A1 activity. In contrast, cells of class 2 (glia-like) stained positive for GFAP (Fig. 26D and 26F) and negative for NeuN and did not exhibit CYP1A1 activity. Extensive cell division occurred on the plate, presumably representing division of undifferentiated progenitor cells but not fully differentiated hepatocytes or glia.

These results suggested that the progenitor neuron-like cells derived from LPCs undergo a further differentiation process after extraction from the gels, apparently along a glial pathway. Consistent with the lack of expression of NeuN, no formation of cells with a typical mature neuron morphology (*i*.*e*., cells with small cell body and extensive neurites) was observed. The continued expression of CYP1A1 activity and the hepatocyte-like morphology of class 1 cells is consistent with the conclusion that culturing LPCs in the peptide hydrogel structure under the media and growth factor conditions described above induced a portion of the cells to differentiate along a pathway that leads ultimately to mature hepatocytes. Thus culture within the peptide hydrogel structure rendered the LPCs plastic for instruction (*e*.*g*., by growth and/or differentiation factors) to produce cells with either hepatocyte or neuronal properties. The results are consistent with the conclusion that the unique properties of the hydrogel environment permitted this type of transdetermination.

### Growth Rate Analysis of LPC-8 Cells after Hydrogel Encapsulation

The growth rate of encapsulated LPC-8 was also measured, as described below.

### Cells, cell culture, and encapsulation methods

LPC 8.1 cells were cultured in DMEM containing 10% fetal bovine serum (FBS) and Xs (400 µM) and monitored for a period of 4 days. Control cells were grown on conventional polystyrene tissue culture dishes. Cells were encapsulated in RAD16-I hydrogel (0.5% w/v) as described above. For some experiments cells were removed from the hydrogels by mechanical disruption and transferred to conventional tissue culture dishes, where they were maintained in the same medium. Since no enzymatic digestion is used, spheroids can be successfully transferred to conventional tissue culture dishes almost entirely intact to facilitate analysis. Cells were counted using a hemacytometer.

### BrdU staining methods

BrdU staining was performed as described herein.

### Results

After the first 24 hr of culture, the number of cells in colonies growing on conventional culture dishes (control) increased exponentially with an approximate doubling time of 24 hr. Cell division was quantified by calculating the relative growth ratio (Rgr), where the mean population of cells per colony duplicates every 24 hr. For the control culture, the cell number increased from 5.3±1.8 cell/colony to 11.0±2.0 cell/colony in one day (Rgr= 2.1) and to 40.6±5.4 in 3 days (Rgr=7.7), where the mean population increased by almost eight (8) times (Table 5). In the peptide hydrogel cultures, the cell growth kinetics were very different. Initially, small clusters of 3 to 6 cells were observed after 24h of culture (4.14±1.9 cells/cluster) (Figs. 27A and 27B and Table 5). In the following days the clusters continued growing and adopted a spheroid morphology (Figs. 27C and 27D). The average number of cells in the spheroids increased slowly when compared to the control cultures (Table 5). For the hydrogel-cultured spheroids, cell density increased from 4.14±1.9 cells/cluster to 6.33±1.9 cells/cluster after 24 hr (Rgr=1.5), and to 15±3.8 cells/cluster after 3 days (Rgr=3.6) (Table 5). This dramatic difference in the growth kinetics suggested that either the generation time significantly increased in the peptide hydrogel cultures or that only a reduced number of cells per spheroid maintained mitogenic activity while the rest underwent cell cycle arrest, or both (Table 5). The effect of the hydrogel on LPC-8 growth occurred despite the presence of Xs, suggesting a different mechanism of cell division control caused by the interaction with the three-dimensional scaffold.

To study the mitotic activity of the spheroids in greater detail, we labeled dividing cells with the thymidine analog, 5'-bromodeoxyuridine (BrdU), which incorporates into DNA during S-phase of the cell cycle. Cells were labeled for 24 hr (*i*.*e*., about 1 generation time) to detect the entire population of dividing cells. Due to the 3-dimensional environment, it was difficult to count the exact number of BrdU⁺ cells in the spheroids by visual inspection. We thus extracted them from the cultures by mechanical disruption of the hydrogel and transferred them to conventional cell culture dishes for analyses, where they formed a small colony on the cell culture dish surface (spheroid-colony), suitable for further visual analysis. We compared BrdU incorporation in 48 hr-old conventional culture dish and 96 hr-old peptide hydrogel culture spheroids. As expected, the BrdU-positive (BrdU⁺) fraction of the cells in the colonies cultured on the dishes was very high (>95%) (Figs. 27E and 27F). In contrast, a reduced fraction of the cells in the spheroid-colonies (<50%) incorporated BrdU, indicating that many cells were arrested after four days in hydrogel cultures (Figs. 27G and 27H). The phenotype of the arrested cells in the spheroid-colonies was unusual; they exhibited an increased cell size with a high incidence of binucleated cells that were often BrdU⁻ (Figs. 27G and 27H). Blue arrows in Figs. 27A-27H indicate binucleated cells.

**Table 5. LPC-8 colony growth rate on regular culture plates or in RAD16-I peptide hydrogels¹.**

| Culture time (hr) | Number of Cells per colony or spheroid (Mean±SD)² | |
|---|---|---|
| | Culture dish (n=3) | RAD 16-I hydrogel (n=8) |
| 24 | 5.3±1.8 | 4.14±1.9 |
| 48 | 11.0±2.0 (∼2.1)³ | 6.33±1,1 (∼1.5) |
| 96 | 40.6±5.4 (∼7.7) | 15.0±3.8 (∼3.6) |

| | | |
|---|---|---|
| ¹LPC-8 cells were cultured on regular 6-well culture dishes (n=3) and plated at initial density of ∼10,000 cells/cm² or encapsulated into RAD16-I peptide hydrogels (0.5% w/v) at initial concentration of ∼100,000 cell/ml (n=8), contained in culture inserts (see Methods). The media used was DMEM with 10% fetal bovine serum and xanthosine (400 µM) to induce exponential growth of LPC-8 cells. Cells were cultured for 24 hr, and the number of cells per colony was counted by visual inspection under a stereo microscope. ²Data is expressed as mean value ± standard deviation (SD). ³Relative growth ratio (Rgr) was calculated after dividing each mean value from 48 hr and 96 hr (Mvₜ, where t= 48 hr or 96 hr) by the initial mean value from 24 hr (Mvᵢ) as follow: Rgr = Mvₜ/ Mvᵢ. | | |

### Phenotypic Characterization of Cells Extracted from Hydrogels after Growth in DMEM

Cells extracted from peptide hydrogels were also characterized as described below.

### Cells, cell culture and encapsulation methods

LPC-8.1 cells were grown in DMEM/10% FBS/pen/strep as described above. Cells were encapsulated in RAD16-I hydrogels as described above. *Cell and Spheroid Extraction from the Hydrogels.* LPC-8.1 cells were cultured for 48 hrs on regular (*i.e.,* conventional) cell culture dishes at a density of ∼10,000 cells/cm² (controls) or for 96 hrs after encapsulation in RAD16-I hydrogels (0.5% w/v) at a density of ∼100,000 cell/ml. Since encapsulated cells grow more slowly than cells grown on regular cell culture dishes, the number of cells present after culturing for 48 hours on regular plates is approximately the same as the number of cells present after culturing encapsulated cells for 96 hours. The spheroid structures were extracted from the hydrogel cultures by mechanical disruption and placed onto regular plates containing the same media used above. Since no enzymatic treatment is used in this process, most of the spheroids can be isolated almost entirely intact. The suspension was incubated overnight to allow spheroid-colony formation.

### Immunostaining methods

Immunostaining was performed as described above except that 4% paraformaldehyde was used. Primary antibodies used were: rabbit polyclonal anti-rat Cytochrome P450 enzyme CYP1A1 and CYP1A2 (CHEMICON International, catalog number: AB1255), rabbit IgG anti-rat albumin-HRP (Accurate Chemicals, YNGRAALBP), rabbit IgG anti-rat C/EBPα (Santa Cruz Biotechnology, catalog number: sc-61), mouse IgG1 monoclonal anti-Cytokeratin 18 (Santa Cruz Biotechnology, sc-6259), mouse IgG1 monoclonal anti-Cytokeratin 19 (Santa Cruz Biotechnology, sc-6278). Secondary antibodies used were: goat anti-mouse IgG Rhodamine-conjugated (Santa Cruz Biotechnology, catalog number: sc-2029); donkey anti-rabbit Rhodamine-conjugated (Santa Cruz Biotechnology, catalog number: sc-2095).

### Measurement of CYP1A1 Activity

Measurement of CYP1A1 activity was performed essentially as described above.

### Light microscopy and photography methods

Light microscopy and photography were performed as described above.

### Results

The data presented above suggested that the presence of various growth factors in the growth environment influences the differentiation and transdifferentiation properties of cells cultured in the hydrogels. To further explore this phenomenon LPC-8 cells were cultured in DMEM/FBS either on conventional tissue culture dishes (control) or in RAD16-I peptide hydrogels for either 48 hours (control) or 96 hours (encapsulated). At this time the cell numbers were approximately equivalent. Cells were then removed from their respective culture environment either by trypsinization (for control cultures) or by mechanical disruption (for spheroids isolated from hydrogels). Cells were then cultured in the same medium overnight on conventional tissue culture dishes to allow spheroid colonies to form. Spheroids were then immunostained for expression of various markers.

Figs. 28A-28L show phenotypic analysis of LPC cells during exponential growth on conventional culture dish either after isolation from regular culture conditions or after isolation from peptide hydrogel culture. Figures 28A, 28C, 28E, 28G, 28I, and 28K show phase contrast micrographs of control or spheroid-derived colonies. Figures 28B, 28D, 28F, 28H, 28J, and 28l show the same colonies immunostained to show expression of C/EPBα, albumin, or CYP1A1/1A2. Table 6 presents a semi-quantitative comparison of expression of these and the additional markers α-fetoprotein, cytokeratin 18 (CK18), and cytokeratin 19 (CK19) in control and spheroid-derived colonies. Table 6 also compares the CYP1A1 activity and the number of binucleated cells (consistent with a hepatocyte phenotype) in control and spheroid-derived colonies.

The expression of α-fetoprotein did not change after culturing LPC-8 cells in hydrogels, suggesting that the treatment did not affect the expression of the fetal marker (Table 6). In contrast, the developmental marker C/EBPα was highly expressed in most of the nucleus of 2 and 10 days-old spheroid-derived colonies compared to the expression in control colonies. This provides evidence that LPC-8 cells encapsulated in hydrogels initiate differentiation along a pathway consistent with hepatic lineage cells (Fig. 28 and Table 6). Other hepatic markers such as albumin and CK18 were upregulated in the spheroid-colonies (Figs. 28E-28H and Table 6), but no detectable levels of CK19 was observed at any time in either control or hydrogel-derived cultures (Table 6). Albumin expression was clearly visible in the cytoplasm, as expected (Fig. 28H). These results suggested that spheroid-colonies displayed a clear hepatocyte or oval cell phenotype because no biliary duct epithelium marker (CK19) was observed (Table 4). The presence in LPC-8 hydrogel-derived spheroid-colonies of the common cellular markers expressed in hepatocytes as well as hepatic oval cells such as albumin, CK18, and α-fetoprotein (Fig. 28 and Table 6) is consistent with the interpretation that the liver stem cell LPC-8 is progressively differentiating in the hydrogels into hepatocytes through an oval cell intermediate as suggested previously for adult hepatic differentiation. Nevertheless, the absence of expression of CK19 (a rat hepatic oval cell marker) in any of the culture conditions tested, the high expression in hydrogel-derived spheroid colonies of C/EBPα, which is mainly expressed in hepatocytes (Fig. 28 and Table 6) and the increasing presence of binucleated cells and expanded cell body (a common phenotype found in hepatocytes) suggests that a terminal differentiation process into hepatocytes may be occurring. Moreover, the majority of the binucleated cells present in the hydrogel-derived spheroid-colonies were also BrdU⁻, suggesting that they were post-mitotic differentiated cells (Figs. 27 and 28 and Table 6). Blue arrows in Figure 28 indicate binucleated cells.

The expression of cytochrome P450 1A1 and 1A2 enzymes (CPY1A1 and CYP1A2) was also up-regulated dramatically in hydrogel-derived spheroid colonies indicating the presence of a potential hepatic function in the hydrogel cultures (Fig. 28I-28L, Table 6). In view of these results we tested activity of CYP1A1 in control and hydrogel cultures over a period of two weeks. Low CYP1A1 activity (0.02±0.01 pmol/cell/hr) was detected in exponentially growing cells on conventional culture dishes (Table 6). However, after only two days of hydrogel culture, an increase in CYP1A1 activity (0.09±0.01 pmol/cell/hr) was observed. This activity was maintained for 10 days (0.09-0.01 pmol/cell/hr), and for up to 2 weeks (0.08±0.01 pmol/cell/hr) (Table 6). This 3 to 4 fold increase in CYP1A1 activity correlates well with the increase in expression of CYP1A1 and CYP1A2 protein (Fig. 28I-28L). Overall, inventors suggest that the results described above indicate that LPC-8 is a clonal, somatic (liver-derived) stem cell that can be instructed to differentiate into a hepatocyte lineage by culturing in a nanoscale environment comprising nanoscale fibers. In particular, inventors suggest that the results described here indicate that LPC-8 cells can be instructed to differentiate into a hepatocyte lineage by culturing them in a three-dimensional nanofiber environment comprising a self-assembling peptide hydrogel. As described in previous sections, under the influence of various agents such as growth factors, LPC-8 cells may also be instructed to differentiate into neural lineages by culturing them in a nanoscale environment comprising nanoscale fibers, *e.g*., by culturing them in a three-dimensional nanofiber environment comprising a self-assembling peptide hydrogel.

**Table 6. Phenotypic characterization of LPC-8 cells during exponential growth in regular culture conditions (2D-culture) or after RAD 16-I peptide hydrogel cultures (3D-culture).**

| Cellular marker¹ | Dish culture (2D)² | RAD 16-I hydrogel (3D) | |
|---|---|---|---|
| | (48 hr) | (48 hr) | (240 hr) |
| α-Fetoprotein | +³ | + | + |
| C/EBPα | - | +++ | +++ |
| Albumin | -/+ | +++ | +++ |
| CK18 | - | + | + |
| CK19 | - | - | - |
| CYP1A1 activity⁴ | 0.02±0.01 | 0.09±0.01 | 0.09-0.01 |
| CYP1A1/CYP1A2 | -/+ | +++ | +++ |
| Binucleated cells | - | + | ++ |

| | | | |
|---|---|---|---|
| ³Quantification in terms of marker expression or phenotype: (-), not detected; (-/+), very low; (+), low; (++), medium; (+++), high. ²Regular polystyrene cell culture dish. ⁴CYP1A1 activity was detected by measuring release of resofurin after incubating the cultures in presence of 7-ethoxyresofurin (see Materials and Methods). ¹α-fetoprotein, present in fetal hepatocytes and hepatic oval cells; C/EBPα, marker for hepatocytes, intestinal epithelial cells, and fat cells; albumin, marker for hepatocytes and hepatic oval cell; CK18, expressed in hepatocytes, biliary duct epithelium and hepatic oval cells; CK19, expressed in biliary duct epithelium and hepatic oval cell; CYP1A1/CYP1A2, present in hepatocytes and other cells; Binucleated cells, common phenotype in hepatocytes. | | | |

### Other Embodiments

While the invention has been described in connection with specific embodiments thereof, it will be understood that it is capable of further modifications.

Except as otherwise indicated, the present invention may employ standard cell culture techniques and media and standard molecular biological and immunological protocols such as are found in reference works such as Freshney. R. I., Culture of Animal Cells: A Manual of Basic Technique, 4th ed., John Wiley & Sons, New York, 2000; Ausubel et al., Current Protocols in Molecular Biology, John Wiley & Sons, NewYork, 2000; Harlow, E., Lane, E., and Harlow, E., (eds.) Using Antibodies: A Laboratory Manual, Cold Spring Harbor Laboratory Press, Cold Spring Harbor, 1998.

## Claims

1. A macroscopic scaffold comprising amphiphilic peptides, living cells and a medium, wherein said peptides are complementary and structurally compatible with alternating hydrophobic and hydrophilic amino acids that are arranged such that each hydrophilic residue in a peptide either hydrogen-bonds or ionically pairs with a hydrophilic residue on an adjacent peptide or is exposed to solvent, and the variation in interpeptide distance is less than 4Å, wherein said peptides self-assemble into a beta-sheet macroscopic scaffold; and wherein said macroscopic scaffold encapsulates the living cells, said cells being present in said macroscopic scaffold in a three-dimensional arrangement, wherein:
said cells are chondrocytes and the scaffold is subjected to application of a
predetermined compression scheme to induce secretion of extracellular matrix components; and the scaffold is cultured for at least 5 weeks.

2. The macroscopic scaffold of claim 1, wherein one or more of the following:
a) the scaffold is subjected to a variable compression scheme; or
b) the scaffold is cultured in medium containing insulin, transferrin, and selenium and less than 10% fetal bovine serum; or
c) the equilibrium compression modulus of said scaffold is at least 40 kPA; or
d) the scaffold comprising a solution that includes insulin, transferrin, and/or selenium and less than 10% fetal bovine serum; or
e) the scaffold further encapsulating a therapeutically active compound or chemoattractant; or
f) said peptides comprise an adhesion site, growth factor binding site, growth factor, or sequence that provides targeting to a cell, tissue, organ, organ system, or site within a mammal; or
g) said cells secrete extracellular matrix components and wherein said secretion of extracellular matrix components increases the equilibrium compression modulus of said scaffold by at least 50 fold;
h) at least 60% of the encapsulated cells are in cell-cell contact with another encapsulated cell or with a cell outside of the scaffold; or
i) the scaffold comprises cells of a desired cell type and chondrocytes, wherein the presence of chondrocytes in said scaffold leads to an increase in stiffness of said scaffold.

3. The macroscopic scaffold as claimed in claim 1, wherein non-chondrocyte cells are also present in said scaffold; and wherein said cells are bone marrow cells, peristeal cells, perichondrial cells, fibroblasts, neuronal cells, hippocampal cells, epidermal cells, endothelial cells, keratinocytes, basal cells, spinous cells, granular cells, non-human embryonic stem cells, ovarian cells, pancreatic cells, cervical cells, liver cells, or foreskin cells.

4. The macroscopic scaffold as claimed in claim 1, in which the equilibrium compression modulus of said scaffold is at least 90 kPA.

5. The macroscopic scaffold as claimed in claim 1, wherein the medium comprises a solution that includes insulin, transferrin, and/or selenium and less than 10% fetal bovine serum; and said solution comprises at least 0.005 mg/L transferrin or at least 0.1 mg/L insulin or at least 0.005 mg/L selenium.

6. A method of forming the macroscopic scaffold of claim 1, said method comprising the steps of:
a) incubating peptides and living cells in an aqueous solution comprising an iso-osmotic solute, wherein said peptides have alternating hydrophobic and hydrophilic amino acids such that when the peptides are exposed to an electrolyte in solution each hydrophilic residue in a peptide either hydrogen-bonds or ionically pairs with a hydrophilic residue on an adjacent peptide or is exposed to solvent and the variation in interpeptide distance is less than 4 Å; and
b) adding an electrolyte to said solution sufficient to initiate self-assembly of said peptides into a beta-sheet macroscopic scaffold, whereby said cells are encapsulated by the formation of said macroscopic scaffold and are present in said macroscopic scaffold in a three-dimensional arrangement;
wherein:
said cells are chondrocytes and the scaffold is subjected to application of a predetermined compression scheme to induce secretion of extracellular matrix components; and the scaffold is cultured for at least 5 weeks.

7. The method of claim 6, wherein:
a) the scaffold is subjected to a variable compression scheme; or
b) the scaffold is cultured in medium containing insulin, transferrin, and selenium and less than 10% fetal bovine serum; or
c) the equilibrium compression modulus of said scaffold is at least 40 kPA, and preferably at least 90 kPA; or
d) the scaffold comprising a solution that includes insulin, transferrin,
and/or selenium and less than 10% fetal bovine serum, and
optionally wherein:
i) said solution comprises at least 0.1 mg/L insulin;
ii) said solution comprises at least 0.005 mg/L transferrin; or
iii) said solution comprises at least 0.005 mg/L selenium; or
e) said solution is contained in a pre-shaped mold dimensioned to determine the volume or shape of said macroscopic scaffold; thereby forming a macroscopic scaffold of predetermined shape or volume; or
f) the method comprises a method of culturing cells and the method includes the step of culturing said cells.

8. The method of claim 6 or 7, wherein:
i) said secretion of extracellular matrix components increases the equilibrium compression modulus of said scaffold by at least 50-fold, and in which case optionally wherein said secretion of extracellular matrix components increases the equilibrium compression modulus of said scaffold by at least 100-fold, and further optionally wherein said secretion of extracellular matrix components increases the equilibrium compression modulus of said scaffold by at least 1 50-fold; or
ii) the dynamic stiffness of the macroscopic scaffold at 1 Hz is at least 1.0 MPa;
iii) said compression scheme comprises subjecting said scaffold to variable pressure;
iv) said compression scheme comprises subjecting said scaffold to dynamic compression between 0.01 and 3 Hz inclusive, superimposed on a static offset compression; and optionally wherein said compression scheme comprises subjecting said scaffold to dynamic compression between 0.1 and 1 Hz inclusive;
v) the strain amplitude is between 0.01 and 10% inclusive; and optionally wherein the strain amplitude is between 1 and 5% inclusive;
vi) said compression scheme comprises subjecting said scaffold to variable or constant pressure for at least one week; and optionally wherein said compression scheme comprises subjecting said scaffold to variable or constant pressure for at least three weeks;
vii) said compression scheme comprises subjecting said scaffold to pressure for one hour and incubating said scaffold in the absence of added pressure for between one and seven hours, inclusive; and optionally wherein said compression scheme is repeated at least four times;
viii) said compression scheme comprises subjecting said scaffold to pressure for 45 minutes and incubating said scaffold in the absence of added pressure for between one and seven hours, inclusive; and optionally
wherein said scaffold is incubated in the absence of added pressure for between five and six hours, inclusive; and further optionally wherein said compression scheme is repeated at least four times;

9. The method of claim 7, or 8, the scaffold is subjected to a predetermined shear loading scheme.

10. The method of claim 7, 8, or 9, wherein:
a) at least 60% of the encapsulated cells are in cell-cell contact with another encapsulated cell;
b) said solution in step a) contains less than 10 mM electrolyte and wherein said peptides do not substantially self-assemble prior to step b); or
c) said scaffold comprises cells of a desired cell type and chondrocytes, wherein the presence of chondrocytes in said scaffold leads to an increase in stiffness of said scaffold.

11. A kit for treating a tissue defect in a patient, said kit comprising:
peptides that have alternating hydrophobic and hydrophilic amino acids such that when the peptides are exposed to an electrolyte in solution each hydrophilic residue in a peptide either hydrogen-bonds or ionically pairs with a hydrophilic residue on an adjacent peptide or is exposed to solvent and the variation in interpeptide distance is less than 4 Å; and sufficient electrolyte to initiate self-assembly of said peptides into a beta-sheet macroscopic scaffold, whereby cells are encapsulated by the formation of said macroscopic scaffold and are present in said macroscopic scaffold in a three-dimensional arrangement; and instructions for initiating self-assembly of said peptides into said scaffold; and wherein:
said cells are chondrocytes and the scaffold is subjected to application of a predetermined compression scheme to induce secretion of extracellular matrix components; and the scaffold is cultured for at least 5 weeks.

12. The kit of claim 11, wherein:
a) the scaffold is subjected to a variable compression scheme; or
b) the scaffold is cultured in medium containing insulin, transferrin, and selenium and less than 10% fetal bovine serum; or
c) the equilibrium compression modulus of said scaffold is at least 40 kPA; or
d) the medium includes insulin, transferrin, and/or selenium; or
e) the kit further comprising a therapeutically active compound or chemoattractant; or
f) said peptides comprise an adhesion site, growth factor binding site, growth factor, or sequence that provides targeting to a cell, tissue, organ, organ system, or site within a mammal; or
g) the kit comprising a means for preparing said cells for addition to a solution comprising peptides; or
h) the kit comprising a means for applying pressure to said scaffold; or
i) the kit comprising a mold for the formation of said scaffold in a desired geometry and/or volume.

13. The scaffold of claim 1 or the kit of claim 11 for use in therapy.

14. The scaffold or kit of claim 13, wherein the therapy is a method of regenerating a tissue and/or is used in a method to treat or prevent a cartilage defect, connective tissue defect, nervous tissue defect, epidermal lining defect, endothelial lining defect, or arthritis.

## Patentansprüche

1. Ein makroskopisches Gerüst, umfassend amphiphile Peptide, lebende Zellen und ein Medium, worin besagte Peptide mit alternierenden hydrophoben und hydrophilen Aminosäuren komplementär und strukturell kompatibel sind, die derart angeordnet sind, dass jeder hydrophile Rest in einem Peptid entweder eine Wasserstoffbrückenbindung oder ein Ionenpaar mit einem hydrophilen Rest auf einem benachbarten Peptid bildet oder dem Lösemittel exponiert ist und die Variation des Interpeptid-Abstands kleiner als 4 Å ist, worin besagte Peptide sich selbst zu einem makroskopischen beta-Faltblatt-Gerüst zusammenbauen; und worin besagtes makroskopisches Gerüst die lebenden Zellen verkapselt, wobei besagte Zellen in besagtem makroskopischem Gerüst in einer dreidimensionalen Anordnung vorhanden sind, worin:
besagte Zellen Chondrocyten sind und das Gerüst einer Anwendung eines vorher festgelegten Kompressionsschemas unterzogen wird, um eine Sekretion extrazellulärer Matrixkomponenten zu induzieren; und das Gerüst wenigstens 5 Wochen kultiviert ist.

2. Das makroskopische Gerüst nach Anspruch 1, worin eines oder mehrere des Folgenden zutrifft:
a) das Gerüst einem variablen Kompressionsschema unterzogen wird; oder
b) das Gerüst in einem Medium kultiviert wird, das Insulin, Transferrin und Selen und weniger als 10% fötales bovines Serum enthält; oder
c) der Gleichgewichts-Kompressionsmodul von besagtem Gerüst wenigstens 40 kPa beträgt; oder
d) das Gerüst eine Lösung umfasst, die Insulin, Transferrin und/oder Selen und weniger als 10% fötales bovines Serum enthält; oder
e) das Gerüst außerdem eine therapeutisch aktive Verbindung oder einen Chemoattraktant verkapselt; oder
f) besagte Peptide eine Adhäsionsstelle, Wachstumsfaktor-Bindungsstelle, Wachstumsfaktor oder Sequenz umfassen, die ein Zielen auf eine Zelle, ein Gewebe, Organ, Organsystem oder eine Stelle in einem Säuger bereitstellt; oder
g) besagte Zellen extrazelluläre Matrixkomponenten sezernieren und worin besagte Sekretion extrazellulärer Matrixkomponenten den Gleichgewichts-Kompressionsmodul von besagtem Gerüst um wenigstens das 50fache erhöht;
h) wenigstens 60% der verkapselten Zellen mit einer weiteren verkapselten Zelle oder einer Zelle außerhalb des Gerüsts in einem Zell-Zell-Kontakt sind; oder
i) das Gerüst Zellen eines erwünschten Zelltyps und Chondrocyten umfasst, worin das Vorhandensein von Chondrocyten in besagtem Gerüst zu einer erhöhten Steifigkeit von besagtem Gerüst führt.

3. Das makroskopische Gerüst wie in Anspruch 1 beansprucht, worin Nichtchondrocyten-Zellen ebenfalls in besagtem Gerüst vorhanden sind; und worin besagte Zellen Knochenmarkzellen, Periostzellen, Perichondriumzellen, Fibroblasten, neuronale Zellen, Hippocampuszellen, Epidermiszellen, Endothelzellen, Keratinocyten, Basalzellen, Stachelzellen, Granularzellen, nicht-humane embryonale Stammzellen, Ovarialzellen, Pankreaszellen, Cervixzellen, Leberzellen oder Vorhautzellen sind.

4. Das makroskopische Gerüst wie in Anspruch 1 beansprucht, in dem der Gleichgewichts-Kompressionsmodul von besagtem Gerüst wenigstens 90 kPa beträgt.

5. Das makroskopische Gerüst wie in Anspruch 1 beansprucht, worin das Medium eine Lösung umfasst, die Insulin, Transferrin und/oder Selen und weniger als 10% fötales bovines Serum enthält; und besagte Lösung wenigstens 0,005 mg/l Transferrin oder wenigstens 0,1 mg/l Insulin oder wenigstens 0,005 mg/l Selen umfasst.

6. Ein Verfahren zur Formung des makroskopischen Gerüsts nach Anspruch 1, wobei besagtes Verfahren die Schritte umfasst:
a) Inkubieren von Peptiden und lebenden Zellen in einer wässrigen Lösung, umfassend einen isoosmotischen gelösten Stoff, worin besagte Peptide alternierende hydrophobe und hydrophile Aminosäuren besitzen, so dass, wenn die Peptide einem Elektrolyt in Lösung exponiert sind, jeder hydrophile Rest in einem Peptid entweder eine Wasserstoffbrückenbindung oder ein Ionenpaar mit einem hydrophilen Rest auf einem benachbarten Peptid bildet oder dem Lösemittel exponiert ist und die Variation des Interpeptid-Abstands kleiner als 4 Å ist; und
b) hinreichende Zugabe eines Elektrolyten zu besagter Lösung, um einen Selbstzusammenbau besagter Peptide zu einem makroskopischen beta-Faltblatt-Gerüst zu initiieren, wodurch besagte Zellen durch die Bildung besagten makroskopischen Gerüsts verkapselt sind und in besagtem makroskopischem Gerüst in einer dreidimensionalen Anordnung vorhanden sind; worin
besagte Zellen Chondrocyten sind und das Gerüst einer Anwendung eines vorher festgelegten Kompressionsschemas unterzogen wird, um eine Sekretion extrazellulärer Matrixkomponenten zu induzieren; und das Gerüst wenigstens 5 Wochen kultiviert wird.

7. Das Verfahren nach Anspruch 6, worin
a) das Gerüst einem variablen Kompressionsschema unterzogen wird; oder
b) das Gerüst in einem Medium kultiviert wird, das Insulin, Transferrin und Selen und weniger als 10% fötales bovines Serum enthält; oder
c) der Gleichgewichts-Kompressionsmodul von besagtem Gerüst wenigstens 40 kPa und vorzugsweise wenigstens 90 kPa beträgt; oder
d) das Gerüst eine Lösung umfasst, die Insulin, Transferrin und/oder Selen und weniger als 10% fötales bovines Serum enthält, und gegebenenfalls worin:
i) besagte Lösung wenigstens 0,1 mg/l Insulin umfasst;
ii) besagte Lösung wenigstens 0,005 mg/l Transferrin umfasst; oder
iii) besagte Lösung wenigstens 0,005 mg/l Selen umfasst; oder
e) besagte Lösung in einer vorgeformten Form enthalten ist, die für eine Festlegung des Volumens oder der Form besagten makroskopischen Gerüsts dimensioniert ist; wobei **dadurch** ein makroskopisches Gerüst mit einer vorher festgelegten Form oder Volumen geformt wird; oder
f) das Verfahren ein Verfahren zur Kultivierung von Zellen umfasst und das Verfahren den Schritt der Kultivierung besagter Zellen umfasst.

8. Das Verfahren nach Anspruch 6 oder 7, worin:
i) besagte Sekretion extrazellulärer Matrixkomponenten den Gleichgewichts-Kompressionsmodul von besagtem Gerüst um wenigstens das 50fache erhöht, und in diesem Fall gegebenenfalls, worin besagte Sekretion extrazellulärer Matrixkomponenten den Gleichgewichts-Kompressionsmodul von besagtem Gerüst um wenigstens das 100fache erhöht, und außerdem gegebenenfalls, worin besagte Sekretion extrazellulärer Matrixkomponenten den Gleichgewichts-Kompressionsmodul von besagtem Gerüst um wenigstens das 150fache erhöht; oder
ii) die dynamische Steifigkeit des makroskopischen Gerüsts bei 1 Hz wenigstens 1,0 MPa beträgt;
iii) besagtes Kompressionsschema ein Aussetzen besagten Gerüsts einem variablen Druck umfasst;
iv) besagtes Kompressionsschema ein Aussetzen besagten Gerüsts einer dynamischen Kompression zwischen einschließlich 0,01 und 3 Hz umfasst, die einer statischen Offset-Kompression überlagert ist; und gegebenenfalls, worin besagtes Kompressionsschema ein Aussetzen besagten Gerüsts einer dynamischen Kompression zwischen einschließlich 0,1 und 1 Hz umfasst;
v) die Verformungsamplitude zwischen einschließlich 0,01 und 10% beträgt; und gegebenenfalls worin die Verformungsamplitude zwischen einschließlich 1 und 5% beträgt;
vi) besagtes Kompressionsschema ein Aussetzen besagten Gerüsts einem variablen oder konstanten Druck über wenigstens eine Woche umfasst; und gegebenenfalls worin besagtes Kompressionsschema ein Aussetzen besagten Gerüsts einem variablen oder konstanten Druck über wenigstens drei Wochen umfasst;
vii) besagtes Kompressionsschema ein Aussetzen besagten Gerüsts einem Druck über eine Stunde und Inkubieren besagten Gerüsts in Abwesenheit des zusätzlichen Drucks zwischen einschließlich einer und sieben Stunden umfasst; und gegebenenfalls worin besagtes Kompressionsschema wenigstens viermal wiederholt wird;
viii) besagtes Kompressionsschema ein Aussetzen besagten Gerüsts einem Druck über 45 Minuten und Inkubieren besagten Gerüsts in Abwesenheit des zusätzlichen Drucks zwischen einschließlich einer und sieben Stunden umfasst; und gegebenenfalls worin besagtes Gerüst in Abwesenheit eines zusätzlichen Drucks zwischen einschließlich fünf und sechs Stunden inkubiert wird; und außerdem gegebenenfalls worin besagtes Kompressionsschema wenigstens viermal wiederholt wird.

9. Das Verfahren nach Anspruch 7 oder 8, worin das Gerüst einem vorher festgelegten Scherbelastungsschema unterzogen wird.

10. Das Verfahren nach Anspruch 7, 8 oder 9, worin:
a) wenigstens 60% der verkapselten Zellen mit einer weiteren verkapselten Zelle in Zell-Zell-Kontakt sind;
b) besagte Lösung in Schritt a) weniger als 10 mM Elektrolyt enthält und worin besagte Peptide sich im Wesentlichen nicht vor Schritt b) selbst zusammenbauen; oder
c) besagtes Gerüst Zellen eines erwünschten Zelltyps und Chondrocyten umfasst, worin das Vorhandensein von Chondrocyten in besagtem Gerüst zu einer erhöhten Steifigkeit von besagtem Gerüst führt.

11. Ein Kit zur Behandlung eines Gewebedefekts in einem Patienten, wobei besagtes Kit umfasst:
Peptide, die alternierende hydrophobe und hydrophile Aminosäuren besitzen, so dass, wenn die Peptide einem Elektrolyt in Lösung exponiert sind, jeder hydrophile Rest in einem Peptid entweder eine Wasserstoffbrückenbindung oder ein Ionenpaar mit einem hydrophilen Rest auf einem benachbarten Peptid bildet oder dem Lösemittel exponiert ist und die Variation des Interpeptid-Abstands kleiner als 4 Å ist; und hinreichend Elektrolyt, um einen Selbstzusammenbau besagter Peptide zu einem makroskopischen beta-Faltblatt-Gerüst zu initiieren, wodurch Zellen durch die Bildung von besagtem makroskopischem Gerüst verkapselt sind und in besagtem makroskopischem Gerüst in einer dreidimensionalen Anordnung vorhanden sind; und Anleitungen für eine Initiierung des Selbstzusammenbaus besagter Peptide zu besagtem Gerüst; und worin besagte Zellen Chondrocyten sind und das Gerüst einer Anwendung eines vorher festgelegten Kompressionsschemas unterzogen wird, um eine Sekretion extrazellulärer Matrixkomponenten zu induzieren; und das Gerüst wenigstens 5 Wochen kultiviert ist.

12. Das Kit nach Anspruch 11, worin:
a) das Gerüst einem variablen Kompressionsschema unterzogen wird; oder
b) das Gerüst in einem Medium kultiviert wird, das Insulin, Transferrin und Selen und weniger als 10% fötales bovines Serum enthält; oder
c) der Gleichgewichts-Kompressionsmodul von besagtem Gerüst wenigstens 40 kPa beträgt; oder
d) das Medium Insulin, Transferrin und/oder Selen enthält; oder
e) das Kit außerdem eine therapeutisch aktive Verbindung oder einen Chemoattraktant enthält; oder
f) besagte Peptide eine Adhäsionsstelle, Wachstumsfaktor-Bindungsstelle, Wachstumsfaktor oder Sequenz umfassen, die ein Zielen auf eine Zelle, ein Gewebe, Organ, Organsystem oder eine Stelle in einem Säuger bereitstellt; oder
g) das Kit ein Mittel zur Präparierung besagter Zellen für eine Zugabe zu einer Peptide umfassenden Lösung umfasst; oder
h) das Kit ein Mittel für eine Ausübung eines Drucks auf besagtes Gerüst umfasst; oder
i) das Kit eine Form für die Bildung besagten Gerüsts mit einer erwünschten Geometrie und/oder Volumen umfasst.

13. Das Gerüst nach Anspruch 1 oder das Kit nach Anspruch 11 für eine therapeutische Verwendung.

14. Das Gerüst oder Kit nach Anspruch 13, worin die Therapie ein Verfahren zur Regenerierung eines Gewebes ist und/oder in einem Verfahren zur Behandlung oder Prävention eines Knorpeldefekts, Bindegewebedefekts, Nervengewebedefekts, Epidermisauskleidungsdefekts, Endothelauskleidungsdefekts oder Arthritis angewandt wird.

## Revendications

1. Echafaudage macroscopique comprenant des peptides amphiphiles, des cellules vivantes et un milieu, dans lequel lesdits peptides sont complémentaires et structurellement compatibles avec des acides aminés hydrophobes et hydrophiles alternés qui sont disposés de manière à ce que chaque résidu hydrophile dans un peptide soit lié par liaison hydrogène ou forme une paire ionique avec un résidu hydrophile sur un peptide adjacent ou soit exposé à un solvant, et que la variation de la distance interpeptide soit inférieure à 4 Å, dans lequel lesdits peptides s'auto-assemblent en échafaudage macroscopique de type feuillet bêta ; et dans lequel ledit échafaudage macroscopique encapsule les cellules vivantes, lesdites cellules étant présentes dans ledit échafaudage macroscopique suivant une disposition tridimensionnelle, dans lequel :
lesdites cellules sont des chondrocytes et l'échafaudage est soumis à l'application d'un schéma de compression prédéterminée pour induire la sécrétion de composants de la matrice extracellulaire ; et l'échafaudage est mis en culture pendant au moins 5 semaines.

2. Echafaudage macroscopique selon la revendication 1, dans lequel un ou plusieurs des cas suivants se présentent :
a) l'échafaudage est soumis à un schéma de compression variable ; ou
b) l'échafaudage est mis en culture dans un milieu contenant de l'insuline, de la transferrine et du sélénium et moins de 10 % de sérum bovin foetal ; ou
c) le module de compression à l'équilibre dudit échafaudage est d'au moins 40 kPA ; ou
d) l'échafaudage comprend une solution qui inclut de l'insuline, de la transferrine et/ou du sélénium et moins de 10 % de sérum bovin foetal ; ou
e) l'échafaudage encapsule en outre un composé thérapeutiquement actif ou un chemoattractant ; ou
f) lesdits peptides comprennent un site d'adhérence, un site de liaison au facteur de croissance, un facteur de croissance ou une séquence qui permet un ciblage vers une cellule, un tissu, un organe, un système d'organe ou un site à l'intérieur d'un mammifère ; ou
g) lesdites cellules sécrètent des composants de matrice extracellulaire et où ladite sécrétion de composants de la matrice extracellulaire augmente d'au moins 50 fois le module de compression à l'équilibre dudit échafaudage ;
h) au moins 60 % des cellules encapsulées sont en contact cellule-cellule avec une autre cellule encapsulée ou avec une cellule se trouvant à l'extérieur de l'échafaudage ; ou
i) l'échafaudage comprend des cellules d'un type cellulaire souhaité et des chondrocytes, dans lequel la présence des chondrocytes dans ledit échafaudage entraîne une augmentation de la rigidité dudit échafaudage.

3. Echafaudage macroscopique selon la revendication 1, dans lequel des cellules qui ne sont pas des chondrocytes sont également présentes dans ledit échafaudage ; et dans lequel lesdites cellules sont des cellules de moelle osseuse, des cellules du périoste, des cellules du périchondre, des fibroblastes, des cellules neuronales, des cellules de l'hippocampe, des cellules épidermiques, des cellules endothéliales, des kératinocytes, des cellules basales, des cellules épineuses, des cellules granulaires, des cellules souches embryonnaires non humaines, des cellules ovariennes, des cellules pancréatiques, des cellules cervicales, des cellules hépatiques ou des cellules de prépuce.

4. Echafaudage macroscopique selon la revendication 1, dans lequel le module de compression à l'équilibre dudit échafaudage est d'au moins 90 kPA.

5. Echafaudage macroscopique selon la revendication 1, dans lequel le milieu comprend une solution qui inclut de l'insuline, de la transferrine et/ou du sélénium et moins de 10 % de sérum bovin foetal; et ladite solution comprend au moins 0,005 mg/litre de transferrine ou au moins 0,1 mg/litre d'insuline ou au moins 0,005 mg/litre de sélénium.

6. Procédé de formation de l'échafaudage macroscopique selon la revendication 1, ledit procédé comprenant les étapes suivantes :
a) incuber les peptides et les cellules vivantes dans une solution aqueuse comprenant un soluté isoosmotique, dans lequel lesdits peptides contiennent des acides aminés hydrophobes et hydrophiles alternés de manière à ce que quand les peptides sont exposés à un électrolyte en solution, chaque résidu hydrophile dans un peptide soit lié par liaison hydrogène ou forme une paire ionique avec un résidu hydrophile sur un peptide adjacent ou soit exposé à un solvant et que la variation de la distance interpeptide soit inférieure à 4 Å ; et
b) ajouter un électrolyte à ladite solution en quantité suffisante pour initier l'auto-assemblage desdits peptides en échafaudage macroscopique de type feuillet bêta, moyennant quoi lesdites cellules sont encapsulées par la formation dudit échafaudage macroscopique et sont présentes dans ledit échafaudage macroscopique suivant une disposition tridimensionnelle ; dans lequel :
lesdites cellules sont des chondrocytes et l'échafaudage est soumis à l'application d'un schéma de compression prédéterminée pour induire la sécrétion de composants de la matrice extracellulaire ; et l'échafaudage est mis en culture pendant au moins 5 semaines.

7. Procédé selon la revendication 6, dans lequel
a) l'échafaudage est soumis à un schéma de compression variable ; ou
b) l'échafaudage est mis en culture dans un milieu contenant de l'insuline, de la transferrine et du sélénium et moins de 10 % de sérum bovin foetal ; ou
c) le module de compression à l'équilibre dudit échafaudage est d'au moins 40 kPA, et de préférence d'au moins 90 kPA ; ou
d) l'échafaudage comprend une solution qui inclut de l'insuline, de la transferrine et/ou du sélénium et moins de 10 % de sérum bovin foetal, et facultativement dans lequel :
i) ladite solution comprend au moins 0,1 mg/litre d'insuline ;
ii) ladite solution comprend au moins 0,005 mg/litre de transferrine ; ou
iii) ladite solution comprend au moins 0,005 mg/litre de sélénium ; ou
e) ladite solution est contenue dans un moule de préforme dont les dimensions déterminent le volume ou la forme dudit échafaudage macroscopique ;
pour ainsi former un échafaudage macroscopique de forme prédéterminée ou de volume prédéterminé ; ou
f) le procédé comprend un procédé de culture de cellules et le procédé inclut l'étape de culture desdites cellules.

8. Procédé selon la revendication 6 ou 7, dans lequel :
i) ladite sécrétion de composants de la matrice extracellulaire augmente d'au moins 50 fois le module de compression à l'équilibre dudit échafaudage, et dans un tel cas facultativement dans lequel ladite sécrétion de composants de la matrice extracellulaire augmente d'au moins 100 fois le module de compression à l'équilibre dudit échafaudage, et en outre facultativement dans lequel ladite sécrétion de composants de la matrice extracellulaire augmente d'au moins 150 fois le module de compression à l'équilibre dudit échafaudage ; ou
ii) la raideur dynamique de l'échafaudage macroscopique à 1 Hz est d'au moins 1,0 MPa;
iii) ledit schéma de compression comprend la soumission dudit échafaudage à une pression variable ;
iv) ledit schéma de compression comprend la soumission dudit échafaudage à une compression dynamique entre 0,01 Hz et 3 Hz inclus, superposée à une compression décalée statique ; et facultativement dans lequel ledit schéma de compression comprend la soumission dudit échafaudage à une compression dynamique entre 0,1 Hz à 1 Hz inclus ;
v) l'amplitude de la déformation est entre 0,01 % et 10 % inclus ; et facultativement dans lequel l'amplitude de la déformation est entre 1 % et 5 % inclus ;
vi) ledit schéma de compression comprend la soumission dudit échafaudage à une pression variable ou constante pendant au moins une semaine ; et facultativement dans lequel ledit schéma de compression comprend la soumission dudit échafaudage à une pression variable ou constante pendant au moins trois semaines ;
vii) ledit schéma de compression comprend la soumission dudit échafaudage à une pression pendant une heure et l'incubation dudit échafaudage en l'absence de pression ajoutée pendant une à sept heures, inclus ; et facultativement dans lequel ledit schéma de compression est répété au moins quatre fois ;
viii) ledit schéma de compression comprend la soumission dudit échafaudage à une pression pendant 45 minutes et l'incubation dudit échafaudage en l'absence de pression ajoutée pendant une à sept heures, inclus ; et facultativement dans lequel ledit échafaudage est incubé en l'absence de pression ajoutée pendant cinq à six heures, inclus ; et en outre facultativement dans lequel ledit schéma de compression est répété au moins quatre fois.

9. Procédé selon la revendication 7 ou 8, l'échafaudage étant soumis à un schéma de charge de cisaillement prédéterminé.

10. Procédé selon la revendication 7, 8 ou 9, dans lequel :
a) au moins 60 % des cellules encapsulées sont en contact cellule-cellule avec une autre cellule capsulée ;
b) ladite solution dans l'étape a) contient moins de 10 mM d'électrolyte et dans laquelle lesdits peptides ne s'auto-assemblent pas de manière appréciable avant l'étape b) ; ou
c) ledit échafaudage comprend des cellules d'un type cellulaire souhaité et des chondrocytes, dans lequel la présence des chondrocytes dans ledit échafaudage entraîne une augmentation de la rigidité dudit échafaudage.

11. Kit destiné au traitement d'un défaut tissulaire chez un patient, ledit kit comprenant :
des peptides contenant des acides aminés hydrophobes et hydrophiles en alternance de manière à ce que quand les peptides sont exposés à un électrolyte en solution, chaque résidu hydrophile dans un peptide soit lié par liaison hydrogène ou forme une paire ionique avec un résidu hydrophile sur un peptide adjacent ou soit exposé à un solvant et que la variation de la distance interpeptide soit inférieure à 4 Å ; et suffisamment d'électrolytes pour initier l'auto-assemblage desdits peptides en échafaudage macroscopique de type feuillet bêta, moyennant quoi des cellules sont encapsulées par la formation dudit échafaudage macroscopique et sont présentes dans ledit échafaudage macroscopique suivant une disposition tridimensionnelle ; et des instructions pour initier l'auto-assemblage desdits peptides afin de former ledit échafaudage ; et dans lequel :
lesdites cellules sont des chondrocytes et l'échafaudage est soumis à l'application d'un schéma de compression prédéterminée pour induire la sécrétion de composants de la matrice extracellulaire ; et l'échafaudage est mis en culture pendant au moins 5 semaines.

12. Kit selon la revendication 11, dans lequel :
a) l'échafaudage est soumis à un schéma de compression variable ; ou
b) l'échafaudage est mis en culture dans un milieu contenant de l'insuline, de la transferrine et du sélénium et moins de 10 % de sérum bovin foetal ; ou
c) le module de compression à l'équilibre dudit échafaudage est d'au moins 40 kPA ; ou
d) le milieu comprend de l'insuline, de la transferrine et/ou du sélénium ; ou
e) le kit comprend en outre un composé thérapeutiquement actif ou un chemoattractant ; ou
f) lesdits peptides comprennent un site d'adhérence, un site de liaison au facteur de croissance, un facteur de croissance ou une séquence qui permet un ciblage vers une cellule, un tissu, un organe, un système d'organes ou un site à l'intérieur d'un mammifère ; ou
g) le kit comprend un moyen pour préparer lesdites cellules afin de les ajouter à une solution comprenant des peptides ; ou
h) le kit comprend un moyen pour appliquer une pression sur ledit échafaudage ; ou
i) le kit comprend un moule pour que ledit échafaudage puisse être formé avec une géométrie souhaitée et/ou un volume souhaité.

13. Echafaudage selon la revendication 1 ou le kit selon la revendication 11, pour un usage thérapeutique.

14. Echafaudage ou kit selon la revendication 13, dans lequel la thérapie est un procédé de régénération d'un tissu et/ou est utilisée dans un procédé permettant de traiter ou de prévenir un défaut du cartilage, un défaut du tissu conjonctif, un défaut du tissu nerveux, un défaut de la couche épidermique, un défaut de la couche endothéliale ou une arthrite.
